# EUROPEAN PATENT APPLICATION

(11) **EP 4 520 816 A1**
(43) Date of publication of application: **12.03.2025**
(21) Application number: 23795655.2
(22) Date of filing: 28.04.2023
(51) Int. Cl.: C12N 1/20, A61K 35/74, A61P 3/00, A61P 3/04, A61P 3/06, A61P 3/10, C12R 1/01

(54) **PHARMACEUTICAL COMPOSITION, MEGASPHAERA, AND USE THEREOF**

(30) Priority: 28.04.2022 CN 202210471327; 28.04.2022 CN 202210471322; 28.04.2022 CN 202210470811; 09.01.2023 CN 202310029400; 09.01.2023 CN 202310029410; 09.01.2023 CN 202310029842
(71) Applicant: Moon (Guangzhou) Biotech Co., Ltd., Guangzhou, Guangdong 510535 (CN)
(72) Inventor: XIAN, Yibo, Guangzhou, Guangdong 510535 (CN); JIANG, Xianzhi, Guangzhou, Guangdong 510535 (CN); ZHANG, Dongya, Guangzhou, Guangdong 510535 (CN); ZHAO, Guozhen, Guangzhou, Guangdong 510535 (CN)
(74) Representative: Boult Wade Tennant LLP
(86) International application number: PCT/CN2023/091789
(87) International publication number: WO 2023/208223

(57) **Abstract**

Provided herein are *Megasphaera* sp. strains deposited with the Guangdong Microbial Culture Collection Center with deposit numbers of GDMCC No: 62001, GDMCC No: 62000, and GDMCC No: 61999, respectively. 16S rRNAs of the strains are set forth in SEQ ID NOs: 1-3, respectively. The bacterial strains are highly productive of butyric acid and/or acetic acid. Further provided is use of the strains in the manufacture of a medicament for preventing and/or treating metabolic diseases.

## Description

### CROSS-REFERENCE TO RELATED APPLICATIONS

The present application claims the right of priority to Chinese patent application No. 202210470811.2 filed on April 28, 2022; 202210471327.1 filed on April 28, 2022; 202210471322.9 filed on April 28, 2022; 202310029400.4 filed on January 9, 2023; 202310029410.8 filed on January 9, 2023; and 202310029842.9 filed on January 9, 2023, the contents of which are incorporated herein by reference in their entirety.

### TECHNICAL FIELD

The present disclosure relates to the field of biomedicine, and in particular to a pharmaceutical composition, *Megasphaera,* and use thereof.

### BACKGROUND OF THE INVENTION

The gut microbiome, composed of the collective genomes and functional materials from trillions of bacteria, archaea, fungi, viruses, and other microeukaryotic colonizers, has been widely recognized as the "second brain" that performs multiple functions in regulating human health and disease development as well as in clinical practice. The intestinal tract is the largest microecological environment in the human body, participating in many important physiological processes such as nutrient absorption, energy metabolism, tissue and organ development, immune defense, and endocrine regulation. There are a large number of symbiotic microorganisms in the human intestinal tract. The total amount of genetic information carried by the microorganisms is 50 to 100 times that of the human genome, and known as the "gut micorbiome". The gut micorbiome is the largest and most direct external environment of the human body and plays a relatively important role in maintaining human health. Accumulating evidence reveals protective interactions between specific gut microbes, products released from the microbes through metabolic activities, and the host in manipulating host immunity, metabolism, and cancer development. With the rapid development of molecular biology, genomics, bioinformatics analysis technology, high-throughput sequencing technology, and microbial culture technology, the role and influence of intestinal flora on intestinal and extraintestinal diseases have become increasingly clear. With regard to microbiome-based therapies, many different bacterial species can affect the human digestive, circulatory, and nervous systems, and are closely associated with a variety of human diseases, including cancers, infections, gastrointestinal inflammation, autoimmune diseases, metabolic disorders, central nervous system diseases, and mental diseases. Studying the relationship between intestinal flora and human health and diseases is not only an important scientific research, but also has important significance and value in clinical diagnosis, treatment, and even translation.

Up to now, there have been research reports on the role of the gut micorbiome in nutritional disorders, metabolic abnormalities, and complex diseases (such as obesity, diabetes, inflammatory bowel disease, metabolic disorders, etc.). However, the types of microorganisms currently studied only involve a very small number of specific species. For example, in the context of treatment of obesity-related diseases, gut microbes usually involve probiotics such as *Bifidobacterium, Bacteroides,* and *Lactobacillus,* while other gut microbes have yet to be developed into viable bacterial drugs for treating or preventing metabolic diseases.

The bacteria of the genus *Megasphaera* mainly exist in the intestinal tract of humans or animals, and belong to strictly anaerobic microorganisms with extremely high requirements for nutrition and culture environment and a long growth cycle. In addition, in the context of verification of pharmaceutical effect, the bacteria of this genus are difficult to verify through *in vitro* cell experiments due to their anaerobic nature. During *in vivo* experiments in animals, the bacteria of this genus are difficult to maintain a stable viable bacterial count due to the difficulty in culturing and high degree of anaerobicity, and there is also a problem of insufficient repeatability. These *in vivo* and *in vitro* related technical problems limit discovery and use of new species of the genus *Megasphaera.*

In addition, in the prior art, such as in the field of probiotics or FMT transplantation, usually a mixture of multiple microorganisms acts on the intestinal tract. The prior art also involves using a mixture of multiple bacteria as a drug for treating metabolic diseases. However, the mechanism of action of multiple bacteria on indications is more complex, and the influence of bacteria on each other has not been well studied. Use of multiple bacteria as a viable bacterial drug will disrupt the homeostasis of the intestinal flora. In contrast, a single bacterium has less impact on the homeostasis of the intestinal flora. In addition, the use of mixed bacteria requires additional consideration of whether the bacteria will affect each other's activities, and it is also not clear which specific bacteria in the mixed bacteria can directly produce a therapeutic effect.

SCFAs from microorganisms have been fully studied in the prior art for the treatment or prevention of metabolic diseases such as obesity and diabetes. The prior art "Gut microbial metabolites in obesity, NAFLD and T2DM" shows that metabolites produced by carbohydrate fermentation which are related to weight control include acetic acid, propionic acid, butyric acid, and succinic acid; and acetate and butyrate have also been proved to induce satiety through central mechanisms, increase thermogenesis in adipose tissue and liver, and induce adipose tissue browning and leptin secretion. In addition, acetic acid, propionic acid, and butyric acid stimulate secretion of the satiety hormones glucagon-like peptide 1 (GLP1) and peptide YY (PYY) in a G-protein-coupled receptor (GPR)-dependent manner.

A study in mice in 2017 shows that long-term oral administration of butyrate can prevent diet-induced obesity, NAFLD progression, and insulin resistance. These effects are primarily associated with a reduction in food intake, butyrate-induced inhibition of the activity of orexigenic neurons expressing neuropeptide Y in the hypothalamus, and decreased neural activity of the brainstem nucleus tractus solitarius and dorsal vagal complex. In addition, intraperitoneal injection of acetic acid, propionic acid, and butyric acid has been proved to suppress energy intake in mice through a mechanism related to vagal afferent stimulation, and propionate and butyrate esters prevent obesity and insulin resistance by inducing intestinal gluconeogenesis (IGN).

In summary, the strain's ability to produce acetic acid (acetate), butyric acid (butyrate), or propionic acid (propionate) in SCFAs can indicate its therapeutic potential in obesity, diabetes, fatty liver, etc. Therefore, further discovery, exploration, and research of microbial strains with metabolic disorder prevention or treatment potential have important application values and market prospects.

In view of this, the present invention is proposed.

### SUMMARY OF THE INVENTION

In one aspect, the present disclosure relates to a pharmaceutical composition comprising a bacterium of the genus *Megasphaera*;
the 16S rRNA sequence of the bacterium of the genus *Megasphaera* is ≥ 95% identical to SEQ;
the SEQ comprises at least one of SEQ ID No: 1, SEQ ID No: 2, and SEQ ID No: 3;
the composition can be used to suppress appetite, prevent and/or alleviate a metabolic disease;
preferably, the suppressing appetite comprises at least one of reducing food intake and reducing appetite;
preferably, the metabolic disease is at least one of a liver disease, obesity, a cardiovascular disease, a cardiovascular and cerebrovascular disease, hyperlipidemia, diabetes, and impaired glucose tolerance;
optionally, the liver disease is at least one of fatty liver, NAFLD, NASH, decreased liver weight, or liver dysfunction;
optionally, the liver disease comprises a disease caused by a high-fat diet, by a high-cholesterol diet, by a high-carbohydrate diet, by high lipids and high cholesterol, by high lipids and high glucose, and/or by high lipids, high cholesterol and high glucose;
optionally, the obesity disease is obesity caused by a high-fat diet, obesity caused by high cholesterol, obesity caused by a high-carbohydrate diet, obesity caused by high lipids and high cholesterol, obesity caused by high lipids and high glucose, obesity caused by high lipids, high cholesterol and high glucose, or obesity in NAFLD/NASH patients;
optionally, the cardiovascular disease or cardiovascular and cerebrovascular disease is atherosclerosis, and/or a cardiovascular disease in NAFLD/NASH patients, and/or a cardiovascular and cerebrovascular disease in NAFLD/NASH patients, and/or a high cholesterol disease;
the liver disease comprises a liver disease or liver dysfunction caused by at least one of a high-fat diet, a high-cholesterol diet, and a high-carbohydrate diet; further, the liver disease comprises a liver disease or liver dysfunction caused by a high-fat diet, by a high-cholesterol diet, by a high-carbohydrate diet, by high lipids and high cholesterol, by high lipids and high glucose, and/or by high lipids, high cholesterol and high glucose;
optionally, the obesity disease comprises obesity caused by at least one of a high-fat diet, a high-cholesterol diet, and a high-carbohydrate diet; further, the obesity disease comprises obesity caused by a high-fat diet, obesity caused by high cholesterol, obesity caused by a high-carbohydrate diet, obesity caused by high lipids and high cholesterol, obesity caused by high lipids and high glucose, obesity caused by high lipids, high cholesterol and high glucose, or obesity in NAFLD/NASH patients;
optionally, the cardiovascular disease or cardiovascular and cerebrovascular disease is atherosclerosis, and/or a cardiovascular disease in NAFLD/NASH patients, and/or a cardiovascular and cerebrovascular disease in NAFLD/NASH patients, and/or a high cholesterol disease;
optionally, the diabetes comprises diabetes caused by obesity, type II diabetes, or diabetes in NAFLD/NASH patients;
optionally, the diabetes comprises diabetes caused by at least one of a high-fat diet, a high-cholesterol diet, and a high-carbohydrate diet; further, the diabetes comprises diabetes caused by a high-fat diet, diabetes caused by high cholesterol, diabetes caused by a high-carbohydrate diet, diabetes caused by high lipids and high cholesterol, diabetes caused by high lipids and high glucose, diabetes caused by high lipids, high cholesterol and high glucose, or diabetes in NAFLD/NASH patients;
preferably, the metabolic disease is at least one of type I diabetes, type II diabetes, gestational diabetes, impaired glucose tolerance, insulin resistance, weight control, overweight, blood glucose control, prediabetes, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, diabetic retinopathy, dermatosis, dyspepsia, or edema.

The pharmaceutical composition further comprises a second active substance or co-drug, wherein the second active substance or co-drug comprises at least one of a GLP-1 receptor agonist, a dual agonist of GLP-1 receptor and GCG receptor, a triple agonist of GLP-1 receptor, GIP receptor and GCG receptor, an AMPK agonist or an active drug that promotes GLP-1 secretion, a DPP-4 receptor inhibitor, a PPAR receptor agonist, a PPARα receptor agonist, a PPARδ receptor agonist, a PPARγ receptor agonist, a PPARα/δ receptor dual agonist, a PPARα/γ receptor dual agonist, a PPARα/δ/γ receptor triple agonist, or an active drug in the mechanism/target of CRTC, PGC-1α, SREBP, FXR, FGF21, ASK1, THR-β, LXR, NF-κB, SNDRI, MC4R, PNLIP, MOA, or DRI;
preferably, the GLP-1 receptor agonist comprises any one of Semaglutide, liraglutide, exenatide, or beinaglutide;
preferably, the AMPK agonist or active drug that promotes GLP-1 secretion comprises Metformin, Semaglutide, and/or liraglutide.

The pathogenesis of some diseases or disorders is characterized by reduced stability of microbiota. Examples of such diseases and disorders are IBS, IBD, diabetes (e.g., type 2 diabetes), allergic diseases, autoimmune diseases, and metabolic diseases/disorders. The bacterial strains of the present disclosure can also be used to treat or prevent diseases by modulating the stability of the microbiota.

Another aspect of the present disclosure also relates to use of the pharmaceutical composition, the bacterial strain, or the bacterium of *Megasphaera* in the manufacture of a medicament for treating and/or preventing a disease;
preferably, the disease comprises at least one of a tumor, an infectious disease, a metabolic disease, an autoimmune disease, an inflammatory disease, or a neurological disease;
preferably, the metabolic disease comprises at least one of a liver disease, obesity, a cardiovascular disease, a cardiovascular and cerebrovascular disease, hyperlipidemia, diabetes, or impaired glucose tolerance;
optionally, the liver disease is at least one of fatty liver, NAFLD, NASH, decreased liver weight, or liver dysfunction;
optionally, the liver disease comprises a disease caused by a high-fat diet, by a high-cholesterol diet, by a high-carbohydrate diet, by high lipids and high cholesterol, by high lipids and high glucose, and/or by high lipids, high cholesterol and high glucose;
optionally, the obesity disease is obesity caused by a high-fat diet, obesity caused by high cholesterol, obesity caused by a high-carbohydrate diet, obesity caused by high lipids and high cholesterol, obesity caused by high lipids and high glucose, obesity caused by high lipids, high cholesterol and high glucose, or obesity in NAFLD/NASH patients;
optionally, the cardiovascular disease or cardiovascular and cerebrovascular disease is atherosclerosis, and/or a cardiovascular disease in NAFLD/NASH patients, and/or a cardiovascular and cerebrovascular disease in NAFLD/NASH patients, and/or a high cholesterol disease;
optionally, the diabetes comprises diabetes caused by obesity, type II diabetes, or diabetes in NAFLD/NASH patients;
optionally, the diabetes is diabetes caused by a high-fat diet, diabetes caused by high cholesterol, diabetes caused by a high-carbohydrate diet, diabetes caused by high lipids and high cholesterol, diabetes caused by high lipids and high glucose, diabetes caused by high lipids, high cholesterol and high glucose, or diabetes in NAFLD/NASH patients.

Preferably, the 16S rRNA sequence of the bacterium of the genus *Megasphaera* is ≥ 95% identical to SEQ; and
the SEQ comprises at least one of SEQ ID No: 1, SEQ ID No: 2, and SEQ ID No: 3.

As compared to the prior art, the present invention has the following beneficial effects:
In the present disclosure, several anaerobic strains belonging to the genus *Megasphaera* has been isolated and screened out from the human intestinal tract, which are natural strains (non-cloned and non-processed strains). Through identification, it has been determined that they belong to new species under the genus *Megasphaera.* All of the strains can express EC2.8.3.9 enzyme, have a butyrate production pathway with at least 70% integrity, can effectively produce butyric acid and some other SCFAs, and can effectively prevent and treat metabolic diseases.

In the present disclosure, utilizing the similar commonality of *Megasphaera* in butyrate production, strains were screened for candidate drugs by further gene and butyrate pathway analysis, thereby improving screening efficiency. It has been verified that all of the screened strains can be effectively used for preventing and treating metabolic diseases. The screened bacterial strains can inhibit the activity of histone acetylase by regulating short-chain fatty acids/short-chain fatty acid salts to achieve the purpose of preventing and treating metabolic diseases.

The microbial preparation provided herein comprises the strain of *Megasphaera* or a metabolite thereof, and has the effect of preventing and treating metabolic diseases. The drug for preventing and/or treating metabolic diseases as provided herein comprises the strain of *Megasphaera* isolated and screened from the human intestinal tract or a metabolites thereof, which can be used for treating diseases and has a lower side effect.

The pharmaceutical composition provided herein can achieve a better effect of treating metabolic diseases by combining a drug containing the strain of *Megasphaera* with other drugs for treating the metabolic diseases. The pharmaceutical composition provided herein can regulate at least one short-chain fatty acid or regulate lactate, and is useful for the treatment of metabolic diseases.

### BRIEF DESCRIPTION OF THE DRAWINGS

In order to more clearly illustrate the technical solutions in the specific embodiments of the present disclosure or the prior art, the accompanying drawings to be used in the description of the specific embodiments or the prior art will be briefly introduced below. Obviously, the accompanying drawings in the following description relate to some embodiments of the present disclosure. For those of ordinary skill in the art, other drawings can be obtained based on these accompanying drawings without exerting any creative effort.
Fig. 1 shows the colonial morphology of the strains MNH05026, MNH22004, and MNH27256 cultured on anaerobic blood plates for 48 h;
Fig. 2 shows the microscopic morphology of the strains MNH05026, MNH22004, and MNH27256;
Fig. 3 shows the microscopic morphology of the strains MNH05026, MNH22004, and MNH27256 upon Gram-staining;
Fig. 4 shows the microscopic morphology of the strains MNH05026, MNH22004, and MNH27256 upon spore staining;
Fig. 5 shows the results of tolerance of the strains MNH05026, MNH22004, and MNH27256 to different concentrations of NaCl;
Fig. 6 shows the results of tolerance of the strains MNH05026, MNH22004, and MNH27256 to different pH values;
Fig. 7 shows the results of tolerance of the strains MNH05026, MNH22004, and MNH27256 to different concentrations of bile salts;
Fig. 8 shows the results of culturing the strain MNH 05026 in the culture medium API 20;
Fig. 9A shows the 16S rRNA gene phylogenetic tree of the strain MNH 05026;
Fig. 9B shows the 16S rRNA gene phylogenetic tree of the strain MNH 22004;
Fig. 9C shows the 16S rRNA gene phylogenetic tree of the strain MNH 27256;
Fig. 10 is a graph showing the effects of the strains MNH05026 and MNH22004 on liver weight in high-fat diet-induced obesity mice;
Fig. 11 is a graph showing the effect of the strain MNH05026 on the serum alanine aminotransferase (ALT) level in high-fat diet-induced obesity mice;
Fig. 12 shows the effects of the strains MNH05026 and MNH27256 on fasting blood glucose (FGB) in high-fat diet-induced obesity mice;
Fig. 13 shows the effect of MNH05026 combined with Metformin on oral glucose tolerance (OGTT) in mice with type II diabetes; in which Fig. 13 (A) shows the effect of MNH05026 combined with Metformin on oral glucose tolerance (OGTT) in mice with type II diabetes, and Fig. 13 (B) shows the area under the curve of oral glucose tolerance of experimental mice in each treatment group;
Fig. 14 shows the effects of MNH05026 and MNH27256 respectively combined with Metformin on fasting blood glucose in mice with type II diabetes;
Fig. 15 shows the fasting blood glucose change in mice with type II diabetes treated with MNH27256 alone and in combination with Metformin;
Fig. 16 shows the effects of the strains MNH05026, MNH27256, and MNH22004 on body weight gain in high-fat diet-induced obesity mice;
Fig. 17 shows the effects of the strains MNH05026, MNH22004, and MNH27256 respectively combined with Semaglutide on body weight in mice with NAFLD/NASH induced by a high-fat, high-carbohydrate, and high-cholesterol diet;
Fig. 18 shows the effects of MNH05026, MNH22004, and MNH27256 respectively combined with Semaglutide on liver weight in mice with NAFLD/NASH induced by a high-fat, high-carbohydrate, and high-cholesterol diet;

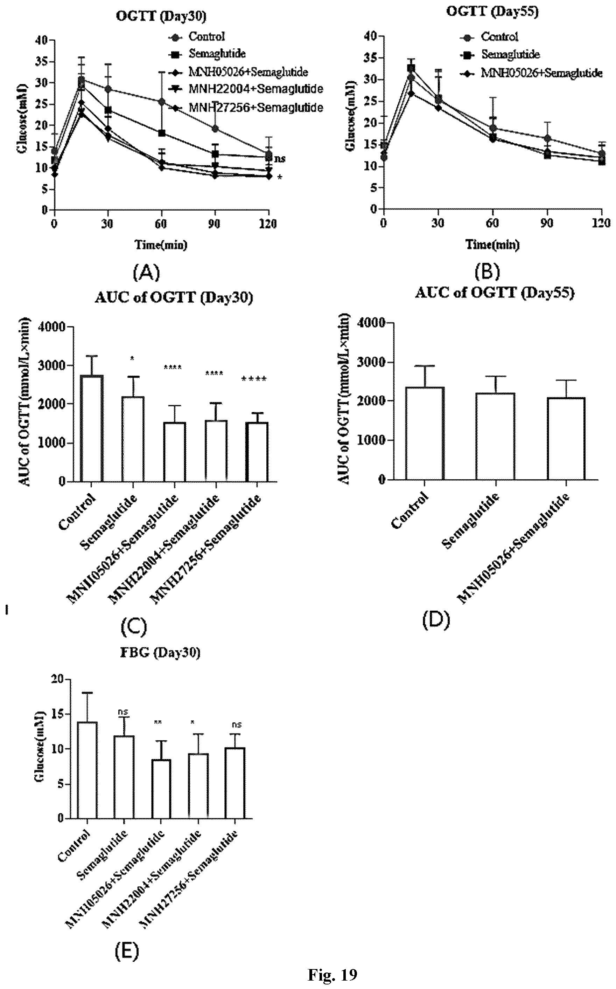

Fig. 19 shows the effects of MNH05026, MNH22004, and MNH27256 combined with Semaglutide on oral glucose tolerance in model mice with NAFLD/NASH induced by a high-fat, high-carbohydrate, and high-cholesterol diet; in which Fig. 19 (A) is a graph showing the effects of MNH05026, MNH22004, and MNH27256 combined with Semaglutide on oral glucose tolerance on Day 30 in model mice with NAFLD/NASH induced by a high-fat, high-carbohydrate, and high-cholesterol diet; Fig. 19 (B) shows the effect of MNH05026 combined with Semaglutide on oral glucose tolerance on Day 55 in model mice with NAFLD/NASH induced by a high-fat, high-carbohydrate, and high-cholesterol diet; Figs. 19 (C) and (D) show the results of the area under the curve of oral glucose tolerance corresponding to Figs. 19 (A) and (B); and Fig. 19 (E) is a graph showing the effects of MNH05026, MNH22004, and MNH27256 combined with Semaglutide on the fasting blood glucose value on Day 30 of oral glucose tolerance test in model mice with NAFLD/NASH induced by a high-fat, high-carbohydrate, and high-cholesterol diet;
Fig. 20 shows the effects of MNH05026, MNH22004, and MNH27256 respectively combined with Semaglutide on subcutaneous fat pad weight, inguinal fat pad weight, brown adipose tissue weight, and epididymis fat pad weight in model mice with NAFLD/NASH induced by a high-fat, high-carbohydrate, and high-cholesterol diet;
Fig. 21 shows the effect of MNH05026 combined with Semaglutide on triglycerides (TG), total cholesterol (CHO), high-density lipoprotein (HDL), and low-density lipoprotein (LDL) in model mice with NAFLD/NASH induced by a high-fat, high-carbohydrate, and high-cholesterol diet;
Fig. 22 shows the effects of MNH05026, MNH22004, and MNH27256 respectively combined with Semaglutide on liver steatosis, lobular inflammation, liver ballooning, liver NAS score, and liver fibrosis in mice with NAFLD/NASH induced by a high-fat, high-carbohydrate, and high-cholesterol diet; and
Fig. 23 is a graph showing the inhibitory effects of MNH22004 and MNH05026 on deacetylase activity.

### DETAILED DESCRIPTION OF THE INVENTION

The technical solutions of the present disclosure will be clearly and completely described below with reference to the accompanying drawings and specific embodiments. However, those skilled in the art will understand that the embodiments described below are part of the embodiments of the present disclosure, rather than all of the embodiments, and are intended to illustrate the present disclosure only, but should not be regarded as limiting the scope of the present disclosure. All other embodiments obtained by those of ordinary skill in the art based on the embodiments of the present disclosure without exerting any creative effort fall within the scope of protection of the present disclosure. If no specific conditions are specified in the examples, the experiments were carried out according to conventional conditions or conditions recommended by the manufacturer. The reagents or instruments used with no manufacturer indicated are all conventional products that are commercially available.

It is known in the art that bacterial species can be classified and identified by traditional classification methods and molecular biology methods. The traditional classification methods include, for example, observation of cell morphology, Gram staining, flagella staining, various metabolic experiments, etc. The molecular biology methods include ribosomal RNA sequencing, determination methods based on whole genome sequencing, etc.

16S rRNA is a ribosomal RNA of prokaryotes. The 16S rRNA gene consists of a variable region and a conserved region. The conserved region is common to all bacteria, while the variable region differs to different extent among different bacteria. By comparing the 16S rRNA gene sequences of bacteria, a phylogenetic tree can be drawn based on their sequence differences and evolutionary distances.

The "identity" between the sequences of two nucleic acid molecules can be determined by a known computer algorithm, such as the "FASTA" program, the GCG program package, BLASTN, or FASTA. The commercially or publicly available program can also be, for example, the DNAStar "MegAlign" program.

With the rapid development of the second-generation and third-generation sequencing technologies, species identification based on whole genome sequencing has become possible, and renders the results of species identification more accurate. The average nucleotide identity (ANI) of bacterial genomes refers to the similarity of homologous genes between two bacterial genomes. The ANI value can be calculated by BLAST or other methods. In the field of bacterial taxonomy, it is generally believed that two strains will be considered as belonging to the same species only when the ANI value reaches 95% or above (Jain C, Rodriguez-R L M, Phillippy A M, et al. High throughput ANI analysis of 90K prokaryotic genomes reveals clear speciesboundaries[J]. Nature Communications, 2018, 9(1): 5114).

At present, there are various well-established tools for calculating ANI values, such as the local calculation softwares Jspecies (/jspecies) and Gegenees (/documentation.html), and the online calculation tools ANI caculator (http:/enveomics.gatech.edu/), EzGenome (/ezgenome/ani), and ANItools.

By using the above methods, those skilled in the art can determine whether an isolated strain belongs to the new species of the genus *Megasphaera* as discovered by the present inventors. For example, when the average nucleotide identity (ANI) value to the strain deposited with GDMCC No: 62001, GDMCC No: 62000, or GDMCC No: 61999 is at least 95%, e.g., 95.5%, 96%, 96.5%, 97%, 97.5%, 98%, 98.5%, 99%, 99.5%, 99.6%, 99.7%, 99.8%, 99.9%, or 100%, it can be determined that the strains belong to the same species.

The *Megasphaera* sp. as described herein can be prepared into a pharmaceutical composition, for example, by using a pharmaceutically acceptable excipient. The pharmaceutical composition comprises a pharmaceutically effective amount of the strain of the *Megasphaera* sp., such as a strain deposited with GDMCC No: 62001, GDMCC No: 62000, or GDMCC No: 61999. Similarly, the *Megasphaera* species to which the strains deposited with GDMCC No: 62001, GDMCC No: 62000, and GDMCC No: 61999 belong can also be prepared into a pharmaceutical composition, for example, by using a pharmaceutically acceptable excipient, which comprises a pharmaceutically effective amount of the strain of the *Megasphaera* sp.

A suitable pharmaceutically acceptable excipient that can be used is, for example, a carrier, an excipient, a diluent, a lubricant, a wetting agent, an emulsifier, a suspension stabilizer, a preservative, a sweetener, or a flavor. For example, the pharmaceutically acceptable excipient is one or more of lactose, glucose, sucrose, sorbitol, mannose, starch, corn starch, trehalose, fructose, sodium ascorbate, L-cysteine hydrochloride, skim milk powder, sodium alginate, calcium chloride, sodium carboxymethyl cellulose, gum arabic, calcium phosphate, alginate, gelatin, calcium silicate, fine crystalline cellulose, polyvinylpyrrolidone, cellulose, water, syrup, methylcellulose, methylparaben, propylparaben, talc, magnesium stearate, and mineral oil.

The term "supernatant" in the context of the present disclosure refers to a culture supernatant of the bacterial strain according to the prevent disclosure, optionally comprising compounds and/or cell debris of the strain, and/or metabolites and/or molecules secreted by the strain.

The pharmaceutical composition provided herein can comprise a pharmaceutically acceptable excipient, diluent, or carrier. Acceptable carriers or diluents for therapeutic use are well known in the pharmaceutical field. Examples of suitable carriers include lactose, starch, dextrose, methylcellulose, magnesium stearate, mannitol, sorbitol, etc. Examples of suitable diluents include ethanol, glycerol, and water. The pharmaceutical carrier, excipient, or diluent can be selected depending on the intended route of administration and standard pharmaceutical practice. The pharmaceutical composition can comprise any suitable binder, lubricant, suspending agent, coating agent, solubilizer, or the like as or in addition to the carrier, excipient or diluent. Examples of a suitable binder include starch, gelatin, a natural sugar, and a natural or synthetic gum. The natural sugar is, for example, glucose, anhydrous lactose, free-flowing lactose, β-lactose, or corn sweetener. The natural or synthetic gum is, for example, gum arabic, tragacanth, sodium alginate, carboxymethyl cellulose, or polyethylene glycol. Examples of a suitable lubricant include sodium oleate, sodium stearate, magnesium stearate, sodium benzoate, sodium acetate, sodium chloride, or the like. A preservative, a stabilizer, a dye, or even a flavoring agent can be provided in the pharmaceutical composition. Examples of the preservative include sodium benzoate, sorbic acid, or parabens. An antioxidant or a suspending agent can also be used. An antioxidant or a suspending agent can also be used.

### Information on bacterial taxonomy

### Use of 16S rRNA in taxonomy:

Judgment criteria: When the similarity between the 16S rRNA gene sequences of two strains is less than 97-98.65%, they can be judged to belong to different species.

### The method for calculating the "identity":

The "identity" between the nucleic acid sequences of two nucleic acid molecules can be determined as percent identity by a known computer algorithm, such as the "FASTA" program, using default parameters, e.g., in Pearson et al. (Other programs include the GCG package (Devereux, J., et al., Nucleic Acids Research 12(I):387 (1984)), BLASTP, BLASTN, and FASTA, for example, the BLAST function of the NCBI database). Other commercially or publicly available programs include the DNAStar "MegAlign" program.

### Diabetes-related

The term "insulin resistance" refers to a decrease in the efficiency of insulin in promoting glucose uptake and utilization due to various reasons, and compensatory secretion of excessive insulin causing hyperinsulinemia in order to maintain the stability of blood glucose. Insulin resistance is prone to lead to metabolic syndrome and type 2 diabetes.

Four metabolic disease-related models were used in the present disclosure: a mouse obesity model induced by a high-fat diet (HFD), a mouse NASH model induced by high-fat, high-fructose, and high-cholesterol, a type 2 diabetes mouse model induced by a high-fat diet combined with streptozotocin (HFD-STZ), and a leptin receptor gene-deficient mouse model (db/db). The four models are all commonly used mouse models for metabolic diseases, and the model mice are usually accompanied by a metabolic disease such as obesity, insulin resistance, hyperglycemia, hyperlipidemia, hypercholesterolemia, NAFLD/NASH, or the like.

Alanine aminotransferase (ALT) and aspartate aminotransferase (AST) are sensitive markers of hepatocellular injury. Alanine aminotransferase (ALT) and aspartate aminotransferase (AST) levels are elevated in case of liver dysfunction (injury) or a liver disease (NAFLD/NASH). In addition, there is a large amount of fat accumulation in the liver of NAFLD/NASH patients, and the liver weight will be increased. Therefore, a reduction in ALT and AST levels and a reduction in liver weight after drug intervention can indicate some therapeutic or ameliorative effects of the drug.

### Liver and kidney function-related diseases:

The term "liver and kidney disease" refers to functional acute renal failure or decompensated liver cirrhosis that occurs in a severe liver disease, which can result in hepatorenal syndrome due to insufficient effective circulating blood volume, reduced prostaglandins, or the like. The present disclosure focuses on liver-related diseases, mainly relevant studies on NAFLD/NASH.

The term "non-alcoholic fatty liver disease (NAFLD)" refers to accumulation of excessive fat in the liver in the form of triglycerides (steatosis). There are also some NAFLD patients who suffer from hepatocellular damage and inflammation in addition to excessive fat (steatohepatitis), i.e., NASH. NASH is widely recognized as a hepatic manifestation of metabolic syndrome, such as type II diabetes, insulin resistance, central obesity, hyperlipidemia (low high-density lipoprotein cholesterol, hypertriglyceridemia), and hypertension.

TG is mainly involved in energy metabolism *in vivo* and produces thermal energy. Too high TG content in the blood can lead to viscous blood, causing lipids to deposit on the blood vessel wall and gradually form small plaques, which is called atherosclerosis. Increased LDL-C is a main and independent risk factor for the onset and development of atherosclerosis. The increased level of LDL-C is also an indicator for measuring coronary heart disease. Since HDL-C can transport cholesterol in the blood vessel wall to the liver for catabolism (i.e., reverse cholesterol transport), it can reduce the deposition of cholesterol on the blood vessel wall and plays an anti-atherosclerotic role.

Diabetes mellitus includes type 1 diabetes (T1D), type 2 diabetes (T2D), and gestational diabetes mellitus (GDM). Type 1 diabetes is a type of diabetes mellitus caused by autoimmune impairment or an idiopathic cause, characterized by absolute destruction of pancreatic islet functions, which mostly occurs in children and adolescents, and must be treated with insulin in order to obtain a satisfactory outcome, or it will be life-threatening. Type 2 diabetes is a multifactorial syndrome characterized by abnormal carbohydrate/fat metabolism, usually including hyperglycemia, hypertension, and abnormal cholesterol. Type 2 diabetes is caused by ineffective role of insulin (low binding to the receptor). Therefore, it is important to test not only fasting blood glucose, but also 2-hour postprandial blood glucose, and especially to perform a pancreatic islet function test. There are two types of diabetes during pregnancy: diabetes diagnosed before pregnancy, called "diabetes with pregnancy"; and diabetes that occurs or is diagnosed only during pregnancy, with normal glucose metabolism or potentially impaired glucose tolerance before pregnancy, also known as "gestational diabetes mellitus (GDM)". More than 80% of pregnant women with diabetes suffer from GDM.

Oral glucose tolerance test is used to measure the function of islet β cells and the body's ability to regulate blood glucose. It is currently recognized as a diagnostic indicator for diagnosing diabetes. In case of glucose metabolism disorder, after a certain amount of glucose is administered orally, blood glucose rises sharply, or it does not rise obviously, but cannot drop to the fasting level or the original level in a short time. This is abnormal glucose tolerance or impaired glucose tolerance. Abnormal glucose tolerance indicates that the body's ability to metabolize glucose is reduced, which is common in type 2 diabetes and obesity.

HOMA-IR is an indicator used to evaluate the insulin resistance level of an individual. It is currently widely used in the clinical evaluation of insulin sensitivity in diabetic patients. It is calculated according to the following equation: fasting blood glucose level (FPG, mmol/L) × fasting insulin level (FINS, µU/mL)/22.5. The HOMA-IR index of a normal individual is 1. As the level of insulin resistance increases, the HOMA-IR index will be above 1.

The L cells in the intestinal tract can secrete glucagon-like peptide-1 (GLP-1), which can promote the production of insulin by islet β cells and inhibit the production of glucagon by islet α cells, thereby regulating the body's blood glucose balance and improving the body's glucose tolerance. The inventors have discovered that *Christensenella* sp. can increase the secretion level of glucagon-like peptide-1 (GLP-1), thereby regulating the body's blood glucose balance, improving the body's glucose tolerance, and further improving the body's insulin sensitivity and leptin sensitivity, and thus achieving the effect of preventing and/or treating diabetes and/or hyperlipidemia.

### Inflammation:

Lipopolysaccharide (LPS), also known as cellular endotoxin, is a phospholipid that constitutes the outer cell wall of Gram-negative bacteria. In addition to maintaining the structural integrity of bacteria, LPS can protect the bacteria from being broken down by bile salts secreted by the gallbladder. Generally, LPS is blocked from the bloodstream by tight junctions in the intestinal lining cells. If LPS enters the blood, it will induce a strong inflammatory response in animals.

Therefore, the level of LPS in the blood can reflect the level of inflammation.

### Resistin and inflammation:

Resistin is a hormone or adipokine secreted by adipose tissue and is associated with obesity and insulin resistance. In humans, resistin has been characterized as a hormone expressed and secreted by immune cells, especially macrophages, and has been implicated in many inflammatory responses, including adipose tissue inflammation due to macrophage infiltration. Resistin can play an important role in the onset and development of obesity and insulin resistance through resistin-induced inflammation. Resistin is also associated with other chronic diseases, such as cardiovascular diseases and cancers. In many studies, resistin has been proposed as an important biomarker of metabolism-related diseases.

### Obesity-related:

The term "obesity" refers to a certain degree of marked overweight with a thick fat layer, which is a state caused by excessive accumulation of body fat, especially triglycerides, and abnormal or excessive fat accumulation that poses a risk to the health. Excessive body fat accumulation as a result of excessive food intake or altered metabolism results in excessive weight gain and causes a pathological or physiological change or latency. A body mass index (BMI) of over 25 is considered overweight and over 30 is considered obese. Obesity will increase the risk of many physical and mental diseases. It is mainly associated with metabolic syndrome, including a combination of diseases such as type 2 diabetes, hypertension, hypercholesterolemia, hypertriglyceridemia, etc. Generally, the effects of obesity on health fall into two broad categories: diseases attributable to increased body fat (e.g., osteoarthritis, obstructive sleep apnea, etc.) and diseases with an increased number of adipocytes (e.g., diabetes mellitus, dyslipidemia, cancer, cardiovascular disease, non-alcoholic fatty liver disease, non-alcoholic steatohepatitis, etc.).

### Glucagon peptide:

Glucagon-like peptide-1 (GLP-1) is a hormone mainly produced by intestinal L cells and belongs to incretins. Glucagon-like peptide-1 receptor agonist (GLP-1RA) is a novel hypoglycemic drug in recent years. It activates GLP-1 receptors, enhances insulin secretion in a glucose concentration-dependent manner, inhibits glucagon secretion, can delay gastric emptying, and reduces food intake through central appetite suppression, thereby achieving the effects of lowering blood glucose and losing weight.

### Leptin:

Leptin is a hormone secreted by adipose tissue, and its content in serum is directly proportional to the size of animal adipose tissue. Leptin acts on the receptor located in the central nervous system (Leptin receptor) to regulate the behavior and metabolism of organisms. When an animal has decreased body fat or is in a low-energy state (such as hunger), the leptin level in serum will drop significantly, thereby stimulating the animal's foraging behavior while reducing its own energy expenditure. Conversely, when the body fat of an organism increases, the leptin level in serum will increase, thereby inhibiting food intake and accelerating metabolism. Leptin regulates the organism's energy balance and body weight through such a negative feedback mechanism.

### Leptin resistance:

As the amount of fat continues to increase, leptin is continuously secreted. Long-term and large amounts of leptin stimulation make the brain no longer sensitive to leptin, which is medically known as "leptin resistance".

The disease related to liver function impairment includes at least one of fatty liver, non-alcoholic fatty liver disease, non-alcoholic steatohepatitis, liver fibrosis, cirrhosis, and liver cancer.

The obesity-related disease includes at least one of obesity, metabolic syndrome, a cardiovascular disease, hyperlipidemia, hypercholesterolemia, hypertension, insulin resistance syndrome, obesity-related gastroesophageal reflux disease, and steatohepatitis.

The diabetes-related disease includes at least one of type II diabetes, insulin resistance syndrome, glucose intolerance, a hyperlipidemic disorder, a complication of diabetic nephropathy, diabetic neuropathy, diabetic ophthalmopathy, a cardiovascular disease, or diabetic foot.

The "obesity-related disease" as described above can be selected from overeating, binge eating, bulimia, hypertension, diabetes mellitus, elevated plasma insulin concentration, insulin resistance, hyperlipidemia, metabolic syndrome, insulin resistance syndrome, obesity-related gastroesophageal reflux disease, atherosclerosis, hypercholesterolemia, hyperuricemia, lower back pain, cardiomegaly and left ventricular hypertrophy, lipodystrophia, non-alcoholic steatohepatitis, cardiovascular disease, and polycystic ovary syndrome, as well as subjects with these obesity-related diseases including those who wish to lose weight.

The second active substance is another therapeutic agent for a metabolic disease, such as a GLP-1 receptor agonists, a dual agonist of GLP-1 receptor and GCG receptor, a triple agonist of GLP-1 receptor, GIP receptor and GCG receptor, an AMPK agonist or an active drug that promotes GLP-1 secretion; including metformin, sulfonylureas, meglitinides, thiazolidinediones, DPP-4 inhibitors, GLP-1 receptor agonists, SGLT2 inhibitors, insulin, pioglitazone, rosiglitazone, pentoxifylline, omega-3-fatty acids, statins, ezetimibe, ursodeoxycholic acid, Semaglutide, liraglutide, exenatide, or beinaglutide.

In some embodiments, the method further comprises administering a prebiotic to the subject. In some embodiments, the prebiotic is fructooligosaccharide, galactooligosaccharide, trans-galactooligosaccharide, xylooligosaccharide, chitooligosaccharide, soy oligosaccharide, gentiooligosaccharide, isomaltooligosaccharide, mannooligosaccharide, maltooligosaccharide, mannooligosaccharide, lactulose, lactosucrose, palatinose, glycosyl sucrose, guar gum, arabic gum, tagatose, amylose, amylopectin, pectin, xylan, or cyclodextrin.

The term "administration" or "administering" generally refers to the route by which a composition (e.g., a pharmaceutical composition) is given to a subject. Examples of routes of administration include oral, rectal, topical, inhalation (nasal), or injection administration. The injection administration includes intravenous (IV), intramuscular (IM), intratumoral (IT), and subcutaneous (SC) administration. The pharmaceutical composition as described herein can be administered in any form by any effective route, including, but not limited to, intratumoral, oral, parenteral, enteral, intravenous, intraperitoneal, topical, transdermal (e.g., using any standard patch), intracutaneous, intraocular, intranasal, local, non-oral, e.g. aerosol, inhalation, subcutaneous, intramuscular, buccal, sublingual, (trans)rectal, vaginal, intraarterial and intrathecal administration. In a preferred embodiment, the pharmaceutical composition as described herein is administered orally, rectally, intratumorally, topically, intravesically, by injection into or near draining lymph nodes, intravenously, by inhalation or aerosol, or subcutaneously. In another preferred embodiment, the pharmaceutical composition as described herein is administered orally, intratumorally, or intravenously.

The term "increase", "enhancement" or "improvement" refers to a change such that the difference between post-treatment and pre-treatment states is at least 10%, 20%, 30%, 40%, 50%, 60%, 70%, 80%, 90%, 2-fold, 4-fold, 10-fold, 100-fold, 10³-fold, 10⁴-fold, 10⁵-fold, 10⁶-fold, and/or 10⁷-fold as appropriate. The characteristics that may be increased include the number of immune cells, bacterial cells, stromal cells, myeloid-derived suppressor cells, fibroblasts, or metabolites; the levels of cytokines; or other physical parameters (such as tumor size).

The term "reduction", "decrease" or "decline" refers to a change such that the difference between post-treatment and pre-treatment states is at least 10%, 20%, 30%, 40%, 50%, 60%, 70%, 80%, 90%, 1/100, 1/1000, 1/10,000, 1/100,000, 1/1,000,000, or undetectable as appropriate. The characteristics that may be reduced include the number of immune cells, bacterial cells, stromal cells, myeloid-derived suppressor cells, fibroblasts, or metabolites; the levels of cytokines; or other physical parameters such as ear thickness (e.g., in animal models of DTH) or tumor size.

The term "insulin resistance" has its common meaning in the art. Insulin resistance is a physiological status in which the natural hormone insulin becomes less effective in lowering blood glucose. The resulting increase in blood glucose can cause the blood glucose level to go beyond the normal range, and lead to adverse health effects such as metabolic syndrome, dyslipidemia, and subsequent type 2 diabetes. As used herein, the terms "insulin resistance-related complication" and "insulin resistance-related disorder" include, but are not limited to, metabolic syndrome, dyslipidemia, and type 2 diabetes, as well as insulin resistance in endocrine diseases (e.g., type 2 diabetes in obese subjects, type 1 diabetes, Cushing's disease, and lipodystrophy syndrome).

As used herein, the term "metabolic disease" or "metabolic disorder" refers to a group of disorders that occur together, which increase the risk of heart diseases, stroke, and type II diabetes. These conditions include elevated blood pressure, high blood glucose, hyperliposis, obesity, and abnormal cholesterol or triglyceride levels. In some embodiments, the metabolic disease is type II diabetes, impaired glucose tolerance, insulin resistance, weight control, overweight, blood glucose control, prediabetes, obesity, hyperglycemia, hyperinsulinemia, fatty liver, non-alcoholic steatohepatitis, hypercholesterolemia, hypertension, hyperlipoproteinemia, hyperlipidemia, hypertriglyceridemia, ketoacidosis, hypoglycemia, thrombotic disease, dyslipidemia, non-alcoholic fatty liver disease (NAFLD), or a related disease. In some embodiments, the related disease is a cardiovascular disease, atherosclerosis, or kidney disease.

As used herein, the term "metabolite" refers to a compound, composition, or molecule that is used as a substrate or product in any cellular or microbial metabolic reaction, or an ion, cofactor, catalyst or nutrient from any cellular or microbial metabolic reaction.

The term "strain" refers to a member of a bacterial species that has a genetic characteristic such that it can be distinguished from closely related members of the same bacterial species. The genetic characteristic can be the absence of all or part of at least one gene, the absence of all or part of at least one regulatory region (e.g., promoter, terminator, riboswitch, or ribosome binding site), the absence ("curing" of at least one natural plasmid), the presence of at least one recombinant gene, the presence of at least one mutated gene, the presence of at least one exogenous gene (a gene from another species), at least one mutated regulatory region (e.g., promoter, terminator, riboswitch, or ribosome binding site), the presence of at least one non-natural plasmid, the presence of at least one antibiotic resistance cassette, or a combination thereof. The genetic characteristic between different strains can be identified by PCR amplification, optionally followed by DNA sequencing of the genomic region of interest or the entire genome. In cases where one strain (as compared to another strain of the same species) acquires or loses antibiotic resistance or acquires or loses biosynthesis ability (e.g., an auxotrophic strain), the strain or nutrient/metabolite can be identified by selection or counter-selection using an antibiotic.

The term "supernatant" in the context of the present disclosure refers to a culture supernatant of the bacterial strain according to the prevent disclosure, optionally comprising compounds and/or cell debris of the strain, and/or metabolites and/or molecules secreted by the strain.

The term "subject" or "patient" refers to any mammal. A subject or patient "in need thereof" as described herein refers to an individual in need of treatment (or prevention) of a disease. The mammal includes humans, laboratory animals (e.g., primates, rats, or mice), farm animals (e.g., cows, sheep, goats, or pigs), and domestic pets (e.g., dogs, cats, or rodents). The subject can be a human. The subject can be a non-human mammal, including, but not limited to, dog, cat, cow, horse, pig, donkey, goat, camel, mouse, rat, guinea pig, sheep, camel, monkey, gorilla, or chimpanzee. The subject or patient can be healthy or can suffer from a metabolic disease at any stage of development.

As used herein, the term "treating" a disease in a subject or "treating" a subject suffering or suspected of suffering from a disease refers to administering to the subject a drug therapy, such as one or more agents, thereby reducing or preventing the deterioration of at least one symptom of the disease. Therefore, in one embodiment, "treating" means inter alia delaying progression, accelerating remission, inducing remission, increasing remission, accelerating recovery, increasing the efficacy of an alternative therapy, decreasing the resistance to an alternative therapy, or a combination thereof.

As used herein, the term "prevention" is well-recognized in the art and, when used in connection with a condition such as local recurrence, is well known in the art. It comprises administering a treatment that reduces the development of, or delays the onset of, a symptom of a medical disorder in a subject as compared to a subject who does not receive the composition.

The present disclosure develops bacterial strains of the genus *Megasphaera* (MNH05026, MNH22004, and MNH27256) and novel compositions comprising the same. They can be used to treat and prevent cancers, autoimmune and inflammatory diseases, metabolic diseases, neurodegenerative diseases, and mental diseases, especially by directly action of short-chain fatty acids (SCFAs) and/or medium-chain fatty acids (MCFAs), or by inhibiting the activity of histone deacetylase (HDAC) through SCFA and/or MCFA mediation to achieve the purpose of treating the above diseases. It has been determined that bacterial strains from the genus *Megasphaera* can efficiently produce a number of SCFAs and MCFAs, including acetic acid, butyric acid, valeric acid, and hexanoic acid. These short-chain fatty acids have been demonstrated to improve symptoms of a variety of diseases, including enhancing the anti-tumor efficacy of immunotherapy, gastrointestinal infectious diseases, inflammatory bowel disease (IBD), metabolic diseases, autoimmune diseases, neurodegenerative diseases, and mental diseases.

Known species under the genus *Megasphaera* include *Megasphaera elsdenii, Megasphaera stantonii, Megasphaera massiliensis, Megasphaera indica, Megasphaera paucivorans, Megasphaerasueciensis, Megasphaera micronuciformis, Megasphaera hexanoica,* and *Megasphaera cerevisiae.* In the species, *Megasphaera elsdenii* type strain DSM 20460, *Megasphaera indica* type strain NMBHI-10, *Megasphaera massiliensis* type strain NP3 and the strain NCIMB 42787 (see CN112601534A), *Megasphaera cerevisiae* type strain DSM 20462, *Megasphaera paucivorans* type strain DSM 16981, *Megasphaera sueciensis* type strain DSM *17042, Megasphaera micronuciformis* type strain DSM 17226, and *Megasphaera hexanoica* type strain MH all have the ability to produce short-chain fatty acids, especially butyric acid (see Table 1 in "Megasphaera hexanoica sp. nov., a medium-chain carboxylic acid-producing bacterium isolated from a cow rumen; DOI: 10.1099/ijseMegasphaera 0.001888").

When drug screening is performed in the present disclosure, the ability of a strain under the genus *Megasphaera* to produce butyric acid is first predicted and analyzed through genomic data analysis at the gene level. A gene element is further identified by determining the key enzyme that can be expressed to produce butyric acid based on the determination of the butyrate production pathway and the integrity of the butyrate production pathway. Bacterial strains containing the gene element/key enzyme (protein) are selected, and the acid production capacity is verified at the experimental data level. Subsequently, the screened candidate strains are subjected to *in vitro* experiments and mouse experiments to verify their effects in tumor suppression and metabolic disease prevention and treatment. According to this screening logic, the bacterial strains MNH05026, MNH22004, and MNH27256 under the genus *Megasphaera* were screened out for anti-tumor experiments and metabolic disease prevention and treatment experiments. The experiments proved that the three *Megasphaera* species of the present disclosure had anti-tumor and metabolic disease prevention and treatment effects.

A bacterial strain of the genus *Megasphaera* of the present disclosure (MNH05026) has a deposit number of GDMCC No: 62001, and a 16S rRNA sequence as set forth in SEQ ID NO: 1;
another bacterial strain of the genus *Megasphaera* of the present disclosure (MNH22004) has a deposit number of GDMCC No: 62000, and a 16S rRNA sequence as set forth in SEQ ID NO: 2; and
yet another bacterial strain of the genus *Megasphaera* of the present disclosure (MNH27256) has a deposit number of GDMCC No: 61999, and a 16S rRNA sequence as set forth in SEQ ID NO: 3.

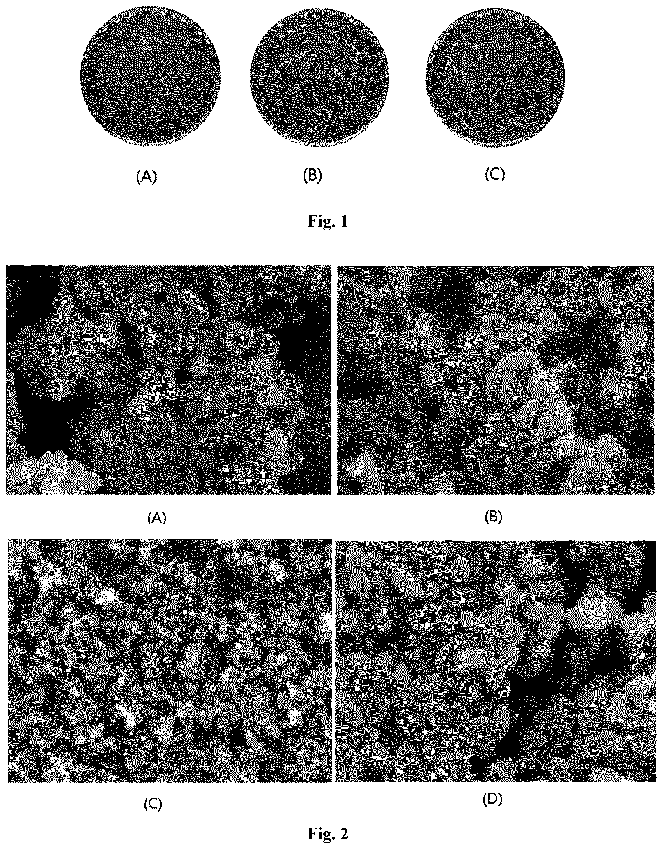

Fig. 1 shows the colonial morphology of the strains MNH05026, MNH22004, and MNH27256 cultured on anaerobic blood plates for 48 h; in which Fig. 1A shows the colonial morphology of the strain MNH 05026 cultured on an anaerobic blood plate for 48 h; Fig. 1B shows the colonial morphology of the strain MNH22004 cultured on an anaerobic blood plate for 48 h; and Fig. 1C shows the colonial morphology of the strain MNH27256 cultured on an anaerobic blood plate for 48 h.

Fig. 2 shows the microscopic morphology of the strains MNH05026 and MNH22004, and Figs. 2C and 2D show the microscopic morphology of MNH27256 at different magnifications; in which Fig. 2A shows the microscopic morphology of the strain MNH05026, Fig. 2B shows the microscopic morphology of the strain MNH22004, and Figs. 2C and 2D show the microscopic morphology of MNH27256 at different magnifications.

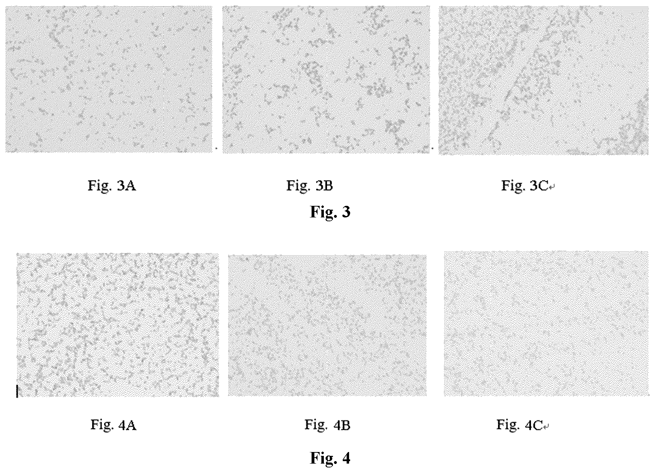

Fig. 3 shows the microscopic morphology of the strains MNH05026, MNH22004, and MNH27256 upon Gram-staining; in which Fig. 3A shows the microscopic morphology of the strain MNH05026 upon Gram-staining; Fig. 3B shows the microscopic morphology of the strain MNH22004 upon Gram-staining; and Fig. 3C shows the microscopic morphology of the strain MNH27256 upon Gram-staining.

Fig. 4 shows the microscopic morphology of the strains MNH05026, MNH22004, and MNH27256 upon spore staining; in which Fig. 4A shows the microscopic morphology of the strain MNH05026 upon spore staining; Fig. 4B shows the microscopic morphology of the strain MNH22004 upon spore staining; and Fig. 4C shows the microscopic morphology of the strain MNH27256 upon spore staining.

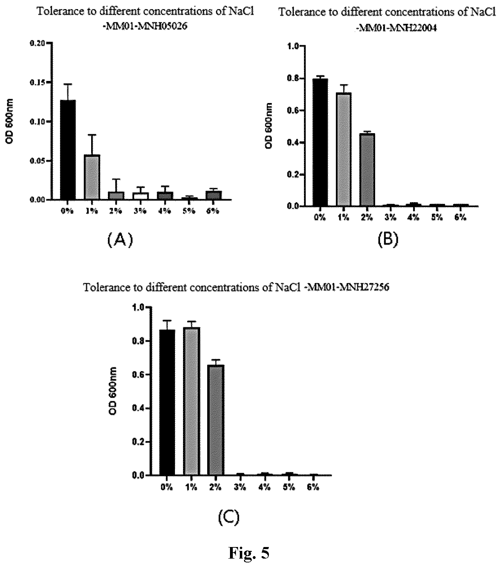

Fig. 5 shows the results of tolerance of the strains MNH05026, MNH22004, and MNH27256 to different concentrations of NaCl; in which Figs. 5A, 5B, and 5C show the results of tolerance of the strains MNH05026, MNH22004, and MNH27256 to different concentrations of NaCl, respectively.

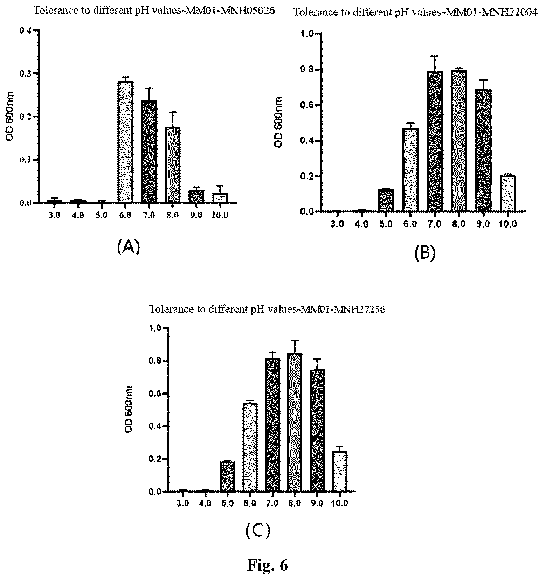

Fig. 6 shows the results of tolerance of the strains MNH05026, MNH22004, and MNH27256 to different pH values; in which Figs. 6A, 6B, and 6C show the results of tolerance of the strains MNH05026, MNH22004, and MNH27256 to different pH values, respectively.

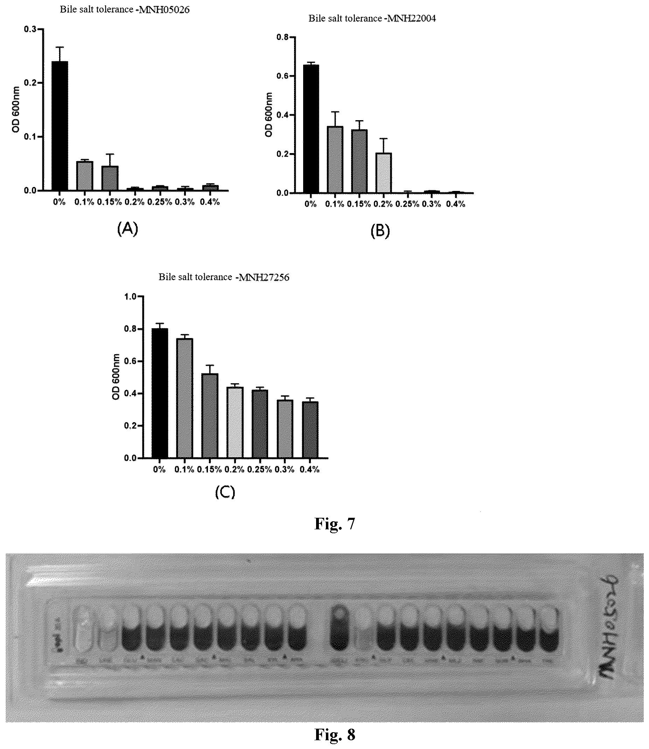

Fig. 7 shows the results of tolerance of the strains MNH05026, MNH22004, and MNH27256 to different concentrations of bile salts; in which Figs. 7A, 7B, and 7C show the results of tolerance of the strains MNH05026, MNH22004, and MNH27256 to different concentrations of bile salts, respectively.

Fig. 8 shows the results of culturing the strain MNH 05026 in the culture medium API 20.

At gene level: On the one hand, all of the bacterial strains MNH05026, MNH22004, and MNH27256 of the genus *Megasphaera* of the present disclosure can express butyrate-acetyl CoA-transferase subunit A (EC2.8.3.9 enzyme, see Uniprot.org for specific interpretation). It can be predicted that they have the ability to produce short-chain fatty acids, such as butyric acid. The GENE IDs of EC2.8.3.9 expressed by the bacterial strains MNH05026, MNH22004, and MNH27256 of the present disclosure involve: 650027236, 641897133, 650594268, 642201644, 650018449, 650536170, 2511555023, and 646248671 (for the sequences corresponding to the GENE IDs, see Integrated Microbial Genome (IMG); http://img.jgi.doe.gov).

On the other hand, the inventors summarized the strains under the genus *Megasphaera* with anti-tumor effects that have been recited and confirmed in literatures or patents. The whole genome data of these strains that have been disclosed were extracted from a database for whole genome data analysis. The integrity of the pyruvate pathway (Pyruvate_pathway) and/or the 4-aminobutyrate pathway (4aminobutyrate_pathway) was evaluated for these strains. The inventors found that only 2 strains had a butyrate pathway/pyruvate pathway integrity of less than 70%; but the two strains had a 4-aminobutyrate pathway integrity of higher than 70%. Both MNH22004 and MNH27256 had complete butyrate production pathways (i.e., the butyrate production pathway was 100% complete). MNH05026 had a butyrate pathway integrity of at least 70% and a 4-aminobutyrate pathway integrity of more than 80%.

At experimental data level: By using the method for measuring short-chain fatty acids (SCFAs) as described in Example 3 of the present disclosure, it has been confirmed that MNH05026, MNH22004, and MNH27256 can effectively produce butyric acid (Table 1). The experimental results are consistent with the prediction by genetic analysis. Subsequent experiments have confirmed that the bacterial strains MNH05026, MNH22004, and MNH27256 of the genus *Megashpaera* that can effectively produce butyric acid can effectively inhibit tumors, can be used to prevent and treat cancers, and can also effectively prevent and treat metabolic diseases.

**Table 1. Results of SCFA yields for various strains of the present disclosure**

| SCFA (ug/g) strain | MNH05026 | MNH22004 | MNH27256 |
|---|---|---|---|
| Acetic acid | 251.2136328 | 463.3187346 | 407.3867501 |
| Propionic acid | 42.84714505 | 103.9982679 | 119.2146502 |
| Isobutyric acid | 120.8069044 | 183.4828134 | 198.1745033 |
| Butyric acid | 214.7535761 | 920.3108438 | 1165.035869 |
| Isovaleric acid | 248.9603152 | 320.4344048 | 329.4617645 |

The bacterial strains MNH05026, MNH22004, and MNH27256 as disclosed herein have good ability to produce a variety of SCFAs such as acetic acid in addition to the ability to produce butyric acid. SCFAs from microorganisms have been fully studied in the prior art for the treatment or prevention of metabolic diseases such as obesity and diabetes. The prior art "Gut microbial metabolites in obesity, NAFLD and T2DM" shows that metabolites produced by carbohydrate fermentation which are related to weight control include acetic acid, propionic acid, butyric acid, and succinic acid; and acetate and butyrate have also been proved to induce satiety through central mechanisms, increase thermogenesis in adipose tissue and liver, and induce adipose tissue browning and leptin secretion. In addition, acetic acid, propionic acid, and butyric acid stimulate secretion of the satiety hormones glucagon-like peptide 1 (GLP-1) and peptide YY (PYY) in a G-protein-coupled receptor (GPR)-dependent manner.

In addition to the effect in treatment of metabolic diseases through the above pathways, butyric acid (butyrate) is also a metabolite that has been fully verified in the prior art to inhibit HDAC enzyme activity (Human gut bacteria as potent class I histone deacetylase inhibitors *in vitro* through production of butyric acid and valeric acid, https://doi.org/10.1371/journal.pone.0201073). Studies have shown that metabolic diseases such as diabetes can be effectively treated by inhibiting HDAC activity. Diabetes is a group of diseases in which a low level of insulin and/or peripheral insulin resistance leads to hyperglycemia. It has been proposed to treat diabetes by inhibiting HDAC activity through multiple mechanisms, including inhibition of Pdxl (Park, et al., 2008, J Clin Invest, 118, 2316-24), and enhancement of the expression of the transcription factor Ngn3 to increase the endocrine repertoire, progenitor cells (Haumaitre, et al., 2008, Mol Cell Biol, 28, 6373-83), enhancement of insulin expression (Molsey, et al., 2003, J Biol Chem, 278, 19660-6), etc. HDAC inhibition is also a promising therapy for advanced diabetic complications such as diabetic nephropathy and retinal ischemia (Christensen, et al., 2011, Mol Med, 17(5-6), 370-390).

Therefore, the present disclosure further discloses prevention and/or treatment of metabolic diseases by inhibiting histone acetylase (HDAC) activity with SCFAs and/or MCFAs produced by the strains MNH05026, MNH22004, and MNH27256; preferably, the histone acetylase is class I, class II, class III, or class IV histone acetylase. In addition, an experiment was carried out for verifying the inhibitory effects of metabolites (such as butyric acid) of the three strains MNH05026, MNH22004, and MNH27256 on HDAC.

The bacterial strains MNH05026, MNH22004, and MNH27256 of the genus *Megasphaera* of the present disclosure are butyric acid producers. Based on the known effects of butyric acid, MNH05026 can also reduce the impermeability of the blood-brain barrier that has a neuroprotective effect (Michel and Prat (2016) Ann Transl Med. 4(1): 15).

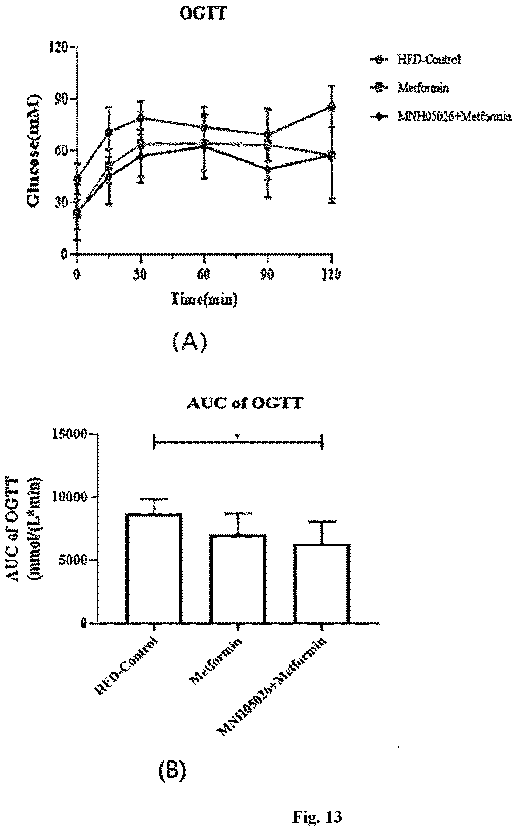

The bacterial strains MNH05026, MNH22004, MNH27256 of the genus *Megasphaera* and compositions thereof of the present disclosure can lead to increased expression of proinflammatory molecules such as proinflammatory cytokines in PBMCs (see Fig. 13). Administration of the pharmaceutical composition of the present disclosure can cause an increased expression of IL-1β in PBMCs. IL-1β is a pro-inflammatory cytokine. The production and secretion of IL-1β are regulated by the inflammasome, a protein complex associated with the activation of an inflammatory response. Since administration of the composition of the present disclosure has been shown to increase the expression of IL-1β, the composition of the present disclosure can be used to treat diseases characterized by a decreased expression of IL-1β.

In addition, the inventors have also determined that the *Megasphaera* sp. MNH05026, MNH22004, and MNH27256 can alleviate LPS-induced inflammation.

In some embodiments, it has been found that the strains of the genus *Megasphaera* and compositions thereof of the present disclosure are particularly beneficial in inducing the production of multiple SCFAs such as butyric acid and inhibiting HDAC activity, thereby achieving the effect of preventing and treating metabolic diseases. In certain embodiments, the strains of the genus *Megasphaera* and compositions thereof of the present disclosure, alone or in combination with an active substance such as Semaglutide, can exert the efficacy of preventing and treating metabolic diseases in a preclinical syngeneic mouse metabolic disease model. The strains of the genus *Megasphaera* and compositions thereof of the present disclosure are used to treat or prevent metabolic diseases, such as liver diseases, obesity, cardiovascular diseases, cardiovascular and cerebrovascular diseases, hyperlipidemia, diabetes, impaired glucose tolerance, etc. Specific examples include, but are not limited to, type II diabetes, impaired glucose tolerance, insulin resistance, weight control, overweight, blood glucose control, prediabetes, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, diabetic retinopathy, dermatosis, dyspepsia, or edema.

Acetic acid, butyric acid, valeric acid, and hexanoic acid have been shown to mediate autoimmune and inflammatory-related diseases. These effects can be achieved by reducing the synthesis of inflammatory cytokines and increasing immunoregulatory T cells (Tregs) and the production of anti-inflammatory cytokines. MNH05026, MNH22004, and MNH27256 can produce acetic acid, butyric acid, valeric acid, and hexanoic acid. MNH05026, MNH22004, and MNH27256 can increase anti-inflammatory cytokines and attenuate LPS-induced inflammation. Acetic acid and butyric acid have demonstrated protective effects in the treatment and prevention of obesity, type 2 diabetes, non-alcoholic fatty liver disease (NAFLD), cardiac metabolic diseases, and related complications. Such protective effects of acetic acid and butyric acid can be achieved by increasing thermogenesis of brown adipose tissue and browning of white adipose tissue, reducing lipid accumulation in the liver and adipose tissue, enhancing intestinal integrity, and exerting immunomodulatory effects. The inventors have discovered that *Megasphaera* sp. (MNH05026, MNH22004, and MNH27256) can not only produce acetic acid and butyric acid, but also induce the production of anti-inflammatory factors and alleviate LPS-induced diseases.

In some embodiments, administration of a composition comprising a *Megasphaera* sp. such as MNH05026, MNH22004, or MNH27256 can reduce HDAC activity.

In some embodiments, the present disclosure provides a method for treating or preventing an inflammatory bowel disease mediated by HDAC activity with a composition comprising a *Megasphaera* sp. such as MNH05026, MNH22004, or MNH27256. Inhibition of HDAC activity has been shown to suppress the production of pro-inflammatory cytokines in the gastrointestinal tract. Therefore, the strains of the genus *Megasphaera* and compositions thereof of the present disclosure can be used to treat inflammatory diseases. In particular, the strains of the genus *Megasphaera* and compositions thereof of the present disclosure can be used to treat or prevent disorders associated with the pathogenesis of increased colonic pro-inflammatory cytokines. In some embodiments, the strains of the genus *Megasphaera* and compositions thereof of the present disclosure are used to treat or prevent inflammatory bowel diseases. In some embodiments, the strains of the genus *Megasphaera* and compositions thereof of the present disclosure are used to treat or prevent ulcerative colitis. In some embodiments, the strains of the genus *Megasphaera* and compositions thereof of the present disclosure are used to treat or prevent Crohn's disease. In certain embodiments, the present disclosure provides the strains of the genus *Megasphaera,* MNH05026, MNH22004, and MNH27256, and compositions thereof, which are useful for treating or preventing inflammatory diseases. In a preferred embodiment, the present disclosure provides the strains of the genus *Megasphaera,* MNH05026, MNH22004, and MNH27256, and compositions thereof, which are useful for treating or preventing colitis.

In certain embodiments of the present disclosure, the bacterial strain in the composition is the strain MNH05026 of the genus *Megasphaera.*

In certain embodiments of the present disclosure, the bacterial strain in the composition belongs to a new species of *Megasphaera,* which is a strain having the following 16S rRNA gene sequence, or a closely related strain, e.g., a strain having a 16S rRNA gene sequence that is at least 95%, 96%, 97%, 98%, 98.63%, 99%, 99.7%, or 99.9% identical to SEQ ID NO: 1, a strain having a 16S rRNA gene sequence that is at least 95%, 96%, 97%, 98%, 98.63%, 99%, 99.5%, or 99.9% identical to SEQ ID NO: 2, or a strain having a 16S rRNA gene sequence that is at least 95%, 96%, 97%, 98%, 98.5%, 98.63%, 99%, 99.5%, or 99.9% identical to SEQ ID NO: 3. Preferably, the strain used in the present disclosure has a 16S rRNA gene sequence as set forth in SEQ ID NO: 1.

In certain embodiments, the present disclosure provides a food product comprising the above *Megasphaera* strain or a composition thereof.

In addition, the present disclosure provides a method for treating or preventing a disease or disorder mediated by HDAC activity, comprising administering a bacterial strain of the genus *Megasphaera* or a composition thereof. The present disclosure also provides a strain comprising such cells or a biologically pure culture of such cells and compositions thereof. The present disclosure also provides a cell of the genus *Megasphaera,* such as the strains MNH05026, MNH22004, or MNH27256 or a derivative thereof, for use in treatment, in particular treatment of the diseases described herein.

The bacterial strains of the genus *Megasphaera* and pharmaceutical compositions thereof of the present disclosure can be used to prevent and/or treat metabolic diseases, including, but not limited to, type II diabetes, impaired glucose tolerance, insulin resistance, weight control, overweight, blood glucose control, prediabetes, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, diabetic retinopathy, dermatosis, dyspepsia, or edema.

Preferably, the bacterial strains of the genus *Megasphaera* of the present disclosure include species having at least 95% identity to SEQ ID NO: 1, SEQ ID NO: 2, or SEQ ID NO: 3.

The bacterial strains of the present disclosure include *Megasphaera elsdenii, Megasphaera stantonii, Megasphaera indica, Megasphaera paucivorans, Megasphaera sueciensis, Megasphaera micronuciformis, Megasphaera hexanoica, Megasphaera cerevisiae,* or species strains having at least 95% identity to SEQ ID NO: 1, SEQ ID NO: 2, or SEQ ID NO: 3, which strains can express EC2.8.3.9.

Preferably, the bacterial strains are regulated by GENE ID: 650027236, 641897133, 650594268, 642201644, 650018449, 650536170, 2511555023, and 646248671 to express EC2.8.3.9.

The bacterial strains and compositions thereof of the present disclosure can regulate at least one short-chain fatty acid or short-chain fatty acid salt, wherein the short-chain fatty acid comprises acetic acid, butyric acid, valeric acid, butyric-valeric acid, or hexanoic acid. When a short-chain fatty acid exerts a therapeutic effect *in vivo,* it usually exists in the form of a short-chain fatty acid salt. Through verification, it has been found that acetate, propionate, and butyrate are the main short-chain fatty acid salts.

The bacterial strains involved in the present disclosure have a 16S rRNA sequence that is at least 95%, 95.5%, 96%, 96.5%, 97%, 97.5%, 98%, 98.5%, 98.65%, 99%, 99.5%, or 99.9% identical to SEQ ID NO: 1.

The bacterial strains and compositions thereof of the present disclosure are used for treating or preventing autoimmune diseases or inflammatory diseases, such as asthma, arthritis, psoriasis, graft-versus-host disease, inflammatory bowel disease, Crohn's disease, ulcerative colitis, allogeneic transplant rejection, chronic inflammatory bowel disease, systemic lupus erythematosus, psoriasis, rheumatoid arthritis, multiple sclerosis, or Hashimoto's disease; allergic diseases, such as food allergy, hay fever, or asthma; or *Clostridium difficile* infection. The inflammatory diseases include meningitis, myelitis, or TNF-mediated inflammatory diseases, such as inflammatory diseases of the gastrointestinal tract, such as pouchitis, cardiovascular inflammatory diseases, such as atherosclerosis, or inflammatory lung diseases, such as chronic obstructive pulmonary disease.

The bacterial strains and compositions thereof of the present disclosure are used for treating or preventing neurological diseases, such as depression, anxiety, post-traumatic stress disorder, obsessive-compulsive disorder, Parkinson's disease, encephalatrophy, vascular or arteriosclerotic Parkinson's disease, mild cognitive impairment, HIV-related cognitive impairment, or Alzheimer's disease (AD).

The composition involved in the present disclosure comprises a pharmaceutically acceptable carrier.

Preferably, the carrier is selected from one or more of a diluent, a dispersing agent, an excipient, a stabilizer, a lubricant, or a disintegrating agent.

The dosage form of the drug involved in the present disclosure includes any one of a liquid formulation, a solid formulation, a capsule formulation, a sustained-release formulation, and a nano-formulation.

The bacterial solution used in the animal experiments of the present disclosure can be administered by gavage according to the body weight of mice, in a dosage of 0.04-0.3 ml/10 g body weight, or 0.2 ml/mouse. The experiments conducted by the above gavage administration all show that the strains MNH05026, MNH22004, and MNH27256 of the present disclosure can be used alone or in combination with other drugs to achieve significant effects of improving liver and kidney functions and treating diseases such as diabetes, obesity, or fatty liver as described in the present disclosure.

The solid MM01 culture medium involved in the present disclosure comprises: peptone 5 g/L, trypsinized casein 5 g/L, yeast powder 10 g/L, beef extract 5 g/L, glucose 5 g/L, K₂HPO₄ 2 g/L, sodium acetate 2 g/L, Tween 80 1 mL/L, hemoglobin 5 mg/L, L-cysteine hydrochloride 0.5 g/L, vitamin K1 1 uL/L, an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution, and agar 15 g/L.

The liquid MM01 culture medium involved in the present disclosure comprises: peptone 5 g/L, trypsinized casein 5 g/L, yeast powder 10 g/L, beef extract 5 g/L, glucose 5 g/L, K₂HPO₄ 2 g/L, sodium acetate 2 g/L, Tween 80 1 mL/L, hemoglobin 5 mg/L, L-cysteine hydrochloride 0.5 g/L, vitamin K1 1 uL/L, and an inorganic salt solution 8 ml/L, which comprises 0.25 g of calcium chloride, 1 g of dipotassium hydrogen phosphate, 1 g of potassium dihydrogen phosphate, 0.5 g of magnesium sulfate, 10 g of sodium bicarbonate, and 2 g of sodium chloride per L of the inorganic salt solution.

The AC liquid medium comprises (per liter): peptone, 20 g; glucose, 5 g; yeast extract, 3 g; beef extract powder, 3 g; and vitamin C, 0.2 g; pH 7.0.

The anaerobic blood plate (purchased from Huankai Microbial) herein has the following formula (per liter): pancreatic digest of casein 10.0 g; cardiac pancreatic digest 3.0 g; corn starch 1.0 g; pepsin digest of meat 5.0 g; yeast extract powder 5.0 g; sodium chloride 5.0 g; agar 15.0 g; sterile defibrinated goat blood 50-100 mL; and distilled water 1000 mL; final pH 7.3±0.2.

The tryptic soytone broth (TSB liquid medium) herein (purchased from Huankai Microbial) comprises the following components (per liter): tryptic soytone 17.0 g; papain hydrolysate of soybean 3.0 g; dipotassium hydrogen phosphate 2.5 g; sodium chloride 5.0 g; and glucose 2.5 g; pH 7.3±0.2.

### Example 1. Isolation of the strains MNH05026, MNH22004, and MNH27256

The intestinal strain MNH05026 involved in the present disclosure was isolated from a stool sample from a healthy female volunteer in Guangzhou City, Guangdong Province. 2-5 g of fresh stool was collected by the donor and placed into a sample collection and storage tube. After being homogenized by shaking, the processed stool sample was placed in an ice box and sent to the laboratory within 24 hours for strain isolation. Physiological saline was dispensed in a biosafety cabinet, 9 mL/tube. Anaerobic blood agar plates with strain isolation medium (purchased from Huankai Microbial) were prepared and transferred to an anaerobic workstation 24 hours in advance. The sample information, type of culture medium, isolation date, etc. were marked.

The fresh stool sample was placed in an anaerobic operation station (Don Whitley Scientific H35), and mixed well by shaking on a vortex shaker for 1 min. 1 mL of the sample was pipetted into 9 mL of physiological saline, and mixed well to afford a 10⁻¹ diluted solution, which was then serially diluted to 10⁻⁶ dilution for later use.

The 10⁻⁶ diluted solution was dropped onto anaerobic blood agar plates with isolation medium in an amount of 100 µL/plate, and spread evenly. After the plate surface was dried, the plates were inverted and incubated at 37 °C for 3-5 days. The growth status of the strain in the isolation medium was observed, and a single colony was picked up with a sterilized toothpick for strain purification. The purified strain was anaerobically cultured at 37 °C. The pure culture strain was prepared into a bacterial solution in 20% glycerol or water, and stored at -86 °C. The strain was identified using a method based on 16S rRNA gene sequence identification.

The intestinal strain MNH22004 involved in the present disclosure was isolated from a stool sample from a healthy female volunteer in Guangzhou City, Guangdong Province. The strain MNH22004 was isolated by the above method.

The intestinal strain MNH27256 involved in the present disclosure was isolated from a stool sample from a healthy female volunteer in Guangzhou City, Guangdong Province. The strain MNH27256 was isolated by the above method.

Deposit of the strains: The strain MNH05026 (abbreviated as A026) was deposited on October 18, 2021 with the Guangdong Microbial Culture Collection Center with a deposit name of *Megasphaera* sp. MNH05026 and a deposit number of GDMCC No: 62001. The deposit address is Guangdong Institute of Microbiology, 5th Floor, No.59 Building, No. 100 Xianlie Zhong Road, Guangzhou. The proposed taxonomic name is *Megasphaera* sp.

The strain MNH22004 of the present disclosure was deposited on October 18, 2021 with the Guangdong Microbial Culture Collection Center with a deposit name of *Megasphaera* sp. MNH22004 and a deposit number of GDMCC NO: 62000. The deposit address is Guangdong Institute of Microbiology, 5th Floor, No.59 Building, No. 100 Xianlie Zhong Road, Guangzhou. The proposed taxonomic name is *Megasphaera* sp.

The strain MNH27256 of the present disclosure was deposited on October 18, 2021 with the Guangdong Microbial Culture Collection Center with a deposit name of *Megasphaera* sp. MNH27256 and a deposit number of GDMCC NO: 61999. The deposit address is Guangdong Institute of Microbiology, 5th Floor, No.59 Building, No. 100 Xianlie Zhong Road, Guangzhou. The proposed taxonomic name is *Megasphaera* sp.

Morphological features: After the strain MNH05026 (abbreviated as A026) was inoculated onto an anaerobic blood plate with culture medium and anaerobically cultured at 37 °C for 48 h, visible colonies were formed on the anaerobic blood plate with culture medium. The colonies were round, pale yellow, opaque, and about 1 mm in diameter, with regular and smooth edges, and with no secretion formed around the colonies. The strain was Gram-negative. Morphological observation under microscope showed that the strain had no spores, no flagella, was non-motile, spherical in shape, and had a diameter of about 5-10 µm. (see Figs. 1A-4A).

After the strain MNH22004 was inoculated onto an anaerobic blood plate with culture medium and anaerobically cultured at 37 °C for 24 h, visible colonies were formed on the anaerobic blood plate with culture medium. The colonies were round, yellow, opaque, and about 3 mm in diameter, with regular and smooth edges, and with no secretion formed around the colonies (see Fig. 1B for the colonial morphology). The strain was Gram-negative (see Fig. 3B for the microscopic morphology of the strain upon Gram-staining, and Fig. 4B for the microscopic morphology of the strain upon spore staining). Morphological observation under microscope showed that the strain had no spores, no flagella, was non-motile, short rod-shaped, and had a size of about 5 × 10-15 µm (see Fig. 2B for the microscopic colonial morphology).

After the strain MNH27256 was inoculated onto an anaerobic blood plate with culture medium and anaerobically cultured at 37 °C for 24 h, visible colonies were formed on the anaerobic blood plate with culture medium. The colonies were round, yellow, opaque, and about 2 mm in diameter, with regular and smooth edges, and with no secretion formed around the colonies (see Fig. 1C for the colonial morphology). The strain was Gram-negative (see Fig. 3C for the microscopic morphology of the strain upon Gram-staining, and Fig. 4C for the microscopic morphology of the strain upon spore staining). Morphological observation under microscope showed that the strain had no spores, no flagella, was non-motile, spherical in shape, and had a diameter of about 5 µm (see Figs. 2C and 2D for the microscopic colonial morphology).

Physiological and biochemical properties of the strains: The strain MNH05026 grew at a temperature ranging from 30 to 42 °C, with an optimal growth temperature of 37 °C. It could grow at pH 6.0-8.0, with an optimal growth pH of 6.0-7.0 (see Fig. 6A). It could tolerate up to 1% NaCl (see Fig. 5A). The strain MNH05026 could survive and grow at a bile salt concentration in the range of 0-0.15%, and could not grow at a bile salt concentration greater than or equal to 0.2% (see Fig. 7A). The strain MNH05026 could not grow under aerobic conditions, but grew well under anaerobic conditions, and thus belongs to obligate anaerobic bacteria.

The strain MNH22004 grew at a temperature ranging from 30 to 42 °C, with an optimal growth temperature of 37 °C. It could grow at pH 5.0-10.0, with an optimal growth pH of 7.0-8.0. It could tolerate up to 2% NaCl. The strain MNH22004 could survive and grow at a bile salt concentration in the range of 0-0.2%, but its growth trend was weakened as the bile salt concentration was increased. The strain MNH22004 could not grow under aerobic conditions, but grew well under anaerobic conditions, and thus belongs to obligate anaerobic bacteria. The tolerances of the strain to NaCl, pH, and bile salts are shown in Figs. 5B to 7B.

The strain MNH27256 grew at a temperature ranging from 30 to 42 °C, with an optimal growth temperature of 37 °C. It could grow at pH 5.0-10.0, with an optimal growth pH of 7.0-8.0. It could tolerate up to 2% NaCl. The strain MNH27256 could survive and grow at a bile salt concentration in the range of 0-0.4%, but its growth trend was weakened as the bile salt concentration was increased. The strain MNH27256 could not grow under aerobic conditions, but grew well under anaerobic conditions, and thus belongs to obligate anaerobic bacteria. The tolerances of the strain to NaCl, pH, and bile salts are shown in Figs. 5C to 7C.

Physiology and biochemistry of the strains by API 20A test: The test was performed using API 20A reagent strips (BioMérieux) according to the instructions. The strains were anaerobically cultured at 37 °C.

Experimental results: The strain MNH05026 could not grow on API 20A basal culture medium (see Fig. 8). The API 20A test results of MNH22004 and MNH27256 are shown in Table 2-A and Table 2-B, respectively.

**Table 2-A. API 20A test results of the strain MNH 22004**

| Test item | Test result | Test item | Test result |
|---|---|---|---|
| IND | Negative | ESC | Negative |
| URE | Negative | GLY | Non-acid producing |
| GLU | Acid-producing | CEL | Non-acid producing |
| MAN | Acid-producing | MNE | Non-acid producing |
| LAC | Non-acid producing | MLZ | Non-acid producing |
| SAC | Non-acid producing | RAF | Non-acid producing |
| MAL | Non-acid producing | SOR | Non-acid producing |
| SAL | Non-acid producing | RHA | Non-acid producing |
| XYL | Non-acid producing | TRE | Non-acid producing |
| ARA | Acid-producing | GEL | Negative |

**Table 2-B. API 20A test results of the strain MNH27256**

| Test item | Test result | Test item | Test result |
|---|---|---|---|
| IND | Negative | ESC | Negative |
| URE | Negative | GLY | Non-acid producing |
| GLU | Acid-producing | CEL | Non-acid producing |
| MAN | Acid-producing | MNE | Non-acid producing |
| LAC | Non-acid producing | MLZ | Non-acid producing |
| SAC | Non-acid producing | RAF | Non-acid producing |
| MAL | Non-acid producing | SOR | Non-acid producing |
| SAL | Non-acid producing | RHA | Non-acid producing |
| XYL | Non-acid producing | TRE | Non-acid producing |
| ARA | Acid-producing | GEL | Negative |

Antibiotic sensitivity tests of the strains MNH05026, MNH22004, and MNH27256: Antibiotic sensitivity tests were carried out on the strains MNH05026, MNH22004, and MNH27256 by a disk diffusion method. The test results are shown in Table 3. The strain MNH05026 was sensitive to antibiotics such as gentamicin, erythromycin, chloramphenicol, tetracycline, penicillin, ciprofloxacin, trimethoprim-sulfamethoxazole, ampicillin, lincomycin, and ceftriaxone; and the strain MNH05026 was not resistant to any of the antibiotics used. The strain MNH22004 was resistant to penicillin and ampicillin, and sensitive to antibiotics such as gentamicin, erythromycin, chloramphenicol, tetracycline, ciprofloxacin, trimethoprim-sulfamethoxazole, ceftriaxone, and lincomycin. The strain MNH27256 was resistant to penicillin, ampicillin and ceftriaxone, and sensitive to antibiotics such as gentamicin, erythromycin, chloramphenicol, tetracycline, ciprofloxacin, trimethoprim-sulfamethoxazole and lincomycin.

**Table 3. Results from Antibiotic sensitivity tests of the strains MNH05026, MNH22004, and MNH27256**

| Antibiotic | Diameter of inhibition zone by MNH05026 (mm) | Diameter of inhibition zone by MNH22004 (mm) | Diameter of inhibition zone by MNH27256 (mm) |
|---|---|---|---|
| Gentamicin (GEN) | 13.97 | 9.00 | 9.37 |
| Erythromycin (ERM) | 15.65 | 21.03 | 9.0 |
| Chloramphenicol (CLM) | 37.02 | 33.29 | 34.45 |
| Tetracycline (TET) | 21.44 | 10.48 | 17.75 |
| Penicillin (PEN) | 29.75 | 0 | 0 |
| Ciprofloxacin (CFX) | 37.66 | 35.42 | 34.69 |
| Trimethoprim-sulfamethoxazole (T/S) | 10.65 | 17.08 | 12.75 |
| Ampicillin (AMP) | 19.91 | 0 | 0 |
| Lincomycin (LIN) | 38.99 | 11.34 | 8.81 |
| Ceftriaxone (CTR) | 29.42 | 1 2.78 | 0 |

### Example 2. Identification of the strains MNH05026, MNH22004, and MNH27256

Genomic DNAs were extracted from fresh cultures of the strains MNH05026, MNH22004, and MNH27256. 16S rRNA gene amplification was performed using the extracted genomic DNAs of the strains as templates.

The primer pair used in the 16S rRNA gene PCR of the present disclosure was as follows:
27F: AGAGTTTGATCMTGGCTCAG, and 1492R: TACGGYTACCTTGTTACGACTT.

The PCR procedure was as follows: PCR reaction cycles: Pre-denaturation: 94 °C, 4 min; Denaturation: 94 °C, 50 sec; Annealing: 52 °C, 40 sec; Extension: 72 °C, 70 sec; Final extension: 72 °C, 10 min; 36 cycles.

After the PCR amplification was completed, the PCR products were purified and sent to Genewiz Co. for 16S rRNA gene sequencing. The 16S rRNA gene sequences of the strains returned after sequencing were submitted to the NCBI Basic Local Alignment Search Tool for 16S rRNA gene analysis to confirm the strain classification information.

The 16S rRNA gene amplification product of the strain MNH05026 was sent for sequencing to obtain the 16S rRNA gene sequence (SEQ ID NO: 1). The determined sequence was analyzed against the data in GenBank by BLAST. The alignment results showed that the strain MNH05026 belonged to the genus *Megasphaera,* and the strains with the highest similarity thereto were *Megasphaera hexanoica* (96.50%) and *Megasphaera elsdenii* (94.43%). The 16S rRNA gene sequence similarities between the strain MNH05026 and other strains of the genus *Megasphaera* were all less than 94.30%. According to Kim, *et al.* (2014), through statistical analysis of thousands of genomic and 16S rRNA gene sequences, it has been found that when the similarity between the 16S rRNA gene sequences of two strains is less than 98.65%, they can be judged to belong to different species. According to this judgment criterion, the experimental strain MNH05026 of the present disclosure can be significantly distinguished from other known strains of the genus *Megasphaera.* Therefore, the strain MNH05026 of the present disclosure represents a new species of the genus *Megasphaera.*

Based on the same analysis, the determined sequences of MNH22004 (SEQ ID NO: 2) and MNH27256 (SEQ ID NO: 3) were analyzed against the data in GenBank by BLAST. The alignment results showed that both MNH22004 (SEQ ID NO: 2) and MNH27256 (SEQ ID NO: 3) belonged to the genus *Megasphaera.* The strains with a higher similarity to MNH22004 were *Megasphaera micronuciformis* (90.92%), *Megasphaera massiliensis* (90.63%), *Megasphaera hexanoica* (90.63%), *Megasphaera elsdenii* (90.61%), *Megasphaera paucivorans* (90.59%), *Megasphaera indica* (90.29%), and *Megasphaera sueciensis* (90.18%). The 16S rRNA gene sequence similarities between the strain MNH22004 and other known strains of the genus *Megasphaera* were all less than 90%. The lower (<95%) 16S rRNA gene sequence similarity indicated that the strain MNH22004 might represent a new species of the genus *Megasphaera.* The strain with the highest similarity to MNH27256 was *Megasphaera stantonii* AJH120 (MG811574), and their 16S rRNA gene similarity was 98.41%. The 16S rRNA gene sequence similarities between the strain MNH27256 and other known strains of the genus *Megasphaera* were all less than 94%, indicating that MNH27256 might represent a new species of the genus *Megasphaera.*

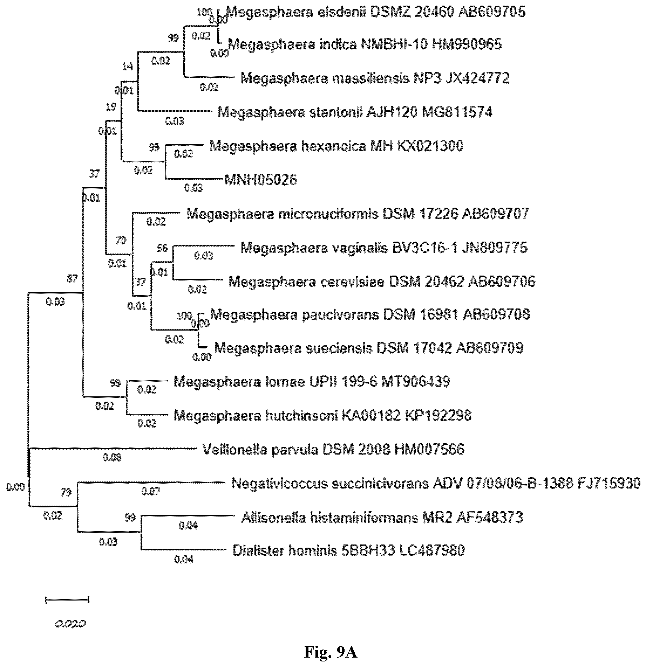

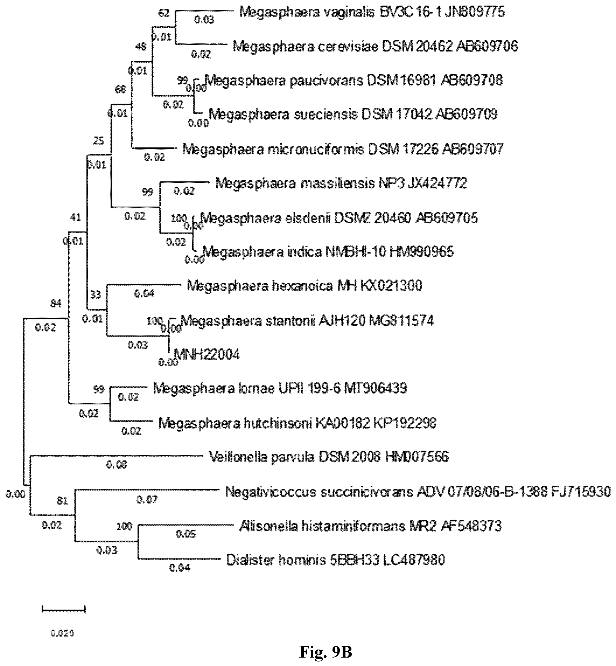

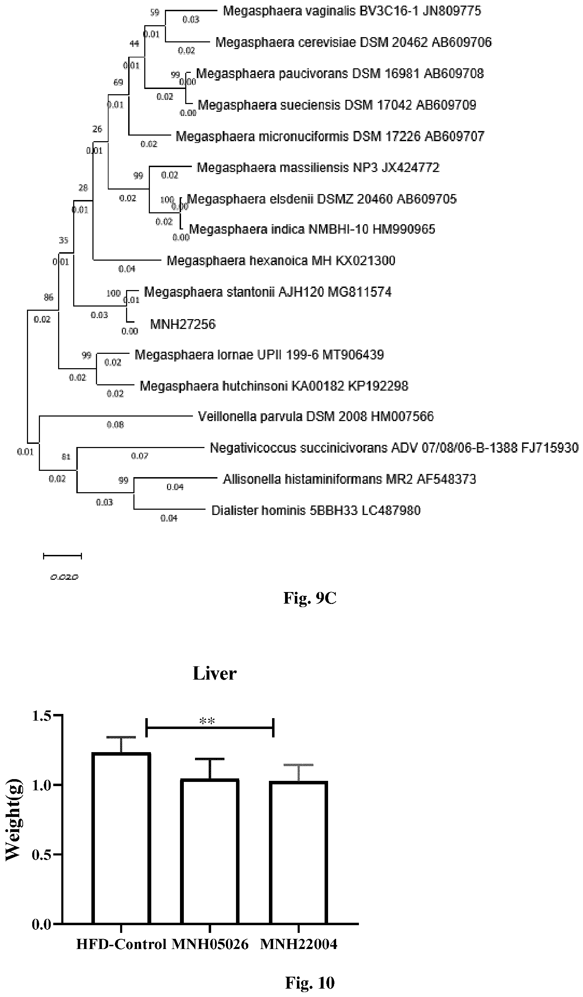

The 16S rRNA gene sequences of MNH05026, MNH22004, and MNH27256 were compared with those of related strains of the genus *Megasphaera* and closely related species retrieved from databases such as GenBank (Table 4). The whole-genome data of the strains *Megasphaera massiliensis* and *Megasphaera elsdenii* under the genus *Megasphaera* were downloaded from NCBI to compare with those of MNH05026, MNH22004, and MNH27256 of the present disclosure (Table 5). Moreover, phylogenetic trees were constructed. As can be seen from the phylogenetic trees, the strain MNH05026 and the *Megasphaera hexanoica* strain collectively form a separate branch (the Bootstrap support value is 99). The strain MNH22004 and the *Megasphaera stantonii* AJH120T strain collectively form a separate branch (the Bootstrap support value is 100). The strain MNH27256 and the *Megasphaera stantonii* AJH120 strain collectively form an evolutionary branch (the Bootstrap support value is 100). Multiple sequence alignment was performed between MNH05026, MNH 22004 and MNH 27256 and the type strains with a relatively high 16S rRNA gene sequence similarity obtained from the NCBI database, and then phylogenetic trees were constructed by the maximum likelihood method using the software MEGA 5. Fig. 9A shows the phylogenetic tree of MNH05026, Fig. 9B shows the phylogenetic tree of MNH22004, and Fig. 9C shows the phylogenetic tree of MNH27256. The phylogenetic tree nodes in the figures only display the Bootstrap values greater than 50%, and the superscript "T" indicates the type strains.

**Table 4. Comparison results of 16S rRNA gene sequences**

| 16S comparison results | | |
|---|---|---|
| | Reference sequence | Identity |
| MNH05026 | Megasphaera_elsdenii | 94.06 |
| MNH05026 | Megasphaera_massiliensis | 93.028 |
| MNH05026 | Megasphaera_massiliensis_PTA_126770 | 93.495 |
| MNH05026 | MNH22004 | 93.238 |
| MNH05026 | MNH27256 | 92.729 |
| MNH22004 | MNH27256 | 98.362 |

**Table 5. Pairwise comparison with Megasphaera elsdenii and Megasphaera massiliensis:**

| Whole-genome comparison results | | | |
|---|---|---|---|
| | Name of reference strain | Reference sequence | ANI |
| MNH05026 | A representative strain of Megasphaera_massiliensis | GCF_000455225.1_Megasphaera_ massiliensis_genomic.fna | 76.8594 |
| MNH05026 | A representative strain of *Megasphaera* elsdenii | GCF_003006415.1_ASM300641v 1_genomic.fna | 77.8759 |
| MNH05026 | *Megasphaera* elsdenii DSM 20460 | GCF_003010495.1_ASM301049v 1_genomic.fna | 77.6074 |
| MNH05026 | A randomly selected strain of Megasphaera_massiliensis | GCF_020181515.1_ASM2018151 v1_genomic.fna | 76.8902 |
| MNH22004 | A representative strain of Megasphaera_massiliensis | GCF_000455225.1_Megasphaera_ massiliensis_genomic.fna | 77.7799 |
| MNH22004 | A representative strain of *Megasphaera* elsdenii | GCF_003006215.1_ASM300641v 1_genomic.fna | 78.9286 |
| MNH22004 | Megasphaera elsdenii DSM 20460 | GCF_003010495.1_ASM301049v 1_genomic.fna | 78.9237 |
| MNH22004 | A randomly selected strain of Megasphaera_massiliensis | GCF_020181515.1_ASM2018151 v1_genomic.fna | 77.9763 |
| MNH27256 | A representative strain of Megasphaera_massiliensis | GCF_000455225.1_Megasphaera_ massiliensis_genomic.fna | 77.7611 |
| MNH27256 | A representative strain of Megasphaera elsdenii | GCF_003006415.1_ASM300641v 1_genomic.fna | 78.885 |
| MNH27256 | Megasphaera elsdenii DSM 20460 | GCF_003010495.1_ASM301049v 1_genomic.fna | 78.8788 |
| MNH27256 | A randomly selected strain of Megasphaera_massiliensis | GCF_020181515.1_ASM2018151 v1_genomic.fna | 77.9586 |
| MNH05026 | MNH27256 | / | 76.8949 |
| MNH05026 | MNH22004 | / | 76.8087 |
| MNH22004 | MNH27256 | / | 97.5967 |

According to Table 5, MNH05026, MNH22004, and MNH27256 are not strains belonging to *Megasphaera massiliensis* and *Megasphaera elsdenii,* and have significant genomic differences from *Megasphaera massiliensis* and *Megasphaera elsdenii.*

It can be further known from Table 4 and Table 5 that MNH05026 (SEQ ID NO: 1), MNH22004 (SEQ ID NO: 2), and MNH27256 (SEQ ID NO: 3) belong to the genus *Megasphaera.* According to the criterion that ANI > 95% indicates the same species, the genomic correlation analysis based on average nucleotide identity (ANI) in the present application shows that MNH05026, MNH22004, and MNH27256 belong to new species of the genus *Megasphaera,* and MNH22004 and MNH27256 belong to a species of the genus *Megasphaera,* and are two different strains of the same species.

Genomic Analysis of MNH05026, MNH22004, and MNH27256:
The genome of the original strain MNH05026 was fragmented by ultrasonication with a fragmentation length range of ~350 bp, and then an Illumina sequencing library was constructed using a standard DNA library construction kit (NEB UltraTM). The constructed sequencing library was subjected to paired-end 150 bp sequencing using NovaSeq (Illumina). The sequencing yielded 1.46 Gbp data, of which Q20 accounted for 97.08%. MNH22004 and MNH27256 were sequenced using the same method to obtain 1.33 Gbp and 1.39 Gbp data, respectively, of which Q20 accounted for 97.27% and 96.95%, respectively.

The raw data of genome sequencing were filtered using fastp (version: 0.20.0), with the following filtration parameter: "--poly_g_min_len 10 --poly_x_min_len 10 -q 15 -u 40 -n 5 -150". The filtered raw data were subjected to genome assembly using SPAdes (version: v3.14.0), with the following assembly parameter "--isolate --cov-cutoff 10". The genome assembly resulted in total gene lengths of 2.79 Mbp, 2.58 Mbp and 2.59 Mbp, N50 lengths of 59.8 kbp, 157.9 kbp and 92.9 kbp, and GC contents of 50.46%, 52.86% and 52.85% for MNH05026, MNH22004 and MNH27256, respectively.

Genomic genes were subjected to genomic gene prediction and analysis using the prokaryotic analysis software genome annotation process prokka (version: 1.14.5), with the following parameter: "--gcode 11 --evalue 1e-09". A total of 2568 CDS sequences were obtained for MNH05026 by the prediction, with an average CDS sequence length of 957 bp. The type strain with the highest genome similarity was *Megasphaera elsdenii,* with an average nucleotide identity (ANI) of 78.58% and a gene coverage of 30.27%. Therefore, it was identified as a new species of the genus *Megasphaera.* A total of 2353 CDS sequences were obtained for MNH22004 by the prediction, with an average CDS sequence length of 967 bp. The type strain with the highest genome similarity to the experimental strain MNH22004 was *Megasphaera elsdenii,* with an average nucleotide identity (ANI) of 79.49% and a gene coverage of 42.37%. Further based on the 16S rRNA gene analysis results of the strain MNH22004, it was confirmed that the strain MNH22004 might represent a new species of the genus *Megasphaera.* A total of 2335 CDS sequences were obtained for MNH27256 by the prediction, with an average CDS sequence length of 975 bp. The type strain with the highest genome similarity to the experimental strain MNH27256 was *Megasphaera elsdenii,* with an average nucleotide identity (ANI) of 79.38% and a gene coverage of 41.04%.

Potential antibiotic resistance genes in the genome were analyzed using the RGI process (version: 4.2.2), in which the antibiotic resistance gene database was CARD (version: 3.0.0, https://card.mcmaster.ca/analyze/rgi). No antibiotic resistance gene was identified for MNH05026. The drug resistance gene information obtained from alignment of MNH22004 and MNH27256 is shown in Table 6.

**Table 6. Information of drug resistance genes**

| Strain gene | Resistance gene | Gene Name | Alignment identity (%) |
|---|---|---|---|
| MNH22004_00592 | ARO:3004442 | tet(W/N/W) | 95.3 |
| MNH27256_01102 | ARO:3002837 | lnuC | 98.17 |

Potential virulence factors and related genes in the genome were analyzed by using NCBI blastp (version: 2.7.1+) to compare against the virulence factor database VFDB (http://www.mgc.ac.cn/cgi-bin/VFs/vS/main.cgi, updated on September 19, 2019). Detailed comparison results are shown in Table 7.

**Table 7. List of potential virulence genes of MNH05026, MNH22004, and MNH27256**

| Strain gene | VFDB gene | Gene Name | Alignment identity (%) |
|---|---|---|---|
| MNH05026_00068 | VFG001967 | glf | 61.345 |
| MNH05026_00534 | VFG011430 | acpXL | 66.667 |
| MNH05026_00865 | VFG048797 | ugd | 69.33 |
| MNH05026_01427 | VFG000077 | clpP | 66.492 |
| MNH05026_01568 | VFG001855 | htpB | 61.027 |
| MNH05026_02287 | VFG002377 | ddhA | 70.968 |
| MNH22004_00028 | VFG000077 | clpP | 65.426 |
| MNH22004_00201 | VFG001855 | htpB | 61.333 |
| MNH22004_01610 | VFG011430 | acpXL | 66.667 |
| MNH22004_02389 | VFG001967 | glf | 61.236 |
| MNH27256_00034 | VFG011430 | acpXL | 66.667 |
| MNH27256_00830 | VFG048797 | ugd | 69.33 |
| MNH27256_01638 | VFG000077 | clpP | 65.426 |
| MNH27256_02035 | VFG001855 | htpB | 61.333 |

Potential secondary metabolism gene clusters in the genome were analyzed using antiSMASH5 (version: 5.1.1). No secondary metabolism gene cluster was identified for MNH05026. The alignment results of MNH22004 and MNH05026 are shown in Table 8.

**Table 8. Potential secondary metabolism gene clusters of MNH22004 and MNH05026**

| Strain | Gene cluster range | Type | From | To | Most similar known gene cluster | Similarity |
|---|---|---|---|---|---|---|
| MNH22004 | Region 13.1 | sactipeptide | 71170 | 88519 | - | - |
| MNH27256 | Region 11.1 | sactipeptide | 67838 | 85557 | - | - |

Potential primary metabolism gene clusters in the genome were analyzed using gutSMASH5 (version: 1.0.0). Detailed alignment results are shown in Tables 9-A, 9-B, and 9-C.

**Table 9-A. List of potential primary metabolism gene clusters of MNH05026:**

| Gene cluster range | Type | From | To | Most similar known gene cluster | Abbrevi ation | Simila rity |
|---|---|---|---|---|---|---|
| Region 1.1 | Flavoenzyme_lipids_ catabolism | 18591 6 | 20953 0 | ---- | ---- | ---- |
| Region 2.1 | OD_fatty_acidsPFOR _II_pathway | 91245 | 12600 6 | PFOR II pathway B. thetaiotaomicron | PFORII | 100% |
| Region 4.1 | OD_AA_metabolism Putrescine2spermidin ePFOR_II_pathwayT PP_AA_metabolism | 1 | 60249 | Arginine2putrescine R. gnavus | PUTR | 100% |
| Region 6.1 | PFOR_II_pathway | 79302 | 92953 | PFOR II pathway B. thetaiotaomicron | PFORII | 100% |
| Region 9.1 | PFOR_II_pathway | 44299 | 67814 | PFOR II pathway B. thetaiotaomicron | PFORII | 100% |
| Region 14.1 | OD_fatty_acids | 40990 | 59886 | | | |
| Region 17.1 | PFOR_II_pathway | 32903 | 56181 | PFOR II pathway B. thetaiotaomicron | PFORII | 100% |
| Region 20.1 | porA | 1 | 15133 | porA C. sporogenes | POR | 40% |
| Region 26.1 | Others_HGD_unassig ned | 12019 | 36323 | ---- | ---- | ---- |
| Region 30.1 | OD_fatty_acids | 1763 | 30762 | ---- | ---- | ---- |
| Region 31.1 | PFOR_II_pathway | 1 | 15154 | PFOR II pathway B. thetaiotaomicron | PFORII | 100% |
| Region 48.1 | Rnf_complex | 1 | 15795 | Rnf complex C. sporogenes | RNF | 83% |
| Region 54.1 | OD_fatty_acids | 1 | 13291 | Aminobutyrate to butyrate C. pasteurianum | AMINO BUT | 20% |

**Table 9-B. Potential primary metabolism gene clusters of MNH22004:**

| Gene cluster range | Type | From | To | Most similar known gene cluster | Abbrevi ation | Similar ity |
|---|---|---|---|---|---|---|
| Region 1.1 | PFOR_II_pathway | 12515 0 | 14867 1 | PFOR II pathway B. thetaiotaomicron | PFORII | 100% |
| Region 2.1 | porA | 31221 | 53807 | porA C. sporogenes | POR | 60% |
| Region 3.1 | Flavoenzyme_lipids_cataboli sm | 22755 | 52907 | ---- | ---- | ---- |
| Region 4.1 | OD_fatty_acidsPFOR_II_pat hwayFlavoenzyme_lipids_ca tabolismacrylate2propionate Flavoenzyme_AA_peptides_ catabolism | 39306 | 88445 | Leucine reductive branch C. difficile | LEU | 50% |
| Region 6.1 | fatty_acids-unassigned | 6397 | 30453 | ---- | ---- | ---- |
| Region 6.2 | Rnf_complex | 12444 1 | 14951 4 | Rnf complex C. sporogenes | RNF | 100% |
| Region 6.3 | Putrescine2spermidineOthers _HGD_unassigned | 15417 2 | 17573 6 | ---- | ---- | ---- |
| Region 7.1 | TPP_AA_metabolism | 12493 0 | 14986 3 | ---- | ---- | ---- |
| Region 11.1 | proline2aminovalerate | 56222 | 80482 | Proline to aminovalerate C. sticklandii | AMI | 58% |
| Region 14.1 | OD_lactate_relatedPFOR_II _pathway | 21761 | 61320 | PFOR II pathway B. thetaiotaomicron | PFORII | 100% |
| Region 23.1 | Arginine2putrescine | 1 | 21392 | ---- | ---- | ---- |

**Table 9-C. Potential primary metabolism gene clusters of MNH27256:**

| Gene cluster range | Type | From | To | Most similar known gene cluster | Abbrevi ation | Similari ty |
|---|---|---|---|---|---|---|
| Region 1.1 | Others_HGD_unassig nedPutrescine2spermi dine | 46325 | 71732 | ---- | ---- | ---- |
| Region 1.2 | Rnf_complex | 76391 | 101464 | Rnf complex C. sporogenes | RNF | 100% |
| Region 2.1 | aminobutyrate2Butyra te | 93401 | 119769 | Aminobutyrate to butyrate C. pasteurianum | AMIN OBUT | 60% |
| Region 2.2 | OD_fatty_acids | 165601 | 201319 | PFOR II pathway B. thetaiotaomicron | PFORII | 100% |
| Region 4.1 | Flavoenzyme_lipids_ catabolism | 147516 | 177673 | ---- | ---- | ---- |
| Region 8.1 | TPP_AA_metabolism | 6896 | 31829 | ---- | ---- | ---- |
| Region 10.1 | OD_fatty_acidsPFOR _II_pathwayFlavoenz yme_lipids_catabolis macrylate2propionate Flavoenzyme_AA_pe ptides_catabolism | 16158 | 65296 | Leucine reductive branch C. difficile | LEU | 50% |
| Region 19.1 | PFOR_II_pathway | 1 | 19449 | PFOR II pathway B. thetaiotaomicron | PFORII | 100% |
| Region 25.1 | fatty_acids-unassigned | 9272 | 31951 | | | |
| Region 26.1 | PFOR_II_pathway | 18125 | 31920 | PFOR II pathway B. thetaiotaomicron | PFORII | 100% |
| Region 30.1 | Arginine2putrescine | 1 | 18883 | ---- | ---- | ---- |
| Region 32.1 | TPP_AA_metabolism | 1 | 17570 | Succinate to propionate B. theta | PRO | 16% |

The ability of the strains to produce butyric acid was evaluated. Using the genes related to the butyrate production pathway as described in the prior art (Vital M, Howe C, Tiedje M. Revealing the Bacterial Butyrate Synthesis Pathways by Analyzing (Meta) genomic Data[J]. Mbio, 2014, 5(2):1-11) as a reference database, the genome sequences of the strains were aligned against the reference database using NCBI blastp (version: 2.7.1+). Detailed alignment results are shown in Table 10. Then, the integrity of the butyrate production pathway was calculated. Through calculation, it was found that the integrity of the butyrate production pathway was 80% for the strain MNH05026, and 100% for MNH27256 and MNH22004.

**Table 10. List of potential butyrate-producing genes of MNH05026, MNH22004, and MNH27256:**

| Strain gene | Name of butyrate production pathway | Gene name | Alignment identity (%) |
|---|---|---|---|
| MNH05026_00402 | 4aminobutyrate | AbfD-Isom | 85.507 |
| MNH05026_00885 | 4aminobutyrate | AbfH | 78.919 |
| MNH05026_01758 | Pyruvate | Bcd | 85.827 |
| MNH05026_02471 | Pyruvate | But | 73.708 |
| MNH05026_01756 | Pyruvate | EtfA | 83.582 |
| MNH05026_01757 | Pyruvate | EtfB | 86.617 |
| MNH05026_00254 | Pyruvate | Hbd | 76.157 |
| MNH22004_00304 | 4aminobutyrate | AbfD-Isom | 90.476 |
| MNH22004_00694 | Pyruvate | Bcd | 87.566 |
| MNH22004_01262 | Pyruvate | But | 75.283 |
| MNH22004_01737 | Pyruvate | Cro | 80.385 |
| MNH22004_01359 | Pyruvate | EtfA | 84.615 |
| MNH22004_01358 | Pyruvate | EtfB | 88.015 |
| MNH22004_01736 | Pyruvate | Hbd | 83.566 |
| MNH22004_01042 | Pyruvate | Thl | 84.184 |
| MNH27256_00316 | 4aminobutyrate | 4Hbt | 81.628 |
| MNH27256_00317 | 4aminobutyrate | AbfD-Isom | 91.304 |
| MNH27256_01293 | Pyruvate | Bcd | 87.831 |
| MNH27256_01143 | Pyruvate | But | 75.51 |
| MNH27256_00660 | Pyruvate | Cro | 80.46 |
| MNH27256_00841 | Pyruvate | EtfA | 84.615 |
| MNH27256_00840 | Pyruvate | EtfB | 88.015 |
| MNH27256_01842 | Pyruvate | Hbd | 83.566 |
| MNH27256_02128 | Pyruvate | Thl | 83.929 |

Fatty acid composition analysis of the strains MNH05026, MNH22004, and MNH27256:
The strain MNH05026 (abbreviated as A026) was inoculated onto a TSA plate and anaerobically cultured at 37 °C for 48 h. Then, the bacterial cells were collected and subjected to fatty acid extraction and methylation treatment. A fatty acid composition analysis was performed for the strain MNH05026 using a fully automatic bacterial identification system from MIDI Co., US (Microbial ID, Inc., Newark, Del) (Kroppenstedt, 1985; Meier, *et al.,* 1993) (see Table 11A). MNH22004 and MNH27256 were analyzed using the same method, and the results are shown in Tables 11B and 11C, respectively.

The results showed that the main fatty acids (>10%) of MNH05026 were 12-carbon saturated fatty acid (C12:0, 11.98%) and 19-carbon unsaturated fatty acid (C18:1 CIS 9 FAME, 11.28%). Other fatty acids and their contents are detailed in Table 11A. The main fatty acids (>10%) of MNH22004 were 12-carbon saturated fatty acid (C12:0, 14.43%), 16-carbon saturated fatty acids (C16:0, 13.87%), 3-hydroxytetradecane saturated fatty acid (C14:0 3OH, 11.84%), and 18-carbon monounsaturated fatty acid (C18:1 CIS 9, 10.14%). Other fatty acids and their contents are detailed in Table 11B. The main fatty acids (>10%) of MNH27256 were 12-carbon saturated fatty acid (C12:0, 15.54%), 18-carbon monounsaturated fatty acid (C18:1 CIS 9, 15.03%), and 3-hydroxytetradecane saturated fatty acid (C14:0 3OH, 13.22%). Other fatty acids and their contents are detailed in Table 11C.

**Table 11A. Fatty acid components of the strain MNH05026:**

| RT | Response | Ar/Ht | RFact | ECL | Peak Name | Percent | Comment1 | Comment2 |
|---|---|---|---|---|---|---|---|---|
| 1.627 | 9.323E+8 | 0.044 | ---- | 6.974 | SOLVENT PEAK | ---- | < min rt | ---- |
| 2.093 | 1078 | 0.029 | ---- | 7.868 | ---- | ---- | < min rt | ---- |
| 2.587 | 1134 | 0.029 | ---- | 8.815 | ---- | ---- | < min rt | ---- |
| 3.162 | 500 | 0.024 | ---- | 9.917 | ---- | ---- | ---- | ---- |
| 3.206 | 412 | 0.025 | 1.155 | 10.000 | 10:0 FAME | 0.18 | ECL deviates 0.000 | Reference 0.003 |
| 3.864 | 1188 | 0.027 | ---- | 10.919 | ---- | ---- | ---- | ---- |
| 3.923 | 3853 | 0.031 | 1.098 | 11.000 | 11:0 FAME | 1.57 | ECL deviates 0.000 | Reference 0.002 |
| 4.497 | 363 | 0.031 | 1.069 | 11.609 | 12:0 ISO FAME | 0.14 | ECL deviates 0.001 | Reference 0.002 |
| 4.797 | 527 | 0.031 | ---- | 11.927 | ---- | ---- | ---- | ---- |
| 4.866 | 30691 | 0.033 | 1.052 | 11.999 | 12:0 FAME | 11.98 | ECL deviates -0.001 | Reference 0.001 |
| 5.589 | 1652 | 0.033 | 1.029 | 12.614 | 13:0 ISO FAME | 0.63 | ECL deviates 0.000 | Reference 0.000 |
| 6.043 | 4478 | 0.049 | 1.016 | 13.000 | 13:0 FAME | 1.69 | ECL deviates 0.000 | Reference 0.000 |
| 6.678 | 3712 | 0.038 | 1.002 | 13.455 | Sum In Feature 2 | 1.38 | ECL deviates -0.001 | 12:0 3OH FAME |
| 6.731 | 1234 | 0.034 | 1.001 | 13.493 | UN 13.493 | 0.46 | ECL deviates 0.000 | ---- |
| 7.111 | 505 | 0.033 | ---- | 13.766 | ---- | ----- | ---- | ---- |
| 7.377 | 2721 | 0.049 | ---- | 13.957 | ---- | ---- | ---- | ---- |
| 7.435 | 3691 | 0.038 | 0.987 | 13.999 | 14:0 FAME | 1.35 | ECL deviates -0.001 | Reference - 0.002 |
| 7.607 | 979 | 0.033 | 0.984 | 14.109 | 13:0 ISO 3OH FAME | 0.36 | ECL deviates -0.005 | ---- |
| 7.715 | 641 | 0.037 | ---- | 14.179 | ---- | ---- | ----- | ---- |
| 8.174 | 13808 | 0.043 | 0.976 | 14.472 | 14:0 DMA | 5.00 | ECL deviates 0.000 | Reference 0.000 |
| 8.410 | 774 | 0.036 | 0.973 | 14.623 | 15:0 ISO FAME | 0.28 | ECL deviates 0.000 | Reference 0.000 |
| 8.552 | 682 | 0.034 | 0.971 | 14.715 | 15:0 ANTEISO FAME | 0.25 | ECL deviates 0.001 | Reference 0.000 |
| 8.623 | 5508 | 0.050 | 0.970 | 14.760 | Sum In Feature 4 | 1.98 | ECL deviates -0.002 | UN 14.762 15:2 ? FA |
| 8.917 | 1491 | 0.044 | 0.966 | 14.948 | 16:0 ALDE | 0.53 | ECL deviates -0.003 | Reference - 0.004 |
| 8.999 | 6748 | 0.042 | 0.965 | 15.001 | 15:0 FAME | 2.42 | ECL deviates 0.001 | Reference 0.000 |
| 9.293 | 804 | 0.043 | ---- | 15.176 | ---- | ---- | ---- | ---- |
| 9.455 | 796 | 0.054 | ---- | 15.272 | ---- | ---- | ---- | ---- |
| 9.820 | 19989 | 0.044 | 0.957 | 15.489 | Sum In Feature 5 | 7.09 | ECL deviates 0.001 | 14:0 3OH FAME |
| 10.300 | 18255 | 0.043 | 0.953 | 15.775 | 16:1 CIS 7 FAME | 6.45 | ECL deviates 0.001 | - |
| 10.378 | 2246 | 0.054 | 0.952 | 15.821 | 16:1 CIS 9 FAME | 0.79 | ECL deviates 0.003 | - |
| 10.678 | 12367 | 0.043 | 0.949 | 16.000 | 16:0 FAME | 4.35 | ECL deviates 0.000 | Reference - 0.002 |
| 10.914 | 715 | 0.037 | 0.947 | 16.135 | 15:0 ISO 3OH FAME | 0.25 | ECL deviates 0.000 | ---- |
| 11.099 | 19618 | 0.044 | 0.946 | 16.241 | Sum In Feature 6 | 6.88 | ECL deviates 0.001 | 16:1 CIS 7 DMA |
| 11.369 | 1825 | 0.043 | ---- | 16.396 | ---- | ---- | ---- | ---- |
| 11.504 | 3401 | 0.040 | 0.943 | 16.474 | 16:0 DMA | 1.19 | ECL deviates 0.003 | Reference 0.001 |
| 11.558 | 5774 | 0.045 | 0.943 | 16.505 | 15:0 30H FAME | 2.02 | ECL deviates -0.001 | ---- |
| 11.777 | 1038 | 0.045 | 0.941 | 16.630 | 17:0 ISO FAME | 0.36 | ECL deviates 0.000 | Reference - 0.002 |
| 12.011 | 6902 | 0.042 | 0.940 | 16.765 | Sum In Feature 7 | 2.41 | ECL deviates 0.000 | 17:1 CIS 8 FAME |
| 12.063 | 9034 | 0.043 | 0.940 | 16.794 | Sum In Feature 8 | 3.15 | ECL deviates 0.000 | 17:1 CIS 9 FAME |
| 12.180 | 4788 | 0.051 | 0.939 | 16.862 | 17:1 CIS 11 FAME | 1.67 | ECL deviates -0.002 | ---- |
| 12.422 | 5798 | 0.045 | 0.938 | 17.001 | 17:0 FAME | 2.02 | ECL deviates 0.001 | Reference - 0.002 |
| 12.815 | 7183 | 0.045 | 0.935 | 17.223 | UN 17.223 | 2.49 | ECL deviates 0.000 | ---- |
| 12.870 | 5533 | 0.042 | 0.935 | 17.254 | 18:1 AT 17.254 DMA | 1.92 | ECL deviates 0.000 | ---- |
| 12.992 | 3520 | 0.049 | ---- | 17.323 | ---- | ---- | ---- | ---- |
| 13.252 | 2209 | 0.042 | 0.933 | 17.470 | 17:0 DMA | 0.76 | ECL deviates 0.001 | Reference - 0.002 |
| 13.699 | 1752 | 0.039 | 0.931 | 17.722 | 18:2 CIS 9,12 FAME | 0.60 | ECL deviates -0.001 | ---- |
| 13.787 | 32682 | 0.048 | 0.931 | 17.772 | 18:1 CIS 9 FAME | 11.28 | ECL deviates 0.001 | ---- |
| 13.882 | 2502 | 0.045 | 0.930 | 17.826 | Sum In Feature 10 | 0.86 | ECL deviates 0.002 | 18:1c11/t9/t6 FAME |
| 13.969 | 3364 | 0.054 | ---- | 17.875 | ---- | ---- | ---- | ---- |
| 14.188 | 7272 | 0.047 | 0.929 | 17.999 | 18:0 FAME | 2.51 | ECL deviates -0.001 | Reference - 0.004 |
| 14.586 | 16845 | 0.047 | 0.927 | 18.225 | 18:1 CIS 9 DMA | 5.79 | ECL deviates 0.001 | ---- |
| 14.703 | 1702 | 0.062 | 0.927 | 18.292 | 18:1 CIS 11 DMA | 0.59 | ECL deviates 0.007 | ---- |
| 14.871 | 897 | 0.039 | ---- | 18.388 | ---- | ---- | ---- | ---- |
| 15.520 | 833 | 0.043 | 0.924 | 18.757 | 19:1 CIS 7 FAME | 0.29 | ECL deviates 0.001 | ---- |
| 15.717 | 9656 | 0.051 | 0.923 | 18.870 | 19 CYC 9,10/:1 FAME | 3.31 | ECL deviates 0.000 | Reference - 0.003 |
| 16.504 | 2322 | 0.045 | 0.920 | 19.322 | 19:0 CYC 9,10 DMA | 0.79 | ECL deviates 0.000 | Reference - 0.004 |
| ---- | 3712 | ---- | ---- | ---- | Summed Feature 2 | 1.38 | 12:0 3OH FAME | 13:0 DMA |
| ---- | 5508 | ---- | ---- | ---- | Summed Feature 4 | 1.98 | UN 14.762 15:2 ? FA | 15:2 FAME |
| ---- | ---- | ---- | ---- | ---- | ---- | ---- | 15:1 CIS 7 | ---- |
| ---- | 19989 | ---- | ---- | ---- | Summed Feature 5 | 7.09 | 15:0 DMA | 14:0 3OH FAME |
| ---- | 19618 | ---- | ---- | ---- | Summed Feature 6 | 6.88 | 15:0 ANTEI 3OH FAME | 16:1 CIS 7 DMA |
| ---- | 6902 | ---- | ---- | ---- | Summed Feature 7 | 2.41 | 17:2 FAME @ 16.760 | 17:1 CIS 8 FAME |
| ---- | 9034 | ---- | ---- | ---- | Summed Feature 8 | 3.15 | 17:1 CIS 9 FAME | 17:2 FAME @ 16.801 |
| ---- | 2502 | ---- | ---- | ---- | Summed Feature 10 | 0.86 | 18:1c11/t9/t6 FAME | UN 17.834 |

**Table 11B. Fatty acid components of the strain MNH22004:**

| RT | Response | Ar/Ht | RFact | ECL | Peak Name | Percent | Comment1 | Comment2 |
|---|---|---|---|---|---|---|---|---|
| 1.626 | 9.42E+8 | 0.045 | ---- | 6.975 | SOLVENT PEAK | ---- | < min rt | ---- |
| 2.587 | 462 | 0.031 | ---- | 8.815 | ---- | ---- | < min rt | ---- |
| 2.659 | 245 | 0.023 | ---- | 8.954 | ---- | ---- | < min rt | ---- |
| 3.206 | 450 | 0.028 | 1.154 | 10.000 | 10:0 FAME | 0.20 | ECL deviates 0.000 | Reference 0.008 |
| 3.866 | 5764 | 0.028 | ---- | 10.921 | ---- | ---- | ---- | ---- |
| 3.924 | 382 | 0.029 | 1.099 | 11.000 | 11:0 FAME | 0.16 | ECL deviates 0.000 | Reference 0.006 |
| 4.499 | 906 | 0.038 | 1.071 | 11.609 | 12:0 ISO FAME | 0.37 | ECL deviates 0.001 | Reference 0.006 |
| 4.868 | 35459 | 0.033 | 1.054 | 11.999 | 12:0 FAME | 14.43 | ECL deviates -0.001 | Reference 0.004 |
| 5.591 | 1077 | 0.035 | 1.031 | 12.613 | 13:0 ISO FAME | 0.43 | ECL deviates -0.001 | Reference 0.003 |
| 5.696 | 629 | 0.034 | 1.028 | 12.703 | 13:0 ANTEISO FAME | 0.25 | ECL deviates 0.000 | Reference 0.003 |
| 6.680 | 2443 | 0.035 | 1.004 | 13.454 | Sum In Feature 2 | 0.95 | ECL deviates -0.002 | 12:0 3OH FAME |
| 7.380 | 4724 | 0.050 | ---- | 13.957 | ---- | ---- | ---- | ---- |
| 7.438 | 2547 | 0.036 | 0.989 | 13.999 | 14:0 FAME | 0.97 | ECL deviates -0.001 | Reference 0.001 |
| 7.611 | 481 | 0.034 | 0.986 | 14.109 | 13:0 ISO 3OH FAME | 0.18 | ECL deviates -0.005 | |
| 8.179 | 1425 | 0.032 | 0.978 | 14.473 | 14:0 DMA | 0.54 | ECL deviates 0.001 | Reference 0.003 |
| 8.227 | 2614 | 0.039 | ---- | 14.504 | ---- | ---- | ---- | ---- |
| 8.413 | 477 | 0.034 | 0.975 | 14.623 | 15:0 ISO FAME | 0.18 | ECL deviates 0.000 | Reference 0.001 |
| 8.626 | 6176 | 0.043 | 0.972 | 14.759 | Sum In Feature 4 | 2.32 | ECL deviates -0.003 | UN 14.762 15:2 ? FA |
| 8.922 | 3131 | 0.046 | 0.968 | 14.949 | 16:0 ALDE | 1.17 | ECL deviates -0.002 | Reference - 0.001 |
| 9.002 | 1305 | 0.039 | 0.967 | 15.000 | 15:0 FAME | 0.49 | ECL deviates 0.000 | Reference 0.001 |
| 9.299 | 1747 | 0.047 | ---- | 15.177 | ---- | ---- | ---- | ---- |
| 9.823 | 32013 | 0.043 | 0.958 | 15.489 | Sum In Feature 5 | 11.84 | ECL deviates 0.001 | 14:0 3OH FAME |
| 10.304 | 20523 | 0.042 | 0.953 | 15.775 | 16:1 CIS 7 FAME | 7.55 | ECL deviates 0.001 | |
| 10.681 | 37823 | 0.042 | 0.950 | 15.999 | 16:0 FAME | 13.87 | ECL deviates -0.001 | Reference - 0.001 |
| 10.916 | 655 | 0.035 | 0.948 | 16.134 | 15:0 ISO 3OH FAME | 0.24 | ECL deviates -0.001 | |
| 11.101 | 26265 | 0.043 | 0.947 | 16.240 | Sum In Feature 6 | 9.60 | ECL deviates 0.000 | 16:1 CIS 7 DMA |
| 11.369 | 1164 | 0.039 | ---- | 16.394 | ---- | ---- | ---- | ---- |
| 11.506 | 16019 | 0.044 | 0.944 | 16.473 | 16:0 DMA | 5.84 | ECL deviates 0.002 | Reference 0.001 |
| 11.780 | 2915 | 0.042 | 0.942 | 16.630 | 17:0 ISO FAME | 1.06 | ECL deviates 0.000 | Reference 0.000 |
| 12.011 | 4053 | 0.054 | 0.940 | 16.762 | Sum In Feature 7 | 1.47 | ECL deviates 0.002 | 17:2 FAME @ 16.760 |
| 12.178 | 2460 | 0.049 | 0.939 | 16.859 | 17:1 CIS 11 FAME | 0.89 | ECL deviates -0.005 | |
| 12.426 | 2893 | 0.047 | 0.937 | 17.001 | 17:0 FAME | 1.05 | ECL deviates 0.001 | Reference 0.000 |
| 12.611 | 866 | 0.035 | 0.936 | 17.106 | UN 17.103 17:0i DMA | 0.31 | ECL deviates 0.002 | Reference 0.001 |
| 12.814 | 1112 | 0.052 | 0.935 | 17.220 | UN 17.223 | 0.40 | ECL deviates -0.003 | ---- |
| 12.995 | 2726 | 0.043 | ---- | 17.323 | ---- | ---- | ---- | ---- |
| 13.146 | 309 | 0.034 | ---- | 17.408 | ---- | ---- | ---- | ---- |
| 13.254 | 723 | 0.036 | 0.933 | 17.469 | 17:0 DMA | 0.26 | ECL deviates 0.000 | Reference - 0.001 |
| 13.705 | 1834 | 0.042 | 0.930 | 17.724 | 18:2 CIS 9,12 FAME | 0.66 | ECL deviates 0.001 | ---- |
| 13.789 | 28244 | 0.047 | 0.930 | 17.772 | 18:1 CIS 9 FAME | 10.14 | ECL deviates 0.001 | ---- |
| 13.889 | 1815 | 0.064 | 0.930 | 17.829 | Sum In Feature 10 | 0.65 | ECL deviates 0.005 | 18:1c11/t9/t6 FAME |
| 14.192 | 8808 | 0.048 | 0.928 | 18.000 | 18:0 FAME | 3.16 | ECL deviates 0.000 | Reference - 0.002 |
| 14.589 | 15537 | 0.048 | 0.926 | 18.225 | 18:1 CIS 9 DMA | 5.56 | ECL deviates 0.001 | ---- |
| 15.011 | 1177 | 0.045 | 0.925 | 18.466 | 18:0 DMA | 0.42 | ECL deviates 0.000 | ---- |
| 15.721 | 5253 | 0.045 | 0.922 | 18.869 | 19 CYC 9,10/:1 FAME | 1.87 | ECL deviates -0.001 | Reference - 0.003 |
| 16.507 | 1468 | 0.039 | 0.919 | 19.321 | 19:0 CYC 9,10 DMA | 0.52 | ECL deviates -0.001 | Reference - 0.003 |
| ---- | 2443 | ---- | ---- | ---- | Summed Feature 2 | 0.95 | 12:0 3OH FAME | 13:0 DMA |
| ---- | 6176 | ---- | ---- | ---- | Summed Feature 4 | 2.32 | UN 14.762 15:2 ? FA | 15:2 FAME |
| ---- | ---- | ---- | ---- | ---- | | ---- | 15:1 CIS 7 | |
| ---- | 32013 | ---- | ---- | ---- | Summed Feature 5 | 11.84 | 15:0 DMA | 14:0 3OH FAME |
| ---- | 26265 | ---- | ---- | ---- | Summed Feature 6 | 9.60 | 15:0 ANTEI 3OH FAME | 16:1 CIS 7 DMA |
| ---- | 4053 | ---- | ---- | ---- | Summed Feature 7 | 1.47 | 17:2 FAME @ 16.760 | 17:1 CIS 8 FAME |
| ---- | 1815 | ---- | ---- | ---- | Summed Feature 10 | 0.65 | 18:1c11/t9/t6 FAME | UN 17.834 |

**Table 11C. Fatty acid components of the strain MNH27256:**

| RT | Response | Ar/Ht | RFact | ECL | Peak Name | Percent | Comment1 | Comment2 |
|---|---|---|---|---|---|---|---|---|
| 1.626 | 9.359E+8 | 0.044 | ---- | 6.976 | SOLVENT PEAK | ---- | < min rt | ---- |
| 2.285 | 286 | 0.035 | ---- | 8.235 | | ---- | < min rt | ---- |
| 2.589 | 353 | 0.030 | ---- | 8.818 | | ---- | < min rt | ---- |
| 2.656 | 290 | 0.025 | ---- | 8.947 | | ---- | < min rt | ---- |
| 3.208 | 484 | 0.038 | 1.154 | 10.002 | 10:0 FAME | 0.31 | ECL deviates 0.002 | Reference 0.009 |
| 3.866 | 5035 | 0.029 | ---- | 10.921 | | ---- | | |
| 3.921 | 288 | 0.022 | 1.099 | 10.997 | 11:0 FAME | 0.18 | ECL deviates - 0.003 | Reference 0.003 |
| 4.499 | 634 | 0.032 | 1.071 | 11.610 | 12:0 ISO FAME | 0.38 | ECL deviates 0.002 | Reference 0.006 |
| 4.868 | 26470 | 0.032 | 1.054 | 12.000 | 12:0 FAME | 15.54 | ECL deviates 0.000 | Reference 0.004 |
| 5.590 | 681 | 0.032 | 1.031 | 12.614 | 13:0 ISO FAME | 0.39 | ECL deviates 0.000 | Reference 0.003 |
| 5.694 | 510 | 0.034 | 1.028 | 12.702 | 13:0 ANTEISO FAME | 0.29 | ECL deviates - 0.001 | Reference 0.001 |
| 6.680 | 2554 | 0.037 | 1.004 | 13.456 | Sum In Feature 2 | 1.43 | ECL deviates 0.000 | 12:0 3OH FAME |
| 7.380 | 3814 | 0.052 | ---- | 13.958 | ---- | ---- | ---- | ---- |
| 7.439 | 881 | 0.032 | 0.989 | 14.000 | 14:0 FAME | 0.49 | ECL deviates 0.000 | Reference 0.001 |
| 7.611 | 460 | 0.041 | 0.986 | 14.111 | 13:0 ISO 3OH FAME | 0.25 | ECL deviates - 0.003 | ---- |
| 8.226 | 3873 | 0.051 | ---- | 14.505 | ---- | ---- | ---- | ---- |
| 8.624 | 4170 | 0.040 | 0.972 | 14.760 | Sum In Feature 4 | 2.26 | ECL deviates - 0.002 | UN 14.762 15:2 ? FA |
| 8.918 | 2480 | 0.044 | 0.968 | 14.948 | 16:0 ALDE | 1.34 | ECL deviates - 0.003 | Reference - 0.003 |
| 9.002 | 521 | 0.035 | 0.967 | 15.002 | 15:0 FAME | 0.28 | ECL deviates 0.002 | Reference 0.001 |
| 9.299 | 1274 | 0.037 | ---- | 15.178 | ---- | ---- | ---- | ---- |
| 9.824 | 24781 | 0.043 | 0.958 | 15.490 | Sum In Feature 5 | 13.22 | ECL deviates 0.002 | 14:0 3OH FAME |
| 10.305 | 10863 | 0.044 | 0.953 | 15.776 | 16:1 CIS 7 FAME | 5.77 | ECL deviates 0.002 | ---- |
| 10.680 | 17718 | 0.044 | 0.950 | 16.000 | 16:0 FAME | 9.37 | ECL deviates 0.000 | Reference - 0.001 |
| 10.918 | 576 | 0.040 | 0.948 | 16.136 | 15:0 ISO 3OH FAME | 0.30 | ECL deviates 0.001 | ----- |
| 11.102 | 14794 | 0.043 | 0.947 | 16.241 | Sum In Feature 6 | 7.80 | ECL deviates 0.001 | 16:1 CIS 7 DMA |
| 11.370 | 745 | 0.039 | ---- | 16.395 | ---- | ---- | ---- | ---- |
| 11.505 | 8635 | 0.047 | 0.944 | 16.473 | 16:0 DMA | 4.54 | ECL deviates 0.002 | Reference 0.001 |
| 11.782 | 1583 | 0.045 | 0.942 | 16.631 | 17:0 ISO FAME | 0.83 | ECL deviates 0.001 | Reference 0.000 |
| 12.012 | 3309 | 0.046 | 0.940 | 16.764 | Sum In Feature 7 | 1.73 | ECL deviates - 0.001 | 17:1 CIS 8 FAME |
| 12.181 | 1606 | 0.054 | 0.939 | 16.860 | 17:1 CIS 11 FAME | 0.84 | ECL deviates - 0.004 | ---- |
| 12.423 | 2003 | 0.043 | 0.937 | 17.000 | 17:0 FAME | 1.05 | ECL deviates 0.000 | Reference - 0.002 |
| 12.608 | 702 | 0.047 | 0.936 | 17.104 | UN 17.103 17:0i DMA | 0.37 | ECL deviates 0.000 | Reference - 0.001 |
| 12.817 | 514 | 0.039 | 0.935 | 17.222 | UN 17.223 | 0.27 | ECL deviates - 0.001 | ---- |
| 12.997 | 1784 | 0.045 | ---- | 17.324 | ---- | ---- | ---- | ---- |
| 13.150 | 474 | 0.043 | ---- | 17.410 | ---- | ---- | ---- | ---- |
| 13.256 | 650 | 0.040 | 0.933 | 17.470 | 17:0 DMA | 0.34 | ECL deviates 0.001 | Reference 0.000 |
| 13.790 | 29023 | 0.047 | 0.930 | 17.772 | 18:1 CIS 9 FAME | 15.03 | ECL deviates 0.001 | |
| 13.885 | 1148 | 0.052 | 0.930 | 17.826 | Sum In Feature 10 | 0.59 | ECL deviates 0.002 | 18:1c11/t9/t6 FAME |
| 14.194 | 6143 | 0.046 | 0.928 | 18.000 | 18:0 FAME | 3.17 | ECL deviates 0.000 | Reference - 0.001 |
| 14.588 | 15167 | 0.047 | 0.926 | 18.224 | 18:1 CIS 9 DMA | 7.82 | ECL deviates 0.000 | ---- |
| 14.877 | 480 | 0.037 | ---- | 18.388 | ---- | ---- | ---- | ---- |
| 15.016 | 1256 | 0.048 | 0.925 | 18.467 | 18:0 DMA | 0.65 | ECL deviates 0.001 | ---- |
| 15.722 | 4922 | 0.050 | 0.922 | 18.869 | 19 CYC 9,10/:1 FAME | 2.53 | ECL deviates - 0.001 | Reference - 0.002 |
| 16.510 | 1310 | 0.039 | 0.919 | 19.321 | 19:0 CYC 9,10 DMA | 0.67 | ECL deviates - 0.001 | Reference - 0.002 |
| ---- | 2554 | ---- | ---- | ---- | Summed Feature 2 | 1.43 | 12:0 3OH FAME | 13:0 DMA |
| ---- | 4170 | ---- | ---- | ---- | Summed Feature 4 | 2.26 | UN 14.762 15:2 ? FA | 15:2 FAME |
| ---- | ----- | ---- | ---- | ---- | | ---- | 15:1 CIS 7 | ---- |
| ---- | 24781 | ---- | ---- | ---- | Summed Feature 5 | 13.22 | 15:0 DMA | 14:0 3OH FAME |
| ---- | 14794 | ---- | ---- | ---- | Summed Feature 6 | 7.80 | 15:0 ANTEI 3OH FAME | 16:1 CIS 7 DMA |
| ---- | 3309 | ---- | ---- | ---- | Summed Feature 7 | 1.73 | 17:2 FAME @ 16.760 | 17:1 CIS 8 FAME |
| ---- | 1148 | ---- | ---- | ---- | Summed Feature 10 | 0.59 | 18:1c11/t9/t6 FAME | UN 17.834 |

According to the morphological, microscopic, physiological and biochemical characteristics, 16S rRNA gene sequence and genomic information, the strain MNH05026 belonged to a new species of the genus *Megasphaera,* and was temporarily named *Megasphaera* sp. MNH05026 (the patent strain deposit number was GDMCC No: 62001). The strains MNH22004 and MNH27256 were different strains of the same new species of the genus *Megasphaera,* and were temporarily named *Megasphaera* sp. MNH22004 (the patent strain deposit number was GDMCC NO: 62000) and *Megasphaera* sp. MNH27256 (the patent strain deposit number was GDMCC NO: 61999).

### Example 3. Short-chain fatty acid (SCFA) assay:

The assay was described with MNH05026 as an example. MNH22004 and MNH27256 were measured by the same method.

Preparation of bacterial cells: The strain MNH05026 was inoculated into TSB liquid medium, anaerobically cultured at 37 °C for 48 hours, centrifuged to collect the bacterial cells, which were stored at -86 °C for later use.

Formulation of standards: Acetic acid, propionic acid, butyric acid, isobutyric acid, valeric acid, isovaleric acid, and hexanoic acid standards were weighed, and formulated with ethyl acetate to form eight mixed standard concentration gradients: 0.1 µg/mL, 0.5 µg/mL, 1 µg/mL, 5 µg/mL, 10 µg/mL, 20 µg/mL, 50 µg/mL, and 100 µg/mL. To 600 µL of the standard was added 25 µL of 4-methylvaleric acid to a final concentration of 500 µM as an internal standard, mixed well, and charged into an injection bottle for GC-MS detection. The injection volume was 1 µL, and the split ratio was 10:1. Split injection was used.

Extraction of metabolites: The sample was thawed on ice. 80 mg of the sample was pipetted into a 2 mL glass centrifuge tube, resuspended in 900 µL of 0.5% phosphoric acid, mixed well by shaking for 2 min, and centrifuged at 14,000 g for 10 min. 800 µL of the supernatant was pipetted, to which an equal amount of ethyl acetate was added for extraction, mixed well by shaking for 2 min, and centrifuged at 14,000 g for 10 min. 600 µL of the upper organic phase was pipetted, to which 4-methylvaleric acid was added to a final concentration of 500 µM as an internal standard, mixed well, and charged into an injection bottle for GC-MS detection. The injection volume was 1 µL, and the split ratio was 10:1. Split injection was used.

Sample detection and analysis: The sample was separated on an Agilent DB-WAX capillary column (30 m × 0.25 mm ID × 0.25 µm) gas chromatography system. Temperature programming: the initial temperature was 90 °C, raised to 120 °C at 10 °C/min, then to 150 °C at 5 °C/min, finally to 250 °C at 25 °C/min, and maintained for 2 min. The carrier gas was helium, with a flow rate of 1.0 mL/min.

Mass spectrometry analysis was performed on an Agilent 7890A/5975C gas chromatograph-mass spectrometer. The inlet temperature was 250 °C, the ion source temperature was 230 °C, the transmission line temperature was 250 °C, and the quadrupole temperature was 150 °C. An electron impact ionization (EI) source was used in full-scan and SIM scanning modes with an electron energy of 70 eV.

The chromatographic peak area and retention time were extracted using the MSD ChemStation software. A standard curve was plotted to calculate the content of short-chain fatty acids in the sample (see Table 12).

**Table 12. Results of short-chain fatty acid (SCFA) yields**

| SCFA Type | Acetic acid | Propionic acid | Isobutyric acid | Butyric acid | Isovaleric acid | Valeric acid | Hexanoic acid |
|---|---|---|---|---|---|---|---|
| MNH05026 Yield (ug/g) | 251.21 | 42.85 | 120.81 | 214.75 | 248.96 | 59.36 | 366.76 |
| MNH22004 Yield (µg/g) | 463.32 | 104 | 183.48 | 920.31 | 320.43 | 3.52 | 0.58 |
| MNH27256 Yield (µg/g) | 407.39 | 119.21 | 198.17 | 1165.04 | 329.46 | 4.53 | 0.28 |

**Conclusion:** The strain MNH05026 can synthesize a large amount of hexanoic acid, acetic acid, isovaleric acid, butyric acid, and isobutyric acid, and a small amount of valeric acid and propionic acid during its growth. The strain MNH22004 can synthesize a large amount of acetic acid, propionic acid, isovaleric acid, butyric acid, and isobutyric acid, especially a relatively larger amount of butyric acid; and a small amount or very little amount of hexanoic acid and valeric acid during its growth. The strain MNH27256 can synthesize a large amount of acetic acid, propionic acid, isovaleric acid, butyric acid, and isobutyric acid, especially a relatively larger amount of butyric acid; and a small amount or very little amount of hexanoic acid and valeric acid during its growth. The term "highly productive" as used herein means that the yield is not less than 200 ug/g. Therefore, it can be determined that MNH05026, MNH22004, and MNH27256 of the present application are highly productive of acetic acid and/or butyric acid.

In the present disclosure, the term "highly productive of acetic acid and/or butyric acid" means that the yields of acetic acid and/or butyric acid in the short-chain fatty acid (SCFA) assay is ≥ 200 µg/g. Preferably, the yield of acetic acid is ≥ 250 ug/g; preferably, the yield of acetic acid is ≥ 300 µg/g; preferably, the yield of acetic acid is ≥ 400 µg/g; and/or, preferably, the yield of butyric acid is ≥ 300 µg/g, ≥ 400 µg/g, ≥ 500 µg/g, ≥ 600 µg/g, ≥ 700 µg/g, ≥ 800 µg/g, ≥ 900 µg/g, ≥ 1000 µg/g, ≥ 1100 µg/g, ≥ 1200 µg/g, ≥ 1300 µg/g, ≥ 1400 µg/g, ≥ 1500 µg/g, or ≥ 1600 µg/g.

### Example 4: Method for preparing bacterial cells for cell screening platform

The method was described with MNH05026 as an example. Bacterial cells of MNH22004 and MNH27256 were prepared by the same method.

A single colony of MNH05026 was picked up with a sterilized toothpick into 10 ml of AC liquid medium (comprising (per liter): peptone, 20 g; glucose, 5 g; yeast extract, 3 g; beef extract powder, 3 g; and vitamin C, 0.2 g; pH 7.0), and anaerobically cultured at 37 °C for 1 day. 1 ml of the culture was sampled for mass spectrometry. After the identification result was correct, 2 ml of the bacterial suspension was pipetted into 80 ml of AC liquid medium and cultured at 37 °C in an anaerobic operation station. After culturing for 24 hours, 30 ml of the bacterial suspension was pipetted into 400 ml of AC liquid medium and cultured at 37 °C in an anaerobic operation station. After culturing for 24 hours, 1 ml of the bacterial solution was sampled for mass spectrometry. After the identification result was correct, the bacterial solution was wholly transferred to a 1 L centrifuge bottle and centrifuged at 6000 rpm and 4 °C for 30 min. The precipitate was resuspended in AC liquid medium : sterile glycerol (4:1).

0.1 ml of a stock solution of the MNH05026 bacterial solution with glycerol was pipetted into 0.9 ml of physiological saline for serial dilution to 10⁻⁸ gradient. 100 µl of the diluted solutions at concentrations of 10⁻⁶, 10⁻⁷, and 10⁻⁸ were pipetted onto anaerobic blood plates, respectively. Glass beads were added and shaken well. Viable bacterial count (CFU) was determined using the plate counting method.

The prepared stock solution of the strain was dispensed into sterile cryovials, with 0.2 ml of the bacterial solution per vial and 10 vials for each strain. They were cryopreserved at -80 °C. After being completely frozen (24 h), a vial of frozen bacteria was removed and thawed to room temperature for later use. Viable bacterial count (CFU) was measured using the diluting and spreading method.

### Example 5. In vivo experiment of the strains MNH05026 and MNH22004 on liver and kidney functions and related diseases in a high-fat diet-induced obesity mouse model

An experiment on the amelioration of liver and kidney functions and related diseases by MNH05026 was conducted using a high-fat diet-induced obesity mouse model. This experiment has been ethically reviewed by the Laboratory Animal Care and Use Committee of Moon Biotech.
1) Experimental animals: C57BL/6J mice, aged 5-6 weeks, 20 mice in total, purchased from Guangdong GemPharmatech Co., Ltd.
2) Test strain: A glycerol cryovial of the strain MNH05026 was thawed at 37 °C, and then inoculated onto an MM01 plate in an anaerobic workstation for activation. The activated strain was inoculated into MM01 liquid medium and anaerobically cultured to obtain a sufficient amount of cultures. The cultured bacterial solution was centrifuged, concentrated, and then resuspended in a solvent to afford a test article with a purity and viable bacterial count (2.9 × 10⁹ CFU/mL) meeting the requirements of animal experiments.
3) Negative control: PBS-Cys containing 25% glycerol
4) Experimental procedure: Upon completion of the quarantine period, male C57BL/6J mice aged 5-6 weeks were fed with a high-fat diet for 10 weeks. 16 mice with a body weight ranging from 35.50 g to 44.49 g were selected and randomized into 2 groups of 8 animals each (an experimental group and a control group) according to body weight. Gavage administration was initiated after the grouping (D1). The experimental group was administered with the MNH05026 bacterial solution in an amount of 0.2 mL/animal, while the control group was given the negative control, twice a day, for a total of 21 days. During the experiment, the mice were given *ad libitum* access to water and food under 12 h/12 h light/dark cycle. During the experiment, one general clinical observation was conducted after each virtual dosing or the end of dosing. The animals were weighed on each Monday and Thursday during the dosing period, on the day of initial dosing (D1), on Day 21 of dosing (D21), and before dissection at the experimental endpoint. The endpoint of this experiment was the day following the end of dosing (D22). The animals were dissected and sampled according to the protocol at the experimental endpoint, and the data were summarized to analyze the body weight, the percent change in body weight, the fasting blood glucose measurement results, various anatomical data results, and serum test data results. All data were expressed as mean ± SD, and plotted and statistically analyzed using the GraphPad Prism 8.0.2 software. For pairwise comparisons, t-test (Student's t test) was used. It was not indicated if there was no significance; and the significance of difference was indicated by *: * p < 0.05, ** p < 0.01, *** p < 0.001, **** p < 0.0001.

Relevant experiments were conducted for MNH22004 using the same methods as in 1) to 4). The experimental results are shown in Fig. 10.

Fig. 10 is a graph showing the effects of the strains MNH05026 and MNH22004 on liver weight in high-fat diet-induced obesity mice.

The results in Fig. 10 showed that MNH05026 and MNH22004 significantly reduced liver weight, with comparable effects, indicating that both MNH05026 and MNH22004 can significantly improve fatty liver caused by a high-fat diet.

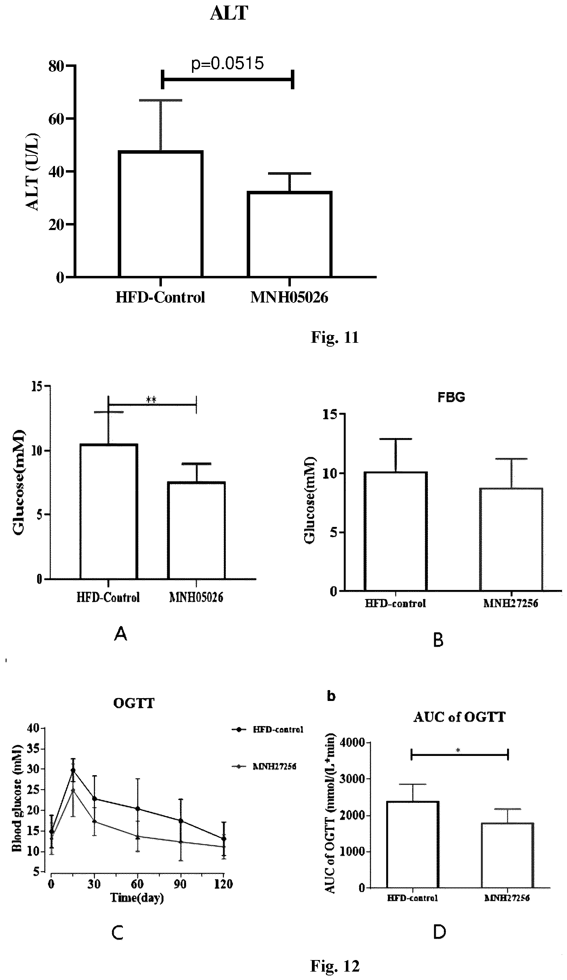

Based on the research results in Fig. 10, the use of *Megasphaera* bacteria in the treatment or prevention of liver function impairment was further explored using MNH05026 as a representative. The results are shown in Fig. 11.

Fig. 11 is a graph showing the effect of the strain MNH05026 on the serum alanine aminotransferase (ALT) level in high-fat diet-induced obesity mice.

The results showed that MNH05026 reduced the content of alanine aminotransferase (ALT) in serum. In Fig. 11, alanine aminotransferase (ALT) and aspartate aminotransferase (AST) are markers of hepatocellular injury. The results in Fig. 11 showed that MNH05026 reduced the serum ALT level, indicating that MNH05026 can improve liver dysfunction caused by a high-fat diet, and can significantly improve fatty liver and liver dysfunction caused by a high-fat diet. In summary, both MNH05026 and MNH22004 can significantly reduce liver weight, and MNH05026 can also reduce the serum ALT level, indicating that MNH05026 and MNH22004 can significantly improve fatty liver and liver dysfunction caused by a high-fat diet.

### Example 6. In vivo experiment on prevention or treatment of diabetes with the strains MNH05026 and MNH27256 in a high-fat diet-induced obesity mouse model

1) Experimental animals and 2) the negative control were the same as those in Example 5; 3) Test strain: A glycerol cryovial of the strain MNH05026 was thawed at 37 °C, and then inoculated onto an M01 plate in an anaerobic workstation for activation. The activated strain was inoculated into M01 liquid medium and anaerobically cultured to obtain a sufficient amount of cultures. The cultured bacterial solution was centrifuged, concentrated, and then resuspended in a solvent to afford a test article with a purity and viable bacterial count (2.9 × 10⁹ CFU/mL) meeting the requirements of animal experiments.

A glycerol cryovial of the strain MNH27256 was thawed at 37 °C, and then inoculated onto an M01 plate in an anaerobic workstation for activation. The activated strain was inoculated into M01 liquid medium and anaerobically cultured to obtain a sufficient amount of cultures. The cultured bacterial solution was centrifuged, concentrated, and then resuspended in a solvent to afford a test article with a purity and viable bacterial count (7.6 × 10⁹ CFU/mL) meeting the requirements of animal experiments.

4) Experimental procedure: Upon completion of the quarantine period, male C57BL/6J mice aged 5-6 weeks were fed with a high-fat diet for 10 weeks. 16 mice with a body weight ranging from 35.50 g to 44.49 g were selected and randomized into 2 groups of 8 animals each (an experimental group and a control group) according to body weight. Gavage administration was initiated after the grouping (D1). The experimental group was administered with the MNH05026 bacterial solution in an amount of 0.2 mL/animal, while the control group was given the negative control, twice a day, for a total of 21 days. During the experiment, the mice were given *ad libitum* access to water and food under 12 h/12 h light/dark cycle. During the experiment, one general clinical observation was conducted after each virtual dosing or the end of dosing. The animals were weighed on each Monday and Thursday during the dosing period, on the day of initial dosing (D1), on Day 21 of dosing (D21), and before dissection at the experimental endpoint. The endpoint of this experiment was the day following the end of dosing (D22). The animals were dissected and sampled according to the protocol at the experimental endpoint, and the data were summarized to analyze the body weight, the percent change in body weight, the fasting blood glucose measurement results, various anatomical data results, and serum test data results. All data were expressed as mean ± SD, and plotted and statistically analyzed using the GraphPad Prism 8.0.2 software. For pairwise comparisons, t-test (Student's t test) was used. It was not indicated if there was no significance; and the significance of difference was indicated by *: * p < 0.05, ** p < 0.01, *** p < 0.001, **** p < 0.0001.

Relevant experiments were conducted for MNH27256 using the same methods as in 1) to 4) above.

### 5) Experimental results

### 5.1) Effects on fasting blood glucose (FGB) in high-fat diet-induced obesity mice

The results of the effects of MNH05026 and MNH27256 on fasting blood glucose (FGB) in high-fat diet-induced obesity mice are shown in Figs. 12A and 12B, respectively. The data are shown as mean ± standard deviation (Mean ± SD). Statistical analysis was performed using t-test (Student's t test); **, p < 0.01, as compared to the HFD-Control group.

Fig. 12 shows the effects of the strains MNH05026 and MNH27256 on fasting blood glucose (FGB) in high-fat diet-induced obesity mice; Fig. 12C shows the results of glucose tolerance (OGTT) for MNH27256 in high-fat diet-induced obesity mice; and Fig. 12D shows the results of the area under the curve of glucose tolerance for MNH27256 in high-fat diet-induced obesity mice.

The results in Figs. 12A and 12B showed that both MNH05026 and MNH27256 reduced fasting blood glucose in high-fat diet-induced obesity mice, indicating that both MNH05026 and MNH22004 can significantly improve an increase in blood glucose caused by a high-fat diet and have the effect of treating diabetes.

### 5.2) Effects of the strain MNH27256 on oral glucose tolerance (OGTT) in high-fat diet-induced obesity mice

Based on the facts that both MNH05026 and MNH27256 strains can reduce FBG in high-fat diet-induced obesity mice, and the hypoglycemic effect of MNH05026 is better than that of MNH27256, MNH27256 was preferentially selected as a representative for further experiments to explore its effect on OGTT in high-fat diet-induced obesity mice.

Oral glucose tolerance test: On the 17th day of administration (D17), OGTT was measured after 12 hours of fasting (e.g., fasting from 20:30:00 in the evening to 08:30:00 the next day). The fasting body weight of mice was weighed, and glucose was administered by gavage according to the fasting body weight of mice. The glucose dose administered was 2 g/kg (g of glucose/kg of fasting body weight of mice). The fasting blood glucose and blood glucose levels at 15 min, 30 min, 60 min, 90 min, and 120 min after glucose administration were measured. Strict timing was conducted for each mouse and blood glucose levels were measured accurately at 6 time points. The measurement results are shown in Figs. 12C and 12D.

Figs. 12C-12D show the results from oral glucose tolerance test of MNH27256 in high-fat diet-induced obesity mice. The OGTT test is a glucose load test used to understand the function of islet β cells and the body's ability to regulate blood glucose, and observe the patient's ability to tolerate glucose. It is currently recognized as a diagnostic indicator for diagnosing diabetes. In case of glucose metabolism disorder, after a certain amount of glucose is administered orally, blood glucose rises sharply, or it does not rise obviously, but cannot drop to the fasting level or the original level in a short time. This is abnormal glucose tolerance or impaired glucose tolerance. Abnormal glucose tolerance indicates that the body's ability to metabolize glucose is reduced, which is common in type 2 diabetes and obesity. Fig. 12C shows the results of oral glucose tolerance, and Fig. 12D shows the results of the area under the curve of oral glucose tolerance. MNH27256 can effectively improve blood glucose increase and increase the glucose tolerance level, which is mainly manifested as reducing the AUC level of OGTT, indicating that the strain has the use of preventing or treating diabetes, especially diabetes induced by obesity, type II diabetes, and/or diabetes in patients with non-alcoholic fatty liver disease/non-alcoholic steatohepatitis.

### Example 7. Treatment of hyperglycemia with the strains MNH05026 and MNH27256 respectively combined with Metformin in mice with type II diabetes

1) Experimental animals: BKS-Leprem2Cd479/Gpt (db/db) mice, aged 5-6 weeks, 24 mice in total, purchased from Guangdong GemPharmatech Co., Ltd.
2) Test strain: A glycerol cryovial of the strain MNH05026 was thawed at 37 °C, and then inoculated onto an M01 plate in an anaerobic workstation for activation. The activated strain was inoculated into M01 liquid medium and anaerobically cultured to obtain a sufficient amount of cultures. The cultured bacterial solution was centrifuged, concentrated, and then resuspended in a solvent to afford a test article with a purity and viable bacterial count (6.3 × 10⁹ CFU/mL) meeting the requirements of animal experiments.
   MNH27256 was activated and cultured using the same methods as for MNH05026 to afford a test article with a purity and viable bacterial count (6.1 × 10⁹ CFU/mL) meeting the requirements of animal experiments.
3) Negative control: PBS containing 0.05% L-Cys HCl
4) Metformin (positive control): 250 mg/kg, by gavage
   Relevant experiments were set up for MNH27256 using the same methods as in 1) to 4) above.
5) Experimental procedure: Upon completion of the quarantine period, 24 male BKS-Leprem2Cd479/Gpt (db/db) mice aged 5-6 weeks were randomized into 3 groups of 8 animals each according to body weight: negative control group (HFD-Control), positive control group (Metformin), and drug combination groups (MNH05026 + Metformin, and MNH27256 + Metformin). Administration was initiated after the grouping (D1). The negative control group was given the negative control, the positive control group was given Metformin (250 mg/kg), and the drug combination group was given the corresponding bacterial solution (0.2 mL/mouse) and Metformin (250 mg/kg). The bacterial solution (MNH05026 or MNH27256) was administered twice a day, and Metformin was administered once a day. Both were administered by gavage for a total of 28 days. Throughout the experiment, the mice were fed with a murine maintenance feed, and given *ad libitum* access to water and food under 12 h/12 h light/dark cycle. An OGTT test was performed on the 27th day of dosing (D27), and fasting blood glucose was measured once on the 28th day of dosing (D28). All groups of mice were dissected and sampled on the day following the end of dosing (D29), and the data were summarized to analyze the data such as fasting blood glucose and OGTT. All data were expressed as mean ± SD, and plotted and statistically analyzed using the GraphPad Prism 8.0.2 software. For mutual comparisons of more than three groups, One-Way ANOVA with Dunnett's multiple comparison test was used for analysis. It was not indicated if there was no significance, and the significance of difference was indicated by *: * p < 0.05, ** p < 0.01, *** p < 0.001, **** p < 0.0001.
6) Experimental results:
   6.1) Effect of MNH05026 combined with Metformin in improving oral glucose tolerance in mice with type II diabetes:
   Oral glucose tolerance test (OGTT): On the 27th day of administration (D27), OGTT was measured after 12 hours of fasting (e.g., fasting from 20:30:00 in the evening to 08:30:00 the next day). The fasting body weight of mice was weighed, and glucose was administered by gavage according to the fasting body weight of mice. The glucose dose administered was 2 g/kg (g of glucose/kg of fasting body weight of mice). The fasting blood glucose and blood glucose levels at 15 min, 30 min, 60 min, 90 min, and 120 min after glucose administration were measured. Strict timing was conducted for each mouse and blood glucose levels were measured accurately at 6 time points.

The model mice used in this experiment (BKS-Lepr^{em2Cd479}/Gpt (db/db) mice) carry a mutation in the Leptin gene and thus exhibit symptoms of type 2 diabetes. DB/DB mice with BKS background are susceptible to diabetes, exhibit severe diabetic symptoms, and have severe damage to islet B cells.

In addition, a point mutation in the Leptin receptor leads to dysfunction in the leptin signaling pathway, which in turn causes mice to experience symptoms such as obesity, insulin resistance, hyperglycemia, and fatty liver.

The oral glucose tolerance test is a glucose load test used to understand the function of islet β cells and the body's ability to regulate blood glucose, and observe the patient's ability to tolerate glucose. It is currently recognized as a diagnostic indicator for diagnosing diabetes. In case of glucose metabolism disorder, after a certain amount of glucose is administered orally, blood glucose rises sharply, or it does not rise obviously, but cannot drop to the fasting level or the original level in a short time. This is abnormal glucose tolerance or impaired glucose tolerance. Abnormal glucose tolerance indicates that the body's ability to metabolize glucose is reduced, which is common in type 2 diabetes and obesity.

Fig. 13 shows the effect of MNH05026 combined with Metformin on oral glucose tolerance (OGTT) in mice with type II diabetes.

Fig. 13A shows the effect of MNH05026 combined with Metformin on oral glucose tolerance (OGTT) in mice with type II diabetes, and Fig. 13B shows the area under the curve of oral glucose tolerance of experimental mice in each treatment group.

Fig. 13A shows experimental results of MNH05026 combined with Metformin for improving oral glucose tolerance (OGTT) in mice with type II diabetes, and Fig. 13B shows the area under the curve of oral glucose tolerance of experimental mice in each experimental group (the data are shown as mean ± standard deviation (Mean ± SD). Statistical analysis was performed using one-way ANOVA with Dunnett's multiple comparison test, * p < 0.05). The results showed that the strain MNH05026 combined with Metformin more effectively improved blood glucose increase and significantly increased the glucose tolerance level as compared to Metformin alone. The main evidence was that the blood glucose values measured at 5 time points in the combination group after glucose administration were all lower than those in the positive control group, and the blood glucose values were significantly reduced in the time periods of 60 minutes and 90 minutes after glucose administration, and finally the AUC level was significant reduced in the combination group. This indicates that the combination of MNH05026 and Metformin can better control blood glucose and prevent and control diabetes, especially type II diabetes. In addition, the experiment also shows that the combination of MNH05026 and Metformin can effectively improve insulin resistance and glucose tolerance, can achieve better results in preventing or treating type II diabetes, and thus has the use of preventing or treating diabetes, especially diabetes caused by obesity, type II diabetes, and/or diabetes in patients with fatty liver/steatohepatitis.

### 6.2) Experimental results of the effects of MNH05026 and MNH27256 respectively combined with Metformin on fasting blood glucose in mice with type II diabetes:

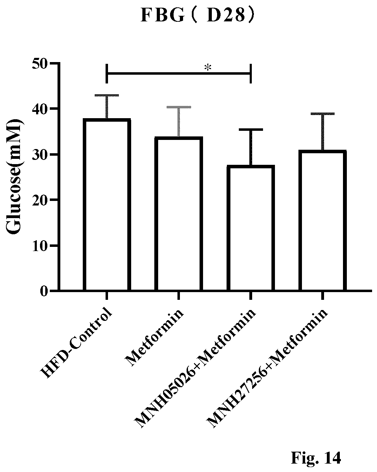

Fig. 14 shows the effects of MNH05026 and MNH27256 respectively combined with Metformin on fasting blood glucose in mice with type II diabetes.

Fig. 14 shows the effect of MNH05026 combined with Metformin on fasting blood glucose, and the effect of MNH27256 combined with Metformin on fasting blood glucose. The data are shown as mean ± standard deviation (Mean ± SD). Statistical analysis was performed using One-Way ANOVA with Dunnett's multiple comparison test. ** p < 0.01.

Metformin is an AMPK agonist, which plays a role in lowering blood glucose and treating diabetes by promoting GLP-1 secretion. This shows that the strain MNH05026 of the present application can promote the therapeutic effect of Metformin, and enhance GLP-1 secretion to achieve the effects of lowering blood glucose and treating diabetes. The results in Fig. 14 showed that MNH05026 and MNH27256 respectively combined with Metformin more significantly reduced fasting blood glucose in mice with type II diabetes, and MNH05026 combined with Metformin more significantly reduced fasting blood glucose than MNH27256 combined with Metformin, indicating that both MNH05026 and MNH27256 strains of the present application can promote the therapeutic effect of Metformin by further promoting GLP-1 secretion, thereby achieving the purposes of lowering blood glucose and treating diabetes. Moreover, MNH05026 combined with Metformin has more significant blood glucose lowering effect and can be used for patients with higher blood glucose levels.

### 6.3) Treatment of hyperglycemia with MNH05026 alone and in combination with Metformin in model mice with type II diabetes:

Based on the fact that MNH27256 combined with Metformin had a more significant blood glucose lowering effect, this experiment was further conducted by using MNH27256 alone and in combination with Metformin to treat hyperglycemia in mice with type II diabetes to verify the effect of MNH27256 in preventing or treating diabetes. The present invention has been ethically reviewed and supervised by the Laboratory Animal Care and Use Committee (IACUC) of Moon Biotech.
1) Experimental animals: C57BL/6J mice, aged 5-6 weeks, 54 mice in total, purchased from Guangdong GemPharmatech Co., Ltd.
2) Test strain: A glycerol cryovial of the strain MNH27256 was thawed at 37 °C, and then inoculated onto an M01 plate in an anaerobic workstation for activation. The activated strain was inoculated into M01 liquid medium and anaerobically cultured to obtain a sufficient amount of cultures. The cultured bacterial solution was centrifuged, concentrated, and then resuspended in a solvent to afford a test article with a purity and viable bacterial count (1.25 × 10¹⁰ CFU/mL) meeting the requirements of animal experiments.
3) Negative control: PBS-Cys containing 25% glycerol
4) Metformin (positive control): 250 mg/kg, by gavage
5) Experimental procedure: Upon completion of the quarantine period, 54 male C57BL/6J mice aged 5-6 weeks were fed with a high-fat diet for 6 weeks, and intraperitoneally (i.p.) injected with STZ once a day for 8 consecutive days, starting from D43. All mice were intraperitoneally (i.p.) injected with an STZ injection at a dose of 50 mg/kg on D43, and intraperitoneally (i.p.) injected with the STZ injection at a dose of 25 mg/kg on D44-D50. Body weight and random blood glucose were measured on D44, D46, D48, and D50, and fasting blood glucose of the animals after fasting for 12 hours was measured on D49 and D51. On D51, using random blood glucose on D50 ≥ 13.9 mM as the primary reference standard and two fasting blood glucose values ≥ 11.1 mM as the secondary reference standard, 32 mice with qualified blood glucose values were selected for subsequent intervention experiment. The mice were randomized into 4 groups of 8 animals each according to fasting blood glucose: negative control group (Control), positive control group (Metformin), MNH27256 group, and drug combination group (MNH27256 + Metformin). Administration was initiated after the grouping (D1). The negative control group was given the negative control, the positive control group was given Metformin (250 mg/kg), the MNH27256 group was given the MNH27256 bacterial solution (0.2 mL/mouse), and the drug combination group was given the MNH27256 bacterial solution (0.2 mL/mouse) and Metformin (250 mg/kg). The MNH27256 bacterial solution was administered twice a day, and Metformin was administered once a day. Both were administered by gavage for a total of 28 days. Throughout the experiment, the mice were fed with a high-fat diet, and given *ad libitum* access to water and food under 12 h/12 h light/dark cycle. Fasting blood glucose data were measured once a week during the dosing. All data were expressed as mean ± SD, and plotted and statistically analyzed using the GraphPad Prism 8.0.2 software. For mutual comparisons of more than three groups, One-Way ANOVA with Dunnett's multiple comparison test was used for analysis. It was not indicated if there was no significance; and the significance of difference was indicated by *: * p < 0.05, ** p < 0.01, *** p < 0.001, **** p < 0.0001.

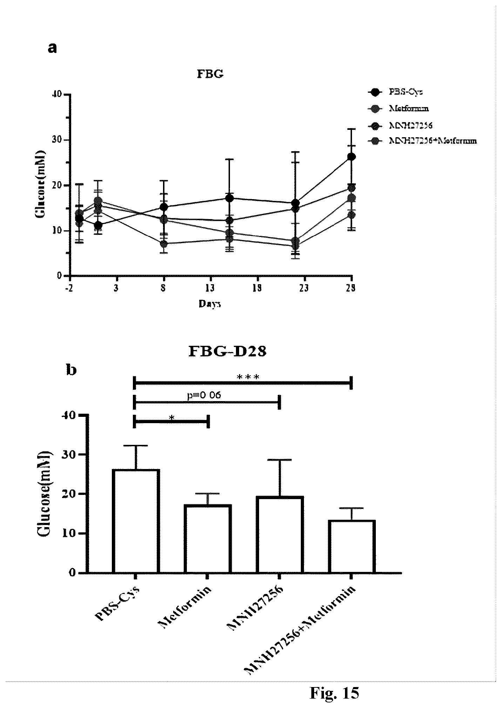

Fig. 15A is a graph showing the fasting blood glucose change in mice with type II diabetes treated with MNH27256 alone and in combination with Metformin.

Fig. 15A shows fasting blood glucose change curves, and Fig. 15B shows the fasting blood glucose values at the intervention endpoint. As shown in Fig. 15A, MNH27256 had an effect of lowering fasting blood glucose when used alone, and MNH27256 had a more obvious blood glucose lowering effect when used in combination with Metformin. In addition, MNH27256 combined with Metformin significantly promoted the blood glucose lowering effect of Metformin. In the initial stage (first 8 days) of administration, MNH27256 had a blood glucose lowering effect comparable to that of Metformin, and at the endpoint of use, the blood glucose level was also comparable to that of the Metformin group. The results show that although the overall effect of MNH27256 alone is not as good as that of Metformin, it is valuable in long-term blood glucose control, and the strain MNH27256 can promote the therapeutic effect of Metformin by further promoting GLP-1 secretion, thereby achieving the effects of lowering blood glucose and treating diabetes. This indicates that MNH27256 alone or in combination with Metformin has the effect of lowering fasting blood glucose, and is useful for preventing or treating diabetes.

### Example 8. In vivo experiment of the strains MNH05026, MNH22004, and MNH27256 in a high-fat diet-induced obesity mouse model to verify use of corresponding strains for treating and preventing obesity and related diseases

1) Experimental animals, 2) Test strains, and 3) Negative control were the same as those in Example 5.

4) Experimental procedure: Upon completion of the quarantine period, male C57BL/6J mice aged 5-6 weeks were fed with a high-fat diet for 10 weeks. 16 mice with a body weight ranging from 35.50 g to 44.49 g were selected and randomized into 2 groups of 8 animals each (an experimental group and a control group) according to body weight. Administration was initiated after the grouping (D1). The experimental group was administered with the MNH05026 bacteria solution (0.2 mL/mouse), while the control group was given the negative control, twice a day, for a total of 21 days. During the experiment, the mice were given *ad libitum* access to water and food under 12 h/12 h light/dark cycle. During the experiment, one general clinical observation was conducted after each virtual dosing or the end of dosing. The animals were weighed on each Monday and Thursday during the dosing period, on the day of initial dosing (D1), on Day 21 of dosing (D21), and before dissection at the experimental endpoint. The endpoint of this experiment was the day following the end of dosing (D22). The animals were dissected and sampled according to the protocol at the experimental endpoint, and the data were summarized to analyze the body weight, the percent change in body weight, the fasting blood glucose measurement results, various anatomical data results, and serum test data results. All data were expressed as mean ± SD, and plotted and statistically analyzed using the GraphPad Prism 8.0.2 software. For pairwise comparisons, t-test (Student's t test) was used. It was not indicated if there was no significance; and the significance of difference was indicated by *: * p < 0.05, ** p < 0.01, *** p < 0.001, **** p < 0.0001.

Relevant experiments were conducted for the strains MNH05026, MNH22004, and MNH27256 using the methods as described in 1) to 4) above.

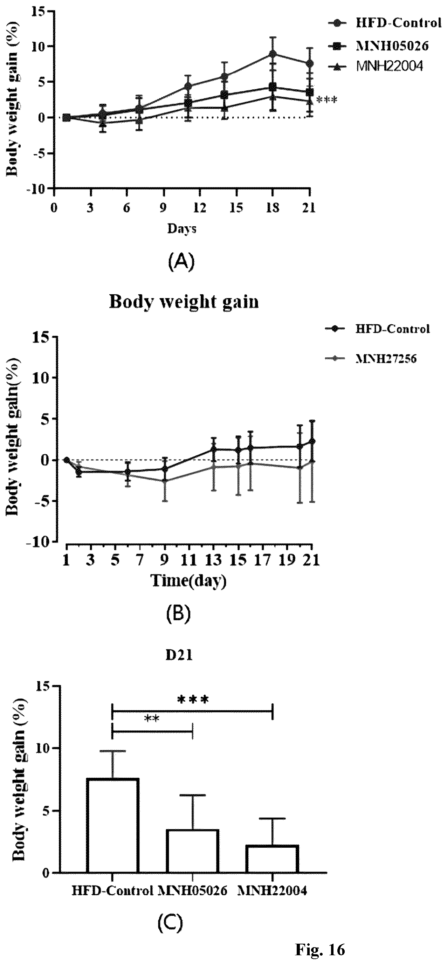

Fig. 16 shows the effects of the strains MNH05026, MNH27256, and MNH22004 on body weight gain in high-fat diet-induced obesity mice.

Fig. 16A shows the effects of MNH05026 and MNH22004 on body weight gain (i.e., weight gain/loss) in obese model mice, and Fig. 16C shows the corresponding weight gain at the experimental endpoint (day 21). The results showed that as compared to the control group, MNH05026 and MNH22004 significantly reduced the weight gain in high-fat diet-induced obesity mice, achieving the purposes of controlling body weight and preventing excessive weight gain. Moreover, within the first 7 days after administration, MNH05026 and MNH22004 slightly reduced the body weight of obese mice to a certain extent. Fig. 16B is a graph showing the effect of MNH27256 on the weight gain of obese mice, showing that MNH05026 was able to stably control the body weight of obese mice throughout the experimental period (21 days in total) after administration. There was no significant increase in the body weight of the mice at the experimental endpoint, and the overall weight of the mice was decreased to varying degrees throughout the experiment as compared to before administration. The maximum weight loss was on the 9th day after administration, which was about 2.6%. In summary, MNH05026, MNH22004, and MNH27256 can significantly inhibit the body weight gain caused by a high-fat diet, and MNH27256 can also reduce the body weight of obese mice to a certain extent, improve the body weight gain caused by a high-fat diet, and has the effect of preventing and treating obesity.

### Example 9. In vivo experiment of the strains MNH05026, MNH22004, and MNH27256 respectively combined with Semaglutide in a mice model of NAFLD/NASH induced by a high-fat, high-carbohydrate, and high-cholesterol diet to verify their use for treating and preventing NAFLD/NASH and related diseases

1) Experimental animals: male C57BL/6J mice, aged 5-6 weeks, 24 mice in total, purchased from Guangdong GemPharmatech Co., Ltd.
2) Test strains: A glycerol cryovial of each of the strains MNH05026, MNH22004, and MNH27256 was thawed at 37 °C, and then inoculated onto an M01 plate in an anaerobic workstation for activation. The activated strain was inoculated into M01 liquid medium and anaerobically cultured to obtain a sufficient amount of cultures. The cultured bacterial solution was centrifuged, concentrated, and then resuspended in a solvent to afford a test article with a purity and viable bacterial count (1.5 × 10⁹ CFU/mL) meeting the requirements of animal experiments.
3) Controls: PBS-Cys containing 25% glycerol PBS-Cys was used as a negative control; and Semaglutide was used as a positive control.
4) Experimental procedure: Upon completion of the quarantine period, 24 male C57BL/6J mice aged 5-6 weeks were fed with a high-fat, high-carbohydrate, and high-cholesterol diet for 26 weeks, and randomized into 3 groups of 8 animals each according to body weight: negative control group (Control), positive control group (Semaglutide), and drug combination group (the corresponding strain + Semaglutide). Administration was initiated after the grouping (D1). The negative control group was given the negative control (PBS-Cys) by gavage, 0.2 mL/mouse, twice a day; the positive control group was given Semaglutide by subcutaneous injection, at a dose of 30 nmol/kg, once every 3 days; and the combined treatment group was given Semaglutide (at a dose and frequency of use the same as those in the positive control group) and the corresponding bacterial solution (0.2 mL/mouse, twice a day) by gavage. The experimental period was 60 days. During the experiment, the mice were given *ad libitum* access to water and food under 12 h/12 h light/dark cycle. During the experiment, one general clinical observation was conducted after the end of each dosing. The animals were weighed twice a week during the dosing, and before dissection at the experimental endpoint. The endpoint of this experiment was the day following the end of dosing (D61). The animals were dissected and sampled according to the protocol at the experimental endpoint, and the data were summarized to analyze the results. All data were expressed as mean ± SD, and plotted and statistically analyzed using the GraphPad Prism 8.0.2 software. Statistical analysis was performed using One-Way ANOVA with Dunnett's multiple comparison test. It was not indicated if there was no significance; and the significance of difference was indicated by *: *: p < 0.05, **: p < 0.01, ***: p < 0.001, ****: p < 0.0001.
   Relevant experiments were conducted for the strains MNH05026, MNH22004, and MNH27256 using the methods as described in 1) to 4) above.
5) Experimental results and analysis:
   5.1) Effects of the *Megasphaera* srains of the present disclosure combined with Semaglutide on the body weight of NAFLD/NASH model mice:

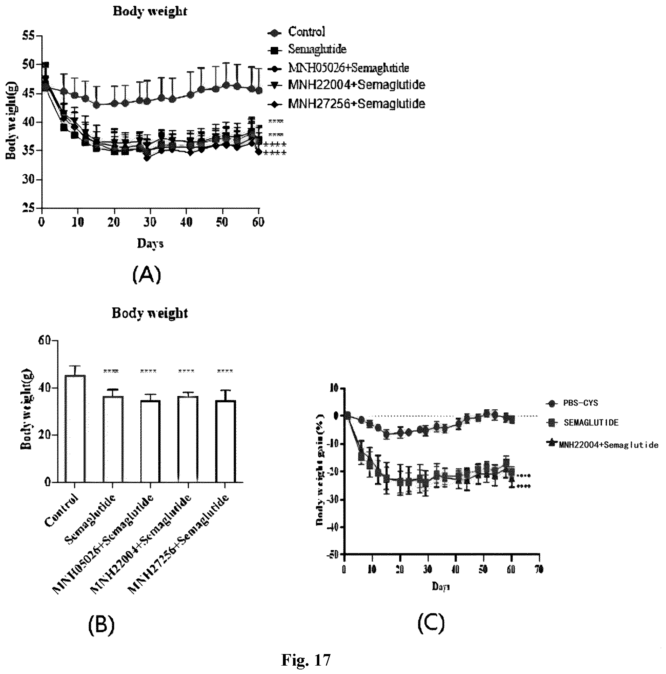

   Fig. 17 shows the effects of the strains MNH05026, MNH22004, and MNH27256 respectively combined with Semaglutide on body weight in mice with NAFLD/NASH induced by a high-fat, high-carbohydrate, and high-cholesterol diet.

The results of the effects of the *Megasphaera* strains combined with Semaglutide on body weight in mice with NAFLD/NASH induced by a high-fat, high-carbohydrate, and high-cholesterol diet are shown in Figs. 17A-17C and Table 13. Fig. 17A is a graph showing the effects of the *Megasphaera* strains MNH05026, MNH22004, and MNH27256 respectively combined with Semaglutide on body weight in mice with NAFLD/NASH induced by a high-fat, high-carbohydrate, and high-cholesterol diet; Fig. 17B shows the body weight values of mice on D60 for the *Megasphaera* strains MNH05026, MNH22004, and MNH27256 respectively combined with Semaglutide; Fig. 17C is a graph showing the body weight gain of mice for MNH22004 in combination with Semaglutide; and Table 13 is part of the data extracted from Fig. 17A. Semaglutide is a GLP-1 receptor agonist and a common weight loss drug in clinical practice. According to Fig. 17A, Fig. 17B and Table 13, in the Semaglutide treatment group, body weight was quickly reduced in the early stage of administration, showing a more obvious effect. However, after more than 30 days of administration, a certain resistance to Semaglutide was developed over time. The body weight of mice began to regain between day 30 and day 60, and the regain rate was higher than that of the combination group. At day 33 of intervention, the weight gain value of mice in the Semaglutide treatment group was -21.30%, and the corresponding values in the MNH05026, MNH22004, and MNH27256 groups were -21.56%, - 25.60%, and -24.02%, respectively. That is, after day 33, MNH05026, MNH27256, and MNH22004 in combination with Semaglutide could further reduce the weight gain of obese mice on the basis of the weight loss caused by Semaglutide. That is, the combination groups began to exhibit better weight control effect than Semaglutide alone. The results show that the *Megasphaera* strains MNH05026, MNH27256, and MNH22004 have a certain effect in reducing the body's resistance to Semaglutide for weight loss, and can improve the therapeutic effect of Semaglutide on obesity. The *Megasphaera* strains MNH05026, MNH22004, and MNH27256 in combination with Semaglutide have the effect of preventing and treating obesity in obese patients. In model mice with NAFLD/NASH induced by a high-fat, high-carbohydrate, and high-cholesterol diet, the *Megasphaera* strains MNH05026, MNH22004, and MNH27256 in combination with Semaglutide can reduce body weight and weight gain, prevent and treat obesity caused by a high-fat, high-carbohydrate, and high-cholesterol diet, and has the effect of preventing and treating obesity in NAFLD/NASH patients.

**Table 13. MNH05026, MNH22004, and MNH27256 combined with Semaglutide reduce the body weight of obese mice:**

| Days | 1d | 33d | 36d | 41d | 44d | 48d | 51d | 54d | 58d | 60d |
|---|---|---|---|---|---|---|---|---|---|---|
| PBS-Cys | 46.14 | 44.29 | 44.04 | 44.81 | 45.74 | 45.82 | 46.50 | 46.28 | 45.84 | 45.57 |
| Semaglutide | 45.97 | 36.18 | 35.94 | 35.98 | 36.34 | 37.02 | 37.26 | 36.84 | 38.09 | 36.67 |
| MNH22004+Sem aglutide | 47.49 | 37.25 | 36.87 | 36.58 | 36.54 | 37.49 | 37.65 | 37.33 | 38.45 | 36.82 |
| MNH05026+Sem aglutide | 47.08 | 35.03 | 35.19 | 34.72 | 35.19 | 36.03 | 35.99 | 35.98 | 37.21 | 34.84 |
| MNH27256+Sem aglutide | 46.87 | 35.61 | 35.71 | 35.59 | 35.64 | 35.90 | 36.37 | 35.60 | 36.31 | 34.89 |

### 5.2) Effects of the Megasphaera strains of the present disclosure combined with Semaglutide on liver weight and liver function impairment:

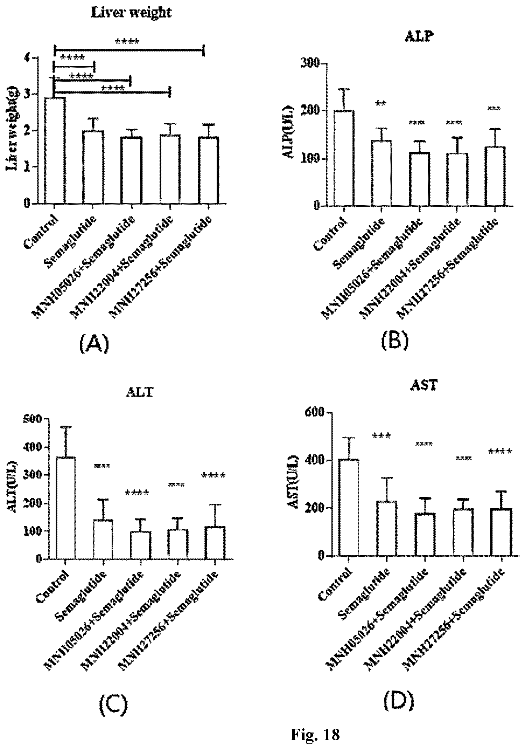

Fig. 18A is a graph showing the effects of MNH05026, MNH22004, and MNH27256 respectively combined with Semaglutide on liver weight in mice with NAFLD/NASH induced by a high-fat, high-carbohydrate, and high-cholesterol diet.

The results of the effects of the *Megasphaera* strains combined with Semaglutide on liver weight and liver function impairment in mice with NAFLD/NASH induced by a high-fat, high-carbohydrate, and high-cholesterol diet are shown in Figs. 18A-18D. The results in Fig. 18A showed that MNH05026, MNH22004, and MNH27256 in combination with Semaglutide significantly reduced the liver weight of model mice with NAFLD/NASH induced by a high-fat, high-carbohydrate, and high-cholesterol diet, with effects better than the positive control Semaglutide. This shows that the combination of the *Megasphaera* strains and Semaglutide can enhance the preventive and therapeutic effects of Semaglutide on liver function impairment and better improve liver function. As compared to the effects of MNH22004 and MNH27256 in combination with Semaglutide, the combination of MNH05026 and Semaglutide more significantly reduced the liver function of model mice with NAFLD/NASH induced by a high-fat, high-carbohydrate, and high-cholesterol diet, and had a better effect of preventing and treating liver function impairment.

Alanine aminotransferase (ALT) and aspartate aminotransferase (AST) are markers of hepatocellular injury. Alkaline phosphatase (ALP) is an enzyme mainly distributed in the liver, and on the microvilli on the blood sinus side and bile capillary side of hepatocytes, and discharged into the small intestine through bile juice. When bile juice cannot be discharged smoothly and the pressure in the bile capillary increases, a large amount of alkaline phosphatase will be produced. High alkaline phosphatase level is often seen in chronic hepatitis, cirrhosis, or alcoholic hepatitis. A large increase in ALP activity also indicates cholestasis, which may be intrahepatic cholestasis or extrahepatic cholestasis. Therefore, ALT, AST, and ALP reflect liver diseases such as hepatocellular injury, cholestasis, or both. Figs. 18B, 18C and 18D showed that MNH05026, MNH22004, and MNH27256 in combination with Semaglutide significantly reduced the levels of aspartate aminotransferase (AST), alanine aminotransferase (ALT), and alkaline phosphatase (ALP) in serum, with effects better than the positive control Semaglutide. This suggests that the strains MNH05026, MNH22004, and MNH27256 of the present application in combination with Semaglutide can prevent or treat liver injury, prevent or treat chronic hepatitis, cirrhosis or alcoholic hepatitis, prevent or treat intrahepatic cholestasis, prevent or treat extrahepatic cholestasis, and have a better effect of improving liver function of NAFLD/NASH patients. The strains MNH05026, MNH22004, and MNH27256 in combination with Semaglutide have the effects of preventing or treating fatty liver in NAFLD/NASH patients, improving liver function in NAFLD/NASH patients, preventing or treating liver injury in NAFLD/NASH patients, preventing or treating intrahepatic cholestasis in NAFLD/NASH patients, and preventing or treating extrahepatic cholestasis in NAFLD/NASH patients.

### 5.3) Effects of the Megasphaera stains of the present disclosure combined with Semaglutide on blood glucose in model mice with NAFLD/NASH induced by a high-fat, high-carbohydrate, and high-cholesterol diet:

Oral glucose tolerance test is used to measure the function of islet β cells and the body's ability to regulate blood glucose. It is currently recognized as a diagnostic indicator for diagnosing diabetes. In case of glucose metabolism disorder, after a certain amount of glucose is administered orally, blood glucose rises sharply, or it does not rise obviously, but cannot drop to the fasting level or the original level in a short time. This is abnormal glucose tolerance or impaired glucose tolerance. Abnormal glucose tolerance indicates that the body's ability to metabolize glucose is reduced, which is common in type II diabetes and obesity.

Fig. 19 shows the effects of MNH05026, MNH22004, and MNH27256 combined with Semaglutide on oral glucose tolerance in model mice with NAFLD/NASH induced by a high-fat, high-carbohydrate, and high-cholesterol diet.

The results of the effects of the *Megasphaera* strains combined with Semaglutide on oral glucose tolerance and fasting blood glucose in mice with NAFLD/NASH induced by a high-fat, high-carbohydrate, and high-cholesterol diet are shown in Figs. 19A-19E. The results in Figs. 19A-19D showed that as compared to Semaglutide alone, MNH05026, MNH22004, and MNH27256 respectively combined with Semaglutide more significantly improved the oral glucose tolerance (OGTT) of the mice, and the three *Megasphaera* strains combined with Semaglutide had similar effects in improving OGTT. As shown in Fig. 19E, Semaglutide alone or the combination of MNH27256 and Semaglutide had no significant effect in lowering the fasting blood glucose of NAFLD/NASH mice. However, MNH05026 and MNH22004 in combination with Semaglutide had a significant effect in lowering the fasting blood glucose of NAFLD/NASH mice, and MNH05026 exhibited a stronger significance.

In summary, MNH05026, MNH22004, and MNH27256 in combination with Semaglutide can further promote the control level of Semaglutide on OGTT and FBG in model mice with NAFLD/NASH induced by a high-fat, high-carbohydrate, and high-cholesterol diet, more significantly reduce their OGTT and FBG, have better effects in preventing and treating diabetes in NAFLD/NASH patients, improving blood glucose control function, and can also be used for type 2 diabetes and diabetes caused by obesity. In the three *Megasphaera* strains in combination with Semaglutide, MNH05026 has the best protective effect, MNH22004 has the second best protective effect, and MNH27256 has the weakest protective effect. For strains with different protective effects, products with different dosages can be developed for patients with different disease courses.

### 5.4) Effects of the Megasphaera srains of the present disclosure combined with Semaglutide on local fat pad weight in model mice with NAFLD/NASH induced by a high-fat, high-carbohydrate, and high-cholesterol diet:

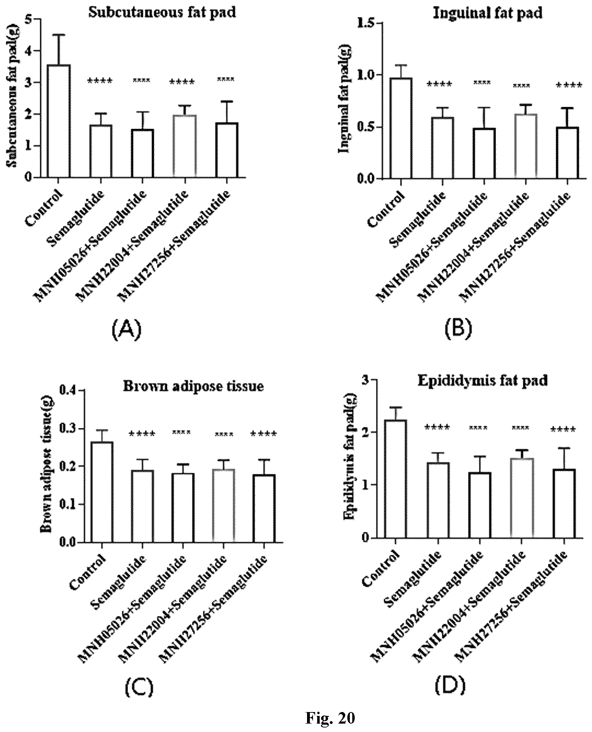

In the present application, the effects of MNH05026, MNH22004, and MNH27256 in combination with Semaglutide on subcutaneous fat pad weight were further explored in mice. Medical weight loss using a drug cannot lead to a decrease in local fat pad. This experiment was conducted to evaluate the effects of the *Megasphaera* strains of the present application on local fat pad during weight loss. In this experiment, the effects of MNH05026, MNH22004, and MNH27256 respectively combined with Semaglutide on subcutaneous fat pad weight, inguinal fat pad weight, brown adipose tissue weight, and epididymis fat pad weight were tested in model mice with NAFLD/NASH induced by a high-fat, high-carbohydrate, and high-cholesterol diet. The experimental results are shown in Figs. 20A-20D.

Fig. 20 shows the effects of MNH05026, MNH22004, and MNH27256 respectively combined with Semaglutide on subcutaneous fat pad weight, inguinal fat pad weight, brown adipose tissue weight, and epididymis fat pad weight in model mice with NAFLD/NASH induced by a high-fat, high-carbohydrate, and high-cholesterol diet.

The results showed that MNH05026, MNH22004, and MNH27256 in combination with Semaglutide significantly reduced local subcutaneous fat pad, inguinal fat pad, epididymis fat pad, and brown adipose tissue. In the strains, MNH05026 had the best overall protective effect, followed by MNH27256, and MNH22004 had the weakest effect. That is, the *Megasphaera* strains MNH05026, MNH22004, and MNH27256 of the present application in combination with Semaglutide can significantly reduce fat accumulation in model mice with NAFLD/NASH induced by a high-fat, high-carbohydrate, and high-cholesterol diet, which is better than or equivalent to the positive control Semaglutide, and has an ideal effect in preventing and treating obesity and fat accumulation in NAFLD/NASH patients.

### 5.5) Effects of the Megasphaera srains of the present disclosure combined with Semaglutide on blood lipids in model mice with NAFLD/NASH induced by a high-fat, high-carbohydrate, and high-cholesterol diet:

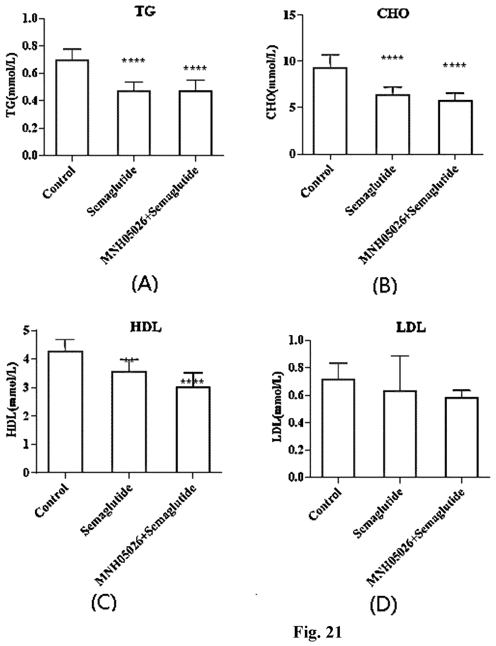

MNH05026, taken as an example, was used in combination with Semaglutide to verify its effects on four blood lipids: triglycerides (TG), total cholesterol (CHO), high-density lipoprotein (HDL), and low-density lipoprotein (LDL), in model mice with NAFLD/NASH induced by a high-fat, high-carbohydrate, and high-cholesterol diet. TG is mainly involved in energy metabolism in the human body and produces thermal energy. Too high TG content in the blood can lead to viscous blood, causing lipids to deposit on the blood vessel wall and gradually form small plaques, i.e., atherosclerosis. Increased LDL-C is a main and independent risk factor for the onset and development of atherosclerosis. The increased level of LDL-C is also an indicator for measuring coronary heart disease. Since HDL-C can transport cholesterol in the blood vessel wall to the liver for catabolism (i.e., reverse cholesterol transport), it can reduce the deposition of cholesterol on the blood vessel wall and plays an anti-atherosclerotic role. Results are shown in Figs. 21A-21D.

Fig. 21 shows the effect of MNH05026 combined with Semaglutide on triglycerides (TG), total cholesterol (CHO), high-density lipoprotein (HDL), and low-density lipoprotein (LDL) in model mice with NAFLD/NASH induced by a high-fat, high-carbohydrate, and high-cholesterol diet.

The results showed that the strain MNH05026 combined with Semaglutide significantly reduced the TG, CHO and HDL levels in blood lipids in model mice with NAFLD/NASH induced by a high-fat, high-carbohydrate, and high-cholesterol diet. This indicates that the strain MNH05026 in combination with Semaglutide can reduce the levels of total cholesterol, triglycerides and low-density lipoprotein in the serum of obese mice to varying degrees, and has the effects of preventing and treating cardiovascular diseases, atherosclerosis, coronary heart disease, and lowering blood lipids. It also indicates that the strain MNH05026 in combination with Semaglutide has the effect of treating/preventing cardiovascular and cerebrovascular diseases. The strain MNH05026 in combination with Semaglutide can significantly reduce the level of low-density lipoprotein in the serum of mice on a high-fat diet, and can be used for preventing or treating hyperlipidemia and atherosclerosis. It can further reduce serum total cholesterol, suggesting that the strain MNH05026 in combination with Semaglutide can prevent and treat cardiovascular diseases, have the effect of lowering blood lipids, have the effect of treating/preventing cardiovascular and cerebrovascular diseases, and can be used for preventing or treating atherosclerosis, coronary heart disease, and hyperlipidemia.

### 5.6) Effects of the Megasphaera srains of the present disclosure combined with Semaglutide on liver steatosis, lobular inflammation, liver ballooning, liver NAS score, and liver fibrosis:

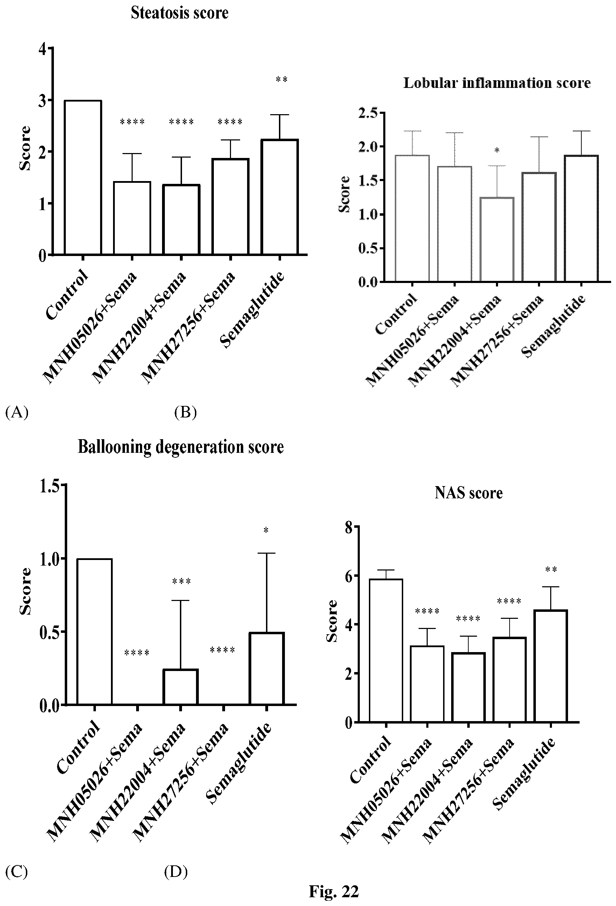

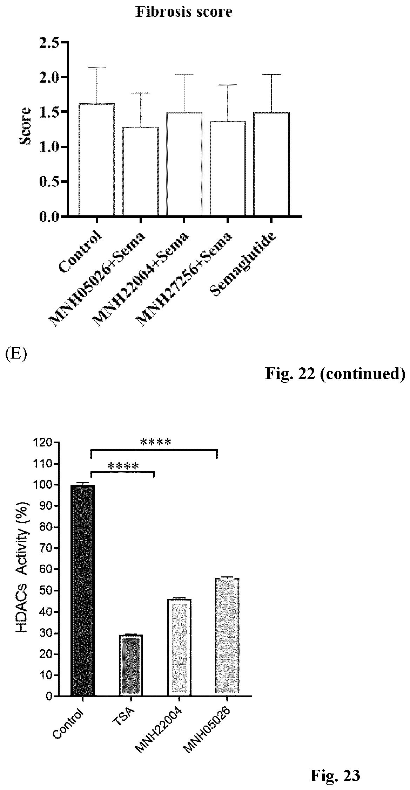

Fig. 22 shows the effects of MNH05026, MNH22004, and MNH27256 respectively combined with Semaglutide on liver steatosis, lobular inflammation, liver ballooning, liver NAS score, and liver fibrosis in mice with NAFLD/NASH induced by a high-fat, high-carbohydrate, and high-cholesterol diet.

The effects of the *Megasphaera* strains combined with Semaglutide on liver steatosis, lobular inflammation, liver ballooning, liver NAS score, and liver fibrosis in mice with NAFLD/NASH induced by a high-fat, high-carbohydrate, and high-cholesterol diet are shown in Figs. 22A-22E. The results in Fig. 22A showed that MNH05026, MNH22004, and MNH27256 in combination with Semaglutide significantly reduced the liver steatosis in model mice with NAFLD/NASH induced by a high-fat, high-carbohydrate, and high-cholesterol diet, with an effect better than the positive control Semaglutide. The results in Fig. 22B showed that MNH05026, MNH22004, and MNH27256 in combination with Semaglutide significantly reduced the liver lobular inflammation in model mice with NAFLD/NASH induced by a high-fat, high-carbohydrate, and high-cholesterol diet, with an effect better than the positive control Semaglutide. The results in Fig. 22C showed that MNH05026, MNH22004, and MNH27256 in combination with Semaglutide significantly reduced the liver ballooning in model mice with NAFLD/NASH induced by a high-fat, high-carbohydrate, and high-cholesterol diet, with an effect better than the positive control Semaglutide. The results in Fig. 22D showed that MNH05026, MNH22004, and MNH27256 in combination with Semaglutide significantly reduced the liver NAS score in model mice with NAFLD/NASH induced by a high-fat, high-carbohydrate, and high-cholesterol diet, with an effect better than the positive control Semaglutide. The results in Fig. 22E showed that MNH05026 and MNH27256 in combination with Semaglutide reduced the liver fibrosis score in model mice with NAFLD/NASH induced by a high-fat, high-carbohydrate, and high-cholesterol diet, with an effect better than the positive control Semaglutide. The above data indicate that the *Megasphaera* strains in combination with Semaglutide can enhance the preventive and therapeutic effects of Semaglutide on liver steatosis, lobular inflammation, liver ballooning, liver NAS score, and liver fibrosis, and has the potential to treat NAFLD/NASH.

### Example 10. Inhibitory effects of the strains MNH22004 and MNH05026 on the activity of histone deacetylases (HDACs)

MNH22004 and MNH27256 are different strains of the same species. Therefore, MNH22004 was selected as a representative, and MNH05026 was selected to verify whether the *Megasphaera* strain has an inhibitory effect on the activity of histone deacetylases. The HDAC Inhibitor Drug Screening Kit (Fluorometric) purchased from Abcam Co. was used in this study to detect the inhibitory effect on HDAC activity *in vitro.*

Preparation of culture supernatant of MNH22004: The strain MNH22004 was inoculated into a liquid culture medium, anaerobically cultured at 37 °C for 48 h, and centrifuged to remove bacterial cells. The culture supernatant was filtered with a 0.22 µm filter, aliquoted, and stored at -80 °C for later use.

Preparation of culture supernatant of MNH05026: The strain MNH05026 was inoculated into a liquid culture medium, anaerobically cultured at 37 °C for 48 h, and centrifuged to remove bacterial cells. The culture supernatant was filtered with a 0.22 µm filter, aliquoted, and stored at -80 °C for later use.

Preparation of test samples: 1) Control group: MM01 culture medium was diluted 10 times with PBS to obtain 10% MM01; 2) Positive control group: TSA, an HDAC inhibitor, was diluted with PBS to a final concentration of 40 µM; 3) MNH22004 group: the culture supernatant of MNH22004 was diluted 10 times with PBS to obtain an experimental sample containing 10% bacterial supernatant; 4) MNH05026 group: the culture supernatant of MNH05026 was diluted 10 times with PBS to obtain an experimental sample containing 10% bacterial supernatant.

Preparation of reaction reagent for HDAC detection: An appropriate amount of detection reaction system was prepared according to the instructions of the kit. 50 µL of the reaction reagent was required for each reaction.

Detection of HDAC activity: 50 µL of the test sample was added to a 96-well white plate, and then 50 µL of the reaction reagent was added respectively. They were mixed well and incubated at 37 °C for 30 min. 10 µL of Lysine Developer was added to the reaction well and mixed well to terminate the reaction. The plate was incubated at 37 °C for 30 min. Finally, the fluorescence intensity of the sample was detected using a microplate reader with the following microplate reader settings: Ex. = 350-380 nm, and Em. = 440-460 nm. To analyze the HDAC inhibitory activity of the sample, the fluorescence intensity value of the Control was set as 100%. The fluorescence intensities of the positive control group and the MNH22004 group were respectively divided by that of the Control and then multiplied by 100% to obtain the relative HDAC activities.

The results (see Fig. 23) showed that, as compared to the HDAC activity in the Control group, the supernatants of both MNH22004 and MNH05026 had a significant inhibitory effect on HDAC activity, which was similar to the effect of TSA in the HDAC inhibitor control group. These results suggest that both MNH22004 and MNH05026 can inhibit HDAC activity, and both MNH22004 and MNH05026 can be used to prevent or treat diseases mediated by HDAC activity by inhibiting HDAC activity.

Diabetes is a group of diseases in which a low level of insulin and/or peripheral insulin resistance leads to hyperglycemia. It has been proposed to treat diabetes by inhibiting HDAC activity, including achieving inhibition of Pdxl (Park, et al., 2008, J Clin Invest, 118, 2316-24), and enhancement of the expression of the transcription factor Ngn3 to increase the endocrine repertoire, progenitor cells (Haumaitre, et al., 2008, Mol Cell Biol, 28, 6373-83), enhancement of insulin expression (Molsey, et al., 2003, J Biol Chem, 278, 19660-6), etc. by inhibiting HDAC activity. HDAC inhibition is also a promising therapy for advanced diabetic complications such as diabetic nephropathy and retinal ischemia (Christensen, et al., 2011, Mol Med, 17(5-6), 370-390). Therefore, the composition of the present disclosure can be used to treat or prevent diabetes mediated by HDAC activity.

Studies showed that the histone deacetylase (HDAC) inhibitor valproic acid (VPA) partially restored the H3K9Ac level, endothelial cell tubulation and activity of diabetic EPC-EV damage, and enhanced the expression of the cell survival and proliferation genes Pdgfd and Sox12 (doi: 10.7150/thno.70821.).

Increasing attention is paid to histone deacetylase inhibitors (HDACis) for their roles in improving islet β-cell function, protecting them from inflammatory factors, improving insulin resistance in peripheral tissues, regulating the immune system to improve metabolism, and alleviating diabetic complications. They are expected to become novel drugs for preventing and treating diabetes and even gestational diabetes.

The composition of the present disclosure is used to treat or prevent diabetes mellitus. In a preferred embodiment, the composition of the prevent disclosure is used to treat or prevent type I diabetes. In a preferred embodiment, the composition of the prevent disclosure is used to treat or prevent type II diabetes. In certain embodiments, the composition of the prevent disclosure is used for the treatment or prevention of diabetes, wherein the treatment or prevention is accomplished by reducing or preventing HDAC activation.

The composition of the present disclosure has an HDAC inhibitory activity, and can be used for nervous system diseases, inflammatory bowel diseases such as IBD, and cardiovascular diseases according to the HDAC inhibitory activity (HDAC inhibition also results in beneficial outcomes in various types of neurodegenerative diseases, inflammation disorders, and cardiovascular diseases. HDAC and HDAC Inhibitor: From Cancer to Cardiovascular Diseases, https://pubmed.ncbi.nlm.nih.gov/26865995/). It can be determined that the strains of the present disclosure can also be used to treat nervous system diseases mediated by HDAC activity, inflammatory bowel diseases such as IBD, and cardiovascular diseases.

For the 16S rRNA sequence of the strain MNH05026, SEQ ID NO: 1 - see Sequence 1 in the sequence listing.

For the 16S rRNA sequence of the strain MNH22004, SEQ ID NO: 2 - see Sequence 2 in the sequence listing.

For the 16S rRNA sequence of the strain MNH27256, SEQ ID NO: 3 - see Sequence 3 in the sequence listing.

The above examples are only used to illustrate but not to limit the technical solutions of the present disclosure. Those of ordinary skill in the art should understand that the technical solutions described in the foregoing embodiments can be modified, or some or all of the technical features can be equivalently substituted without departing from the spirit and scope of the present disclosure. Such modifications or substitutions will not cause the essence of the corresponding technical solutions to deviate from the scope of technical solutions in the embodiments of the present disclosure.

## Claims

1. A bacterial strain of the genus *Megasphaera,* wherein the 16S rRNA sequence of the bacterial strain has at least 95%, 96%, 96.5%, 97%, 98%, 98.65%, 99%, 99.5%, 99.9%, or 100% identity to the 16S rRNA sequence of SEQ ID NO: 1, SEQ ID NO: 2, and/or SEQ ID NO: 3, and the bacterial strain is used for preventing and/or treating a metabolic disease;
preferably, the bacterial strain of the genus *Megasphaera* for preventing and/or treating a metabolic disease is selected from any one or more of *Megasphaera stantonii, Megasphaera indica, Megasphaera paucivorans, Megasphaera sueciensis, Megasphaera micronuciformis, Megasphaera hexanoica, Megasphaera cerevisiae, Megasphaera hominis, Megasphaera butyrica, Megasphaera hutchinsoni, Megasphaera lornae,* or *Megasphaera vaginalis.*

2. The bacterial strain according to claim 1, wherein the bacterial strain comprises a 16S rRNA sequence with at least 97%, 97.5%, 98%, 98.5%, 98.65%, 99%, 99.5%, or 99.9% identity to SEQ ID NO: 2 and/or SEQ ID NO: 3.

3. The bacterial strain according to claim 1, wherein the bacterial strain has a butyrate production pathway with at least 70% integrity; preferably, the butyrate production pathway is pyruvate pathway and/or 4-aminobutyrate pathway;
preferably, the bacterial strain is highly productive of butyric acid and/or acetic acid;
preferably, the bacterial strain is selected from any one or more of *Megasphaera stantonii, Megasphaera indica, Megasphaera paucivorans, Megasphaera sueciensis, Megasphaera micronuciformis, Megasphaera hexanoica, Megasphaera cerevisiae, Megasphaera hominis, Megasphaera butyrica, Megasphaera hutchinsoni, Megasphaera lornae,* or *Megasphaera vaginalis.*

4. The bacterial strain according to any one of claims 1 to 3, wherein the bacterial strain was deposited with the Guangdong Microbial Culture Collection Center with a deposit number of GDMCC No: 62001, GDMCC No: 62000, and/or GDMCC No: 61999;
preferably, the microbial strain has an average nucleotide identity (ANI) value of at least 79%, 80%, 85%, 88%, 90%, 95%, 96%, 97%, 98%, 99%, 99.5%, 99.8%, 99.9%, or 100% to the bacterial strain of the genus *Megasphaera* with a deposit number of GDMCC No: 62001, GDMCC No: 62000, and/or GDMCC No: 61999;
preferably, the average nucleotide identity (ANI) value is at least 79%, 80%, 81%, 82%, 83%, 84%, 85%, 86%, 87%, 88%, 89%, 90%, 91%, 92%, 93%, 94%, 95%, 95.5%, 96%, 96.5%, 97%, 97.5%, 98%, 98.5%, 99%, 99.5%, 99.6%, 99.7%, 99.8%, 99.9%, or 100%.

5. A microbial agent, wherein the microbial agent comprises at least one of the bacterial strain of the genus *Megasphaera* of any one of claims 1 to 4, a supernatant, an extract, a pure culture, an isolated culture, or a metabolite of the bacterial strain of the genus *Megasphaera;*
preferably, the metabolite of the bacterial strain comprises at least one of (a) to (d): (a) acetic acid or acetate, (b) propionic acid or propionate, (c) butyric acid or butyrate, or (d) valeric acid or valerate;
preferably, the bacterial strain is highly productive of acetic acid and/or butyric acid.

6. A culture, which is a culture of the strain of any one of claims 1 to 4;
preferably, the culture is a pure culture or an isolated culture;
preferably, the culture is a fermentation broth or a supernatant of the fermentation broth of the strain of any one of claims 1 to 4.

7. A composition, comprising the microbial agent of claim 5 or the culture of claim 6 or the bacterial strain of any one of claims 1 to 4, and at least one of an excipient, a diluent, or a carri er;
preferably, the microbial agent is in a dosage form of a suspension, a granule, a tablet, a suppository, a pulvis, a capsule, a microcapsule, an oral liquid, or a powder;
preferably, the composition is a food, a nutraceutical, a dietary supplement, a special medical food, a probiotic beverage, or a probiotic powder;
preferably, the composition is obtainable by freeze drying, sublimation drying, or spray drying.

8. The composition according to claim 7, wherein the composition further comprises at least one of a GLP-1 receptor agonist, a dual agonist of GLP-1 receptor and GCG receptor, a triple agonist of GLP-1 receptor, GIP receptor and GCG receptor, an AMPK agonist, or an active drug that promotes GLP-1 secretion;
preferably, the GLP-1 receptor agonist comprises any one of Semaglutide, liraglutide, exenatide, or beinaglutide;
preferably, the AMPK agonist or active drug that promotes GLP-1 secretion comprises Metformin, Semaglutide, and/or liraglutide;
optionally, the composition is administered by a mode comprising: administering a first active substance followed by a second active substance or a co-drug; or administering a second active substance or a co-drug followed by a first active substance; or administering a first active substance and a second active substance or a co-drug simultaneously; or administering a first active substance and a second active substance or a co-drug according to their respective frequencies of use; or administering a first active substance followed by administering a second active substance at a reduced dosage or frequency of use.

9. A pharmaceutical composition for a metabolic disease, comprising a first active substance, wherein the first active substance is at least one of the bacterial strain of any one of claims 1 to 4, the microbial agent of claim 5, the culture of claim 6, or the composition of claim 7;
preferably, the pharmaceutical composition comprises a pharmaceutical composition having at least one of the functions (a) to (c): (a) suppressing appetite, (b) preventing the metabolic disease, or (c) treating the metabolic disease;
more preferably, the suppressing appetite comprises reducing food intake and/or reducing appetite;
more preferably, the metabolic disease comprises at least one of a liver disease, obesity or obesity-related disease, cardiovascular disease, diabetes, dyslipidemia, cardiovascular and cerebrovascular disease, glucose intolerance, atherosclerosis, coronary heart disease or hypertension, type I diabetes, type II diabetes, impaired glucose tolerance, insulin resistance, obesity, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, or diabetic retinopathy;
wherein the liver disease is at least one of fatty liver, NAFLD/NASH, liver dysfunction, extrahepatic cholestasis, hepatitis, liver injury, or intrahepatic cholestasis;
optionally, the liver disease is caused by at least one of a high-fat diet, a high-cholesterol diet, a high-carbohydrate diet, high blood lipids, high blood glucose, or high cholesterol,
optionally, the liver disease comprises a disease caused by a high-fat diet, by a high-cholesterol diet, by a high-carbohydrate diet, by high lipids and high cholesterol, by high lipids and high glucose, and/or by high lipids, high cholesterol and high glucose;
wherein the obesity or obesity-related disease is overweight, obesity, metabolic syndrome, cardiovascular disease, cardiovascular and cerebrovascular disease, hyperlipidemia, hypercholesterolemia, hypertension, insulin resistance syndrome, obesity-related gastroesophageal reflux disease, or steatohepatitis;
optionally, the obesity disease is caused by at least one of a high-fat diet, a high-carbohydrate diet, high cholesterol, high blood lipids, high blood glucose, NAFLD, or NASH;
optionally, the obesity disease comprises obesity caused by a high-fat diet, obesity caused by high cholesterol, obesity caused by a high-carbohydrate diet, obesity caused by high lipids and high cholesterol, obesity caused by high lipids and high glucose, obesity caused by high lipids, high cholesterol and high glucose, or obesity in NAFLD or NASH patients;
optionally, the cardiovascular disease or cardiovascular and cerebrovascular disease is caused by at least one of atherosclerosis, NAFLD, NASH, high blood lipids, high blood glucose, or high cholesterol;
optionally, the cardiovascular disease or cardiovascular and cerebrovascular disease is atherosclerosis, coronary heart disease, a cardiovascular disease in NAFLD or NASH patients, a cardiovascular and cerebrovascular disease in NAFLD or NASH patients, and/or a high cholesterol disease;
wherein the diabetes is type I diabetes, type II diabetes, gestational diabetes, diabetes mediated by HDAC activity, diabetic nephropathy, diabetic neuropathy, diabetic retinopathy, diabetes caused by impaired islet B cells, diabetes caused by insulin resistance, or diabetes caused by obesity;
optionally, the diabetes is caused by at least one of islet cell dysfunction, decreased insulin secretion, increased insulin resistance, a high-fat diet, a high-carbohydrate diet, high cholesterol, high blood lipids, high blood glucose, NAFLD, or NASH;
optionally, the diabetes comprises diabetes caused by at least one of a high-fat diet, a high-carbohydrate diet, or a high-cholesterol diet.

10. The pharmaceutical composition for a metabolic disease according to claim 9, further comprising a second active substance, wherein the second active substance comprises at least one of a GLP-1 receptor agonist, a dual agonist of GLP-1 receptor and GCG receptor, a triple agonist of GLP-1 receptor, GIP receptor and GCG receptor, an AMPK agonist or an active drug that promotes GLP-1 secretion, a DPP-4 receptor inhibitor, a PPAR receptor agonist, a PPARα receptor agonist, a PPARδ receptor agonist, a PPARγ receptor agonist, a PPARα/δ receptor dual agonist, a PPARα/γ receptor dual agonist, a PPARα/δ/γ receptor triple agonist, or an active drug in the mechanism/target of CRTC, PGC-1α, SREBP, FXR, FGF21, ASK1, THR-β, LXR, NF-κB, SNDRI, MC4R, PNLIP, MOA, or DRI;
preferably, the GLP-1 receptor agonist comprises any one of Semaglutide, liraglutide, exenatide, or beinaglutide;
preferably, the AMPK agonist or active drug that promotes GLP-1 secretion comprises Metformin, Semaglutide, and/or liraglutide;
optionally, the composition is administered by a mode comprising: administering a first active substance followed by a second active substance or a co-drug; or administering a second active substance or a co-drug followed by a first active substance; or administering a first active substance and a second active substance or a co-drug simultaneously; or administering a first active substance and a second active substance or a co-drug according to their respective frequencies of use; or administering a first active substance followed by administering a second active substance at a reduced dosage of use; or administering a first active substance followed by administering a second active substance at a reduced frequency of use.

11. Use of the bacterial strain of the genus *Megasphaera* of any one of claims 1 to 4, the microbial agent of claim 5, the culture of claim 6, the composition of claim 7 or 8, or the pharmaceutical composition for a metabolic disease of claim 9 or 10 in the manufacture of a medicament for preventing and/or treating a metabolic disease;
wherein the bacterial strain and/or a metabolite of the bacterial strain and/or a supernatant of the bacterial strain inhibits HDAC activity;
preferably, the bacterial strain and/or a metabolite of the bacterial strain and/or a supernatant of the bacterial strain is used for treating or preventing a disease mediated by HDAC activity;
optionally, the disease mediated by HDAC activity comprises a metabolic disease;
further, the metabolic disease is at least one of a liver disease, obesity or obesity-related disease, cardiovascular disease, diabetes, dyslipidemia, cardiovascular and cerebrovascular disease, glucose intolerance, atherosclerosis, coronary heart disease or hypertension, type I diabetes, type II diabetes, impaired glucose tolerance, insulin resistance, obesity, weight control, overweight, blood glucose control, prediabetes, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, or diabetic retinopathy;
preferably, the inhibiting HDAC activity comprises inhibiting HDAC activity by regulating a short-chain fatty acid/short-chain fatty acid salt; the short-chain fatty acid comprises one or more of acetic acid, butyric acid, and valeric acid; more preferably, the short-chain fatty acid is butyric acid; the short-chain fatty acid salt comprises one or more of acetate, propionate, and valerate; more preferably, the short-chain fatty acid salt is butyrate; preferably, the histone acetylase is class I, class II, class III, and/or class IV histone acetylase.

12. The use according to claim 11, wherein the microbial strain, supernatant, culture, extract, or metabolite of the microbial strain increases GLP-1 expression.

13. Use of a bacterial strain of the genus *Megasphaera* and/or a composition comprising the bacterial strain of the genus *Megasphaera* in the manufacture of a medicament for preventing or treating a metabolic disease;
wherein the bacterial strain has a 16S rRNA sequence with at least 95%, 96%, 96.5%, 97%, 97.5%, 98%, 98.5%, 98.65%, 99%, 99.5%, 99.9%, or 100% identity to the 16S rRNA sequence of SEQ ID NO: 1, SEQ ID NO: 2, and/or SEQ ID NO: 3; the composition comprising the bacterial strain of the genus *Megasphaera* comprises at least one of the bacterial strain, a supernatant, a culture, an extract, or a metabolite of the bacterial strain;
preferably, the bacterial strain is selected from any one or more of *Megasphaera stantonii, Megasphaera indica, Megasphaera paucivorans, Megasphaera sueciensis, Megasphaera micronuciformis, Megasphaera hexanoica, Megasphaera cerevisiae, Megasphaera hominis, Megasphaera butyrica, Megasphaera hutchinsoni, Megasphaera lornae,* or *Megasphaera vaginalis;*
preferably, the composition comprising the bacterial strain of the genus *Megasphaera* further comprises at least one of an excipient, a diluent, or a carrier.

14. The use according to claim 13, wherein the bacterial strain was deposited with the Guangdong Microbial Culture Collection Center with a deposit number of GDMCC No: 62001, GDMCC No: 62000, and/or GDMCC No: 61999;
preferably, the bacterial strain has an average nucleotide identity (ANI) value of at least 79% to the bacterial strain of the genus *Megasphaera* with a deposit number of GDMCC No: 62001, GDMCC No: 62000, and/or GDMCC No: 61999;
preferably, the average nucleotide identity (ANI) value is 79%, 80%, 81%, 82%, 83%, 84%, 85%, 86%, 87%, 88%, 89%, 90%, 91%, 92%, 93%, 94%, 95%, 95.5%, 96%, 96.5%, 97%, 97.5%, 98%, 98.5%, 99%, 99.5%, 99.6%, 99.7%, 99.8%, 99.9%, or 100%.

15. The use according to claim 13 or 14, wherein the metabolic disease is a metabolic disease mediated by HDAC activity;
preferably, the metabolic disease is diabetes mediated by HDAC activity; and more preferably, the metabolic disease is type I diabetes or type II diabetes or advanced diabetes or diabetic nephropathy or diabetic retinopathy mediated by HDAC activity.

16. The use according to any one of claims 13 to 15, wherein the metabolic disease comprises diabetes, which is manifested as a low level of insulin and/or peripheral insulin resistance leading to hyperglycemia.

17. The use according to claim 13, 14 or 15, wherein the metabolic disease is a disease caused by metabolic disorder, and preferably, the metabolic disorder comprises: (1) diabetes caused by glucose metabolic disorder, (2) diabetes caused by abnormal glucose tolerance or impaired glucose tolerance, (3) diabetes caused by impaired islet B cells, or (4) diabetes caused by insulin resistance.

18. The use according to any one of claims 13 to 17, wherein the diabetes comprises type I diabetes, type II diabetes, or gestational diabetes.

19. The use according to claim 13 or 14, wherein
the metabolic disease comprises obesity or obesity-related disease; such as at least one of obesity, coronary heart disease, atherosclerosis, fatty liver, alcoholic steatohepatitis, hypercholesterolemia, hypertension, hyperlipoproteinemia, hyperlipidemia, hypertriglyceridemia, uremia, ketoacidosis, thrombotic disease, dyslipidemia, non-alcoholic fatty liver disease (NAFLD), non-alcoholic steatohepatitis (NASH), atherosclerosis, or nephropathy.

20. The use according to any one of claims 13 to 19, wherein
the medicament is in a dosage form suitable for children or adults.

21. The use according to any one of claims 13 to 20, wherein the bacterial strain comprises viable bacteria, attenuated bacteria, killed bacteria, lyophilized bacteria, or irradiated bacteria.

22. The use according to any one of claims 13 to 21, wherein the medicament is in a dosage form for gastrointestinal administration or parenteral administration.

23. The use according to any one of claims 13 to 22, wherein the medicament is an oral drug or an injection.

24. The use according to any one of claims 13 to 23, wherein the medicament further comprises one or more second active ingredients.

25. The use according to claim 24, wherein the second active ingredient comprises at least one of a GLP-1 receptor agonist, a dual agonist of GLP-1 receptor and GCG receptor, a triple agonist of GLP-1 receptor, GIP receptor and GCG receptor, an AMPK agonist, or an active drug that promotes GLP-1 secretion;
preferably, the second active ingredient comprises one or more of metformin, sulfonylureas, meglitinides, thiazolidinediones, DPP-4 inhibitors, GLP-1 receptor agonists, SGLT2 inhibitors, insulin, pioglitazone, rosiglitazone, pentoxifylline, omega-3-fatty acids, statins, ezetimibe, ursodeoxycholic acid, Semaglutide, liraglutide, exenatide, or beinaglutide.

26. The use according to claim 15 or claim 16, wherein the bacterial strain promotes, elevates, or enhances the effect of the second active ingredient in the metabolic disease.

27. A pharmaceutical composition comprising a bacterial strain of the genus *Megasphaera,* wherein the bacterial strain is selected from a strain with at least 95%, 96%, 96.5%, 97%, 97.5%, 98%, 98.5%, 98.65%, 99%, 99.5%, 99.9%, or 100% identity to the 16S rRNA sequence of SEQ ID NO: 1, SEQ ID NO: 2, and/or SEQ ID NO: 3, or a strain of the genus *Megasphaera* that expresses EC2.8.3.9 and/or has a butyrate production pathway with more than 70% integrity;
preferably, the bacterial strain is selected from at least one of *Megasphaera stantonii, Megasphaera indica, Megasphaera paucivorans, Megasphaera sueciensis, Megasphaera micronuciformis, Megasphaera hexanoica, Megasphaera cerevisiae, Megasphaera hominis, Megasphaera butyrica, Megasphaera hutchinsoni, Megasphaera lornae,* or *Megasphaera vaginalis;*
preferably, the bacterial strain is regulated by GENE ID: 650027236, 641897133, 650594268, 642201644, 650018449, 650536170, 2511555023, and/or 646248671 to express EC2.8.3.9.

28. The pharmaceutical composition comprising a bacterial strain of the genus *Megasphaera* according to claim 27, wherein the composition can regulate at least one short-chain fatty acid or regulate a short-chain fatty acid salt, wherein the short-chain fatty acid comprises acetic acid, butyric acid, valeric acid, butyric-valeric acid, or hexanoic acid; and the short-chain fatty acid salt comprises acetate, propionate, or butyrate;
preferably, the composition can be used for treating a metabolic disease.

29. The pharmaceutical composition comprising a bacterial strain of the genus *Megasphaera* according to any one of claims 27 and 28, **characterized by** any one or more of (1) to (9) below:
(1) the pharmaceutical composition is in a dosage form suitable for infants, children, or adults;
(2) the pharmaceutical composition is in a dosage form for gastrointestinal administration or parenteral administration;
(3) the pharmaceutical composition is an oral preparation or an injection;
(4) the strain can at least partially proliferate in the intestinal tract of a subject;
(5) the strain, or a metabolite or culture of the strain, is present in the pharmaceutical composition in a mass percent content of 1-80%, 2-70%, 5-60%, 10-50%, 20-40%, 45%, 50%, 55%, or 60%;
(6) the strain is in a form selected from viable bacteria, attenuated bacteria, killed bacteria, lyophilized bacteria, or irradiated bacteria;
(7) the pharmaceutical composition comprises 1×10³ to 1×10¹³ colony forming units (CFU) of the strain per gram of the pharmaceutical composition by weight;
(8) the pharmaceutical composition is in powder or liquid dosage form;
(9) the pharmaceutical composition is prepared into a lyophilized powder, a tablet, a capsule, a granule, or an injection.

30. Use of the bacterial strain of any one of claims 1 to 4 in the manufacture of a medicament;
wherein, the medicament is used for at least one selected from the following:
reducing liver weight;
treating early-stage steatohepatitis focus;
slowing fat accumulation in liver cells;
reducing serum AST or ALT;
reducing inflammatory lesion in abdominal white fat;
reducing the body weight of a mammal;
reducing the food intake of a mammal;
reducing the body fat of a mammal;
reducing the serum level of at least one of total cholesterol, low-density lipoprotein, and triglycerides in a mammal;
increasing the serum high-density lipoprotein level in a mammal;
improving impaired oral glucose tolerance in a mammal;
lowering fasting blood glucose of a mammal;
reducing the HOMA-IR index in a mammal;
repairing digestive tract mucosal injury;
treating or preventing coronary heart disease;
treating or preventing atherosclerosis;
treating or preventing hyperglycemia;
treating or preventing hyperlipidemia;
treating or preventing hypercholesterolemia;
treating or preventing liver function impairment;
treating or preventing fatty liver;
treating or preventing NAFLD or NASH;
treating or preventing hypertension;
treating or preventing diabetes, preferably gestational diabetes, type II diabetes, or diabetes mediated by HDAC activity;
treating or preventing obesity;
treating or preventing metabolic syndrome;
treating or preventing localized excess sebum, excess inguinal fat, excess epididymal fat, and/or excess brown adipose tissue.

31. A method for treating or preventing a metabolic disease, comprising the step of administering to a subject in need thereof an effective amount of the bacterial strain of any one of claims 1 to 4, the microbial agent of claim 5, the culture of claim 6, or the composition of claim 7;
preferably, the metabolic disease is at least one of a liver disease, obesity or obesity-related disease, cardiovascular disease, diabetes, dyslipidemia, cardiovascular and cerebrovascular disease, glucose intolerance, atherosclerosis, coronary heart disease or hypertension, type I diabetes, type II diabetes, impaired glucose tolerance, insulin resistance, obesity, weight control, overweight, blood glucose control, prediabetes, hyperglycemia, hyperinsulinemia, fatty liver, alcoholic steatohepatitis, hypercholesterolemia, hypertension, hyperlipoproteinemia, hyperlipidemia, hypertriglyceridemia, uremia, ketoacidosis, hypoglycemia, thrombotic disease, dyslipidemia, non-alcoholic fatty liver disease (NAFLD), non-alcoholic steatohepatitis (NASH), atherosclerosis, nephropathy, diabetic neuropathy, or diabetic retinopathy;
preferably, the strain or a metabolite or culture of the strain treats or prevents the metabolic disease by at least one manner selected from: (a) reducing body weight; (b) reducing fasting blood glucose; (c) reducing blood lipids; (d) inhibiting HDAC activity; (e) inhibiting HDAC activity by at least one of acetic acid or acetate, propionic acid or propionate, butyric acid or butyrate, valeric acid or valerate in SCFAs; (f) inhibiting a tumor by at least one short-chain fatty acid or short-chain fatty acid salt of acetic acid or acetate, propionic acid or propionate, butyric acid or butyrate, valeric acid or valerate in SCFAs; or (g) exerting a therapeutic effect by regulating the intestinal flora of the subject.

32. The method according to claim 31, wherein the strain or a metabolite of the strain or the culture of claim 6 achieves the treatment or prevention of the metabolic disease by inhibiting HDAC activity.
